# EUROPEAN PATENT APPLICATION

(11) **EP 2 020 411 A1**
(43) Date of publication of application: **04.02.2009**
(21) Application number: 07743935.4
(22) Date of filing: 23.05.2007
(51) Int. Cl.: C07D 471/22, A61K 31/499, A61P 7/02, A61P 9/10, A61P 43/00

(54) **SPIRO TETRACYCLIC COMPOUND**

(30) Priority: 23.05.2006 JP 2006143194
(71) Applicant: MOCHIDA PHARMACEUTICAL CO., LTD., Shinjuku-ku, Tokyo 160-0004 (JP)
(72) Inventor: NISHIDA, Hidemitsu, Tokyo 160-8515 (JP); SAWAMA, Yuka, 44265 Dortmund (DE)
(74) Representative: Grünecker, Kinkeldey, Stockmair & Schwanhäusser Anwaltssozietät
(86) International application number: PCT/JP2007/060501
(87) International publication number: WO 2007/136085

(57) **Abstract**

Disclosed is an anti-coagulant which has extremely excellent inhibitory effect on FXa and an extremely poor inhibitory effect on hERG channel, and can be administered through the oral route. Specifically, dislosed is a compound represented by the formula (I) or a pharmaceutically acceptable salt thereof.

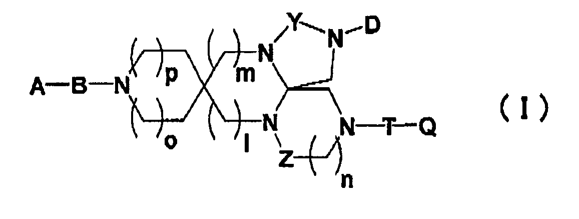

## Description

### TECHNICAL FIELD

This invention relates to an orally administrable tetracyclic compound having spiro union or a salt thereof which is useful as a drug, and in particular, as an inhibitor of activated blood coagulation factor X (hereinafter referred to as FXa), and which exhibits potent anticoagulation action with reduced side effects.

### BACKGROUND ART

With the westernization of the life style and the increase in the number of aged people, ischemic heart diseases and other pathology of heart and blood vessels are increasing year after year. In particular, thromboembolic diseases such as myocardial infarction, cerebral thrombosis, pulmonary embolism, and peripheral arterial and venous occlusive disease are increasing each year and treatment of such diseases has become a serious social issue. In the treatment and prevention of such thrombosis, anticoagulation therapy has been playing an important role in internal medicine together with antiplatelet therapy and fibrinolytic therapy. For the treatment and prevention of thrombosis, safety that permits long-term drug administration and the development of a reliable and appropriate anticoagulant activity are essential.

Heretofore, anticoagulants such as warfarin and heparin have been used in order to prevent and treat thrombosis caused by hypercoagulability. However, such use of the anticoagulants has been pointed to be associated with various demerits including the risk of bleeding and interactions with other drugs. Warfarin is extensively used in the world as the solo peroral anticoagulant. However, due to its characteristics based on the mechanism of action, the concentration range for the development of efficacy is narrow and yet it takes a long time to develop efficacy and the half-life in blood is as long as 36 hours; what is more, for several reasons such as the great individual difference of effective dose, it is difficult to control the anticoagulability of warfarin (see Non-Patent Document 1) and frequent monitoring is required in order to prevent bleeding as a side effect. In addition, warfarin also has many other side effects such as nausea, vomiting, diarrhea, and alopecia; thus, warfarin is a drug that involves considerable difficulty in clinical use. On the other hand, heparin is extensively used in the world as an intravenously administrable anticoagulant. However, since it is a direct inhibitor of thrombin, heparin has a high risk of bleeding and needs frequent monitoring as in the case of warfarin; what is more, due to its characteristics based on the mechanism of action, adequate coagulation inhibiting effect is not expected at a lowered antithrombin III level; thus, heparin is a drug that involves considerable difficulty in clinical use. In view of such situation, improved anticoagulants have been desired that has none of the defects inherent in warfarin and heparin.

The blood coagulation cascade is a chain reaction involving limited protein hydrolysis triggered by activation of the extrinsic coagulation cascade or the intrinsic coagulation cascade, and once the cascade is activated, the reaction is amplified like an avalanche. Since the final stage of the blood coagulation cascade is thrombin-mediated conversion of fibrinogen to fibrin, efforts have recently been made to develop thrombin inhibitors; however, drugs that directly inhibit thrombin are known to increase the risk of bleeding.

FXa is a key enzyme, which is located in the upstream of the thrombin in the coagulation cascade, and also at the junction of the extrinsic and the intrinsic coagulation cascade. One molecule of FXa is known to produce about a hundred molecules of thrombin per minute. Therefore, an FXa inhibitor can potentially inhibit the coagulation cascade more efficiently than a thrombin inhibitor (see Non-Patent Documents 2 and 3).

Low molecular weight FXa inhibitors are still under development, and development of some inhibitors such as DX-9065a, YM-60868, JTV-803, and DPC-906 is slowing down. As a consequence, no low molecular weight FXa inhibitor is on the market as a pharmaceutical. In addition, the technology of using a tetracyclic spiro compound is unknown in the field of such FXa inhibitor.

In the development of pharmaceutical products, the desired pharmacological activity is not the sole requirement. Another requirement is that strict criteria be met in various aspects including absorption, distribution, metabolism and excretion. For example, the drugs are required to pass various examinations for drug interaction, desensitization or tolerance, absorption from digestive tract in the oral administration, transfer rate to small intestine, absorption rate and first pass effect, organ barrier, protein binding, induction of drug metabolizing enzyme, excretion pathway and body clearance, administration method (site, method, and purpose of administration), and the like, and a drug meeting all such requirements is seldom discovered.

In recent years, proarrhythmic activity due to a drug, in particular, the prolongation of QT interval on an electrocardiogram has been attracting attention. One method known in the art that can be used in estimating the QT prolongation effect is evaluation of actions on hERG (human ether-a-go-go related gene) channel. Therefore, discovery of drugs having extremely weak effects on the hERG current whose importance in the generation of side effects on the heart is recognized is one of the most important issues in the drug development.

The anticoagulants also share such general challenge of the drug development. In the case of the FXa inhibitor, circumvention of the problem of the side effects associated with the oral administration of the warfarin as well as risk of bleeding based on the thrombin inhibition as found in the case of heparin whose administration is only accomplished by intravenous injection is required.

Non-Patent Document 1: N. Eng. J. Med., 1991, 324(26), 1865-1875.
Non-Patent Document 2: Thrombosis Research, 1980, vol. 19, pages 339-349.
Non-Patent Document 3: Mebio, 1997, vol. 14, No. 8.

### DISCLOSURE OF THE INVENTION

### PROBLEMS TO BE SOLVED BY THE INVENTION

In view of such situation, there is a demand for an anticoagulant drug which exhibits high safety and excellent effectivity with reduced side effects. To be more specific, there is a high demand for an anticoagulant which can be orally administered to mammals including humans, and in particular, which can be readily used in clinical practice, and which has realized at least one of avoidance of interaction with other drugs, reduced side effects including reduced risk of bleeding, improved dose response, extremely low inhibitory action of hERG current, and the like.

### MEANS TO SOLVE THE PROBLEMS

The present invention provides a pharmaceutical composition containing, as an effective component, a tetracyclic compound having spiro bond represented by formula (I), or a salt or a solvate thereof, or its derivative or a pharmaceutically acceptable salt or a solvate thereof.

### EFFECTS OF THE INVENTION

In order to solve the problems as described above, the inventors of the present invention conducted an intensive study to provide a compound having an excellent FXa inhibitory action, and found that the compound having spiro skeleton and represented by formula (I) exhibits extremely excellent FXa inhibitory action with extremely weak hERG channel inhibitory action, and that this compound is a potent candidate for use as an orally administerable anticoagulant.
Accordingly, the pharmaceutical composition of the present invention is an orally administerable anticoagulant exhibiting extremely excellent FXa inhibitory action and having an extremely weak hERG channel inhibitory action.

### BEST MODE FOR CARRYING OUT THE INVENTION

As will be described in the following aspects, the present invention provides a tetracyclic compound having spiro bond represented by formula (I) or its salt, a pharmaceutical composition containing such compound or its salt as an effective component, and use of such compound or its salt for medical application.
Next, various aspects of the present invention are described. In the compound of the present invention, "C₁₋₆", for example, means that "the group is a straight chain or branched group containing 1 to 6 carbon atoms" unless otherwise noted. In the case of a cyclic group, such notation denotes "the number of ring member carbon atoms".

The compound of formula (I) according to the present invention is not particularly limited for its molecular weight. The molecular weight, however, is preferably up to 1000, and more preferably up to 700 (and otherwise stated, the total number of carbon atoms constituting the compound is less than 40). Such limitation of molecular weight is routinely employed for identifying the structure of the compound as a major limiting factor in addition to the pharmacologically characteristic basic skeleton in recent drug design. In particular, the molecular weight is preferably limited to the range of up to 1000 when oral absorbability of the drug is taken into consideration.

### [ASPECTS OF THE INVENTION]

### [1] First aspect of the present invention

The compound of the present invention is a compound represented by the following formula (I) or its pharmaceutically acceptable salt or a solvate thereof: 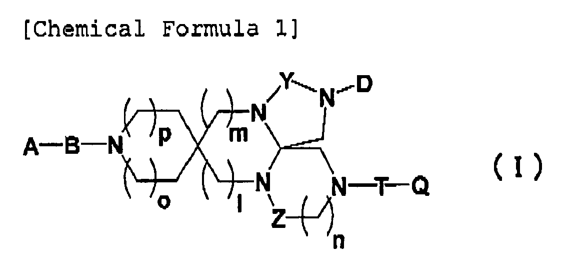 wherein
Q is an optionally substituted hydrocarbon group or heterocyclic group;
T is -S(O)ₓ-, carbonyl group, an optionally substituted C₁₋₂ alkylene group, or single bond;
A is hydrogen atom, or a group selected from (1) a saturated or unsaturated 3 to 6 membered cyclic hydrocarbon group, or a saturated or unsaturated 3 to 6 membered heterocyclic group, (2)amino group, (3) imidoyl group, (4) an aliphatic hydrocarbon group, and (5) a C₂₋₆ alkanoyl group, wherein the group selected from (1) to (5) is optionally substituted;
B is single bond, carbonyl group, -S(O)_{y}-, or an optionally substituted C₁₋₂ alkylene group;
D is hydrogen atom, -CO-R⁵ (wherein R⁵ is hydrogen atom or a substituent), or an optionally substituted C₁₋₆ alkyl group;
Y is methylene group, carbonyl group, or thiocarbonyl group;
Z is methylene group, carbonyl group, or thiocarbonyl group; and
l, m, n, o, p, x and y are independently an integer selected from 0, 1, and 2, with the proviso that 1 and m are not simultaneously 0; and
the ring containing N to which A-B- is bonded, the ring containing Y, the ring containing Z to which -T-Q is bonded, and the ring forming spiro union and containing two nitrogen atoms are optionally further substituted.

Next, various groups included in formula (I) are described in detail.
[1-1] In the compound of formula (I), Q is a hydrocarbon group or a heterocyclic group, and these groups may optionally have substituents. Exemplary "hydrocarbon groups" within the definition of Q include aliphatic hydrocarbon groups, alicyclic hydrocarbon groups, and aryl groups, and the preferred is an aryl group.
Exemplary "aliphatic hydrocarbon groups" include straight- or branched-chain hydrocarbon groups, for example, alkyl groups, alkenyl groups, and alkynyl groups.

Exemplary "alkyl groups" include C₁₋₁₀ (and more preferably C₁₋₆) alkyl groups, for example, methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, n-hexyl, 1-methyl-heptyl, and n-nonyl.
Exemplary "alkenyl groups" include C₂₋₆ alkenyl groups, for example, vinyl, allyl, isopropenyl, 2-methylallyl, butenyl, pentenyl, and hexenyl.
Exemplary "alkynyl groups" include C₂₋₆ alkynyl groups, for example, ethynyl, 1-propynyl, 2-propynyl, butynyl, pentynyl, and hexynyl.

Exemplary "alicyclic hydrocarbon groups" include saturated and unsaturated alicyclic hydrocarbon groups, for example, cycloalkyl group, cycloalkenyl group, and cycloalkanedienyl group.
Exemplary "cycloalkyl groups" include C₃₋₉ cycloalkyl groups, for example, cyclopropyl, cyclobutyl, cyclopentyl, cyclohexyl, cycloheptyl, cyclooctyl, and cyclononyl.
Exemplary "cycloalkenyl groups" include C₃₋₆ cycloalkenyl groups, for example, 1-cyclopropen-1-yl, 1-cyclobuten-1-yl, 1-cyclopenten-1-yl, 2-cyclopenten-1-yl, 3-cyclopenten-1-yl, and 1-cyclohexen-1-yl.
Exemplary "cycloalkanedienyl groups" include C₄₋₆ cycloalkanedienyl groups, for example, 2,4-cyclopentadien-1-yl and 2,5-cyclohexadien-1-yl.
Exemplary "aryl groups" include C₆₋₁₄ aryl groups, for example, phenyl, naphthyl, biphenylyl, 2-anthryl, phenanthryl, acenaphthyl, 5,6,7,8-tetrahydronaphthalenyl, and partially hydrogenated fused aryl groups such as indanyl and tetrahydronaphtyl, and among these, the preferred are phenyl, 2-naphthyl, and 1-naphthyl.

Exemplary heterocyclic groups of the "optionally substituted heterocyclic groups" in Q include aromatic heterocyclic groups, and saturated or unsaturated non-aromatic heterocyclic groups. Exemplary such heterocyclic groups include those having a five- to fourteen-membered ring, and preferably, a five- to twelve-membered ring containing at least one heteroatom (preferably 1 to 4 heteroatoms) selected from N, O and S in addition to the carbon atoms.

Exemplary "aromatic heterocyclic groups" include monocyclic and fused heterocyclic groups. Preferable monocyclic aromatic heterocyclic groups are those containing 5 to 6 ring members, for example, pyrrolyl, furyl, thienyl, oxazolyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, tetrazolyl, pyridyl, pyridazinyl, pyrimidinyl, pyrazinyl, 1,2,3-triazinyl, 1,2,4-triazinyl, 1,2,5-triazinyl, 1,3,5-triazinyl, and thiadiazinyl.

Preferable fused aromatic heterocyclic groups are those containing 8 to 12 ring members, for example, monovalent groups formed by condensation of the five- or six-membered aromatic ring as described above with one or more (preferably 1 to 2) aromatic rings (for example, benzene ring) followed by removal of hydrogen atom at an arbitrary position from the thus formed ring. Exemplary such groups include indolyl, isoindolyl, 1H-indazolyl, benzofuranyl(-2-yl), isobenzofuranyl, benzothienyl(-2-yl), isobenzothienyl, benzindazolyl, benzoxazolyl(-2-yl), 1,2-benzisoxazolyl, benzothiazolyl(-2-yl), 1,2-benzisothiazolyl, 2H-benzopyranyl(-3-yl), (1H-)benzimidazolyl(-2-yl), 1H-benzotriazolyl, 4H-1,4-benzoxazinyl, 4H-1,4-benzothiazinyl, quinolyl, isoquinolyl, cinnolinyl, quinazolinyl, quinoxalinyl, phthalazinyl, naphthyridinyl, purinyl, pteridinyl, carbazolyl, carbolinyl, acridinyl, phenoxazinyl, phenothiazinyl, phenazinyl, phenoxazinyl, thianthrenyl, phenanthridinyl, phenanthrolinyl, indolizinyl, (4,5,6,7-) tetrahydrothiazolo[5,4-c]pyridyl(-2-yl), (4,5,6,7-) tetrahydrothieno[3,2-c]pyridyl, (1,2,3,4-) tetrahydroisoquinolyl(-6-yl), thiazolo[5,4-c]pyridyl(-2-yl), pyrrolo[1,2-b]pyridazinyl, pyrazo[1,5-a]pyridyl, imidazo[1,2-a]pyridyl, imidazo[1,5-a]pyridyl, imidazo[1,2-b]pyridazinyl, imidazo[1,5-a]pyrimidinyl, 1,2,4-triazolo[4,3-a]pyridyl, 1,2,4-triazolo[4,3-b]pyridazinyl, chromenyl (2H-chromenyl), 1H-pyrazoio[3,4-b]pyridyl, and [1,2,4]triazoro[1,5-a]pyrimidinyl (Preferred embodiments are indicated in the brackets). Also included are partially hydrogenated fused aromatic heterocyclic groups such as tetrahydroquinolinyl, tetrahydroisoquinolinyl, tetrahydrobenzoxazepinyl, tetrahydrobenzoazepinyl, tetrahydronaphthopyridinyl, tetrahydroquinoxalinyl, chromanyl, dihydrobenzoxazinyl, 3,4-dihydro-2H-1,4-benzothiazinyl, dihydrobenzothiazolyl, 3,4-dihydro-2H-1,4-benzoxazinyl, isochromanyl, indolinyl, pteridinyl, 2,3-dihydrobenzo[b][1,4]dioxinyl, 1,2,3,4-tetrahydro-1-methylquinolinyl, 1,3-dihydro-1-oxoisobenzofuranyl, and 6,7,8,9-tetrahydro-5H-cyclohepta[b]pyridyl.

Exemplary "non-aromatic heterocyclic groups" include three- to eight-membered saturated and unsaturated non-aromatic heterocyclic groups, for example, aziridinyl, azetidinyl, oxiranyl, oxetanyl, thietanyl, pyrrolidinyl, tetrahydrofuryl, thiolanyl, pyrazolinyl, pyrazolidinyl, piperidyl, tetrahydropyranyl, piperazinyl, morpholinyl, oxazolinyl, thiazolinyl, thiomorpholinyl, quinuclidinyl, oxazinyl, isoxazolinyl, and tetrahydropyridinyl.

Exemplary "substituents" of the "optionally substituted hydrocarbon group" or the "optionally substituted heterocyclic group" in Q include (a) alkyl, alkenyl, alkynyl, aryl, cycloalkyl, and cycloalkenyl, (b) heterocyclic groups, (c)amino, (d) imidoyl, amidino, hydroxy, thiol, and oxo, (e) halogen atoms such as fluorine, chlorine, bromine, and iodine, cyano, and nitro, (f) carboxyl, and (g) carbamoyl, thiocarbamoyl, sulfonyl, sulfinyl, sulfide and acyl. Of the (a) to (g) as described above, the groups excluding (e) may further comprise a substituent.
The "optionally substituted hydrocarbon group" and the "optionally substituted heterocyclic group" in Q may be arbitrarily mono- to penta-substituted with such substituents. Such substituents (a) to (g) are described in further detail.

(a) The alkyl, alkenyl, alkynyl, aryl, cycloalkyl, and cycloalkenyl groups may be any of the "alkyl groups", "alkenyl groups", "alkynyl groups", "aryl groups", "cycloalkyl groups" and "cycloalkenyl groups" mentioned as examples of the "hydrocarbon group" for Q, and the preferred are C₁₋₆ alkyl groups, C₂₋₆ alkenyl groups, C₂₋₆ alkynyl groups, C₆₋₁₄ aryl groups, C₃₋₇ cycloalkyl groups and C₃₋₆ cycloalkenyl groups.
These groups may further include an optional substituent RI (wherein RI represents a group selected from a C₁₋₆ alkoxy, a C₁₋₆ alkoxycarbonyl, carboxyl, carbamoyl which is optionally mono- or di-substituted with a C₁₋₆ alkyl, a halogen, a C₁₋₆ alkyl, a halogenated C₁₋₆ alkyl, amino which is optionally mono- or di-substituted with a C₁₋₆ alkyl, a C₂₋₆ alkenoylamino, nitro, hydroxy, phenyl, phenoxy, benzyl, pyridyl, oxo, cyano, and amidino).

(b) The heterocyclic group may be any of the "aromatic heterocyclic groups" and "non-aromatic heterocyclic groups" mentioned as examples of the "heterocyclic group" for Q, and the preferred are (i) "five- or six-membered, monocyclic aromatic heterocyclic groups", (ii) "eight- to twelve-membered, fused aromatic heterocyclic groups", and (iii) "three- to eight-membered, saturated or unsaturated, non-aromatic heterocyclic groups", which contain 1 to 4 heteroatoms selected from nitrogen atom, oxygen atom and sulfur atom in addition to the carbon atoms.
These groups may further include an optional substituent RII (wherein RII represents a halogen atom such as fluorine, chlorine, bromine, or iodine, a C₁₋₆ alkyl group, a C₁₋₆ alkanoyl group, or benzoyl group).

(c) The "optionally substituted amino group" may be, for example, amino group which is optionally mono- or di-substituted with substituent RIII (wherein RIII represents a group selected from a C₁₋₆ alkyl, a C₁₋₆ alkanoyl, a C₂₋₆ alkenoyl, benzoyl, benzyl, phenyl, pyridyl optionally substituted with a group selected from c₁₋₆ alkyl, halogen, and trifluoromethyl, and a C₁₋₆ alkoxycarbonyl which is optionally substituted with 1 to 5 halogen atoms), or a three- to eight-membered monocyclic amino group which is optionally substituted with a group selected from a C₁₋₆ alkyl, a C₇₋₁₀ aralkyl, and a C₆₋₁₀ aryl.

(d) Exemplary substituents in the "optionally substituted imidoyl group, the optionally substituted amidino group, the optionally substituted hydroxy group, and the optionally substituted thiol group" include RIII (wherein RIII represents a group selected from a C₁₋₆ alkyl, a C₁₋₆ alkanoyl, a C₂₋₆ alkenoyl, benzoyl, benzyl, phenyl, pyridyl optionally substituted with a group selected from C₁₋₆ alkyl, halogen, and trifluoromethyl, and a C₁₋₆ alkoxycarbonyl which is optionally substituted with 1 to 5 halogen atoms) described in (c). Therefore, examples of (d) include a C₁₋₆ alkylimidoyl group, formimidoyl group or amidino group, a C₁₋₆ alkoxy group, benzyloxy group, a C₁₋₆ alkanoyloxy group, phenoxy group, pyridyloxy group optionally substituted with a group selected from C₁₋₆ alkyl, halogen, and trifluoromethyl, and oxo group.

(e) Halogen atoms such as fluorine, chlorine, bromine, and iodine, cyano group, and nitro group.

(f) The "optionally substituted carboxyl groups" include carboxyl group, C₁₋₆ alkoxycarbonyl groups, C₇₋₁₂ aryloxycarbonyl groups, and C₆₋₁₀ aryl-C₁₋₄ alkoxycarbonyl groups, and the aryl group in such (f) is optionally substituted with substituent RIV. RIV represents amino group which is optionally mono- or di-substituted with the substituent RII (wherein RII represents a halogen atom such as fluorine, chlorine, bromine, or iodine, a C₁₋₆ alkyl group, a C₁₋₆ alkanoyl group, or benzoyl group) of (b); a halogen atom; hydroxy group; nitro group; cyano group; a C₁₋₆ alkyl group which is optionally substituted with 1 to 5 halogen atoms; or an alkoxy group which is optionally substituted with 1 to 5 halogen atoms.

(g) The "optionally substituted carbamoyl group, the optionally substituted thiocarbamoyl group, the optionally substituted sulfonyl, the optionally substituted sulfinyl, the optionally substituted sulfide and the optionally substituted acyl group" are, for example, the groups represented by -CONR_{g}R_{g}', -CSNR_{g}R_{g}', -SO_{y}-R_{g}, or -CO-R_{g}, wherein: Rg represents hydrogen atom or a substituent RV (wherein RV represents a C₁₋₆ alkyl, a C₃₋₆ cycloalkyl, a C₆₋₁₀ aryl, a C₇₋₁₀ aralkyl, or a heterocyclic group, and the heterocyclic group is a heterocyclic group containing 1 to 4 heteroatoms selected from oxygen atom, sulfur atom, and nitrogen atom in addition to the carbon atoms which is any one of (i) a five- or six-membered monocyclic aromatic heterocyclic group, (ii) an eight- to twelve-membered, fused aromatic heterocyclic group, and (iii) a three- to eight-membered saturated or unsaturated non-aromatic heterocyclic group, and the alkyl, the cycloalkyl, the aryl, the aralkyl, or the heterocyclic group is optionally further substituted with substituent RIV of (f)); R_{g}' is hydrogen atom or a group selected from C₁₋₆ alkyl groups, C₃₋₆ cycloalkyl groups, and C₇₋₁₀ aralkyl groups, and y is 0, 1, or 2.

In the compound of formula (I), Q is preferably as described below.
[1-1-a] Examples of the "optionally substituted hydrocarbon group" or the "optionally substituted heterocyclic group" include:
(1) C₁₋₁₀ alkyl groups; (2) C₂₋₆ alkenyl groups; (3) C₂₋₆ alkynyl groups; (4) C₃₋₉ cycloalkyl groups; (5) C₃₋₆ cycloalkenyl groups; (6) C₄₋₆ cycloalkanedienyl groups; (7) C₆₋₁₄ aryl groups; and (8) a heterocyclic group containing 1 to 4 heteroatoms selected from oxygen atom, nitrogen atom and sulfur atom in addition to the carbon atoms which is any one of (i) "five- or six-membered, monocyclic aromatic heterocyclic groups", (ii) "eight- to twelve-membered, fused aromatic heterocyclic groups", and (iii) "three- to eight-membered, saturated or unsaturated, non-aromatic heterocyclic groups", and each group of the above (1) to (8) may be either unsubstituted or substituted with 1 to 5 substituents of the class selected from (a-1) to (g-1) as described below.

The classes are:
(a-1): a C₁₋₆ alkyl group, a C₂₋₆ alkenyl group, a C₂₋₆ alkynyl group, a C₆₋₁₄ aryl group, a C₃₋₇ cycloalkyl group, and a C₃₋₆ cycloalkenyl group. These substituents are optionally further substituted with substituent RI (wherein RI represents a group selected from a C₁₋₆ alkoxy; a C₁₋₆ alkoxycarbonyl; carboxyl; carbamoyl which is optionally mono- or di-substituted with a C₁₋₆ alkyl; a halogen; a C₁₋₆ alkyl; a halogenated C₁₋₆ alkyl; amino which is optionally mono- or di-substituted with a C₁₋₆ alkyl; a C₂₋₆ alkenoylamino; nitro; hydroxy; pyridyl; oxo; cyano; and amidino).
(b-1): a heterocyclic group containing 1 to 4 heteroatoms selected from oxygen atom, sulfur atom, and nitrogen atom in addition to the carbon atoms which is any one of (i) a "five- or six-membered, monocyclic aromatic heterocyclic group", (ii) an "eight- to twelve-membered, fused aromatic heterocyclic group", and (iii) a "three- to eight-membered, saturated or unsaturated, non-aromatic heterocyclic group". These heterocyclic groups are optionally further substituted with RII (wherein RII represents a group selected from a halogen atom such as fluorine, chlorine, bromine, or iodine, a C₁₋₆ alkyl, a C₁₋₆ alkanoyl, and benzoyl).
(c-1): amino group which is optionally substituted with substituent RIII (wherein RIII represents a group selected from a C₁₋₆ alkyl, a C₁₋₆ alkanoyl, a C₂₋₆ alkenoyl, benzoyl, benzyl, phenyl, pyridyl optionally substituted with a group selected from C₁₋₆ alkyl, halogen, and trifluoromethyl, and a C₁₋₆ alkoxycarbonyl which is optionally substituted with 1 to 5 halogen atoms), and a three- to eight-membered monocyclic amino group which is optionally substituted with a group selected from a C₁₋₆ alkyl, a C₇₋₁₀ aralkyl and a C₆₋₁₀ aryl.
(d-1): an imidoyl group, amidino group, hydroxy group, and a thiol group. These substituents are optionally further substituted with groups selected from substituents RIII as described above in the (c-1).
(e-1): a halogen atom such as fluorine, chlorine, bromine, or iodine, cyano group, and nitro group.
(f-1): carboxyl group, a C₁₋₆ alkoxycarbonyl group, a C₇₋₁₂ aryloxycarbonyl group, and a C₆₋₁₀ aryl-C₁₋₄ alkoxycarbonyl group; and the aryl group in such (f-1) is optionally further substituted with substituent RIV' (wherein RIV' represents amino which is optionally mono- or di-substituted with groups selected from RIII as described above in the (c-1); C₁₋₆ alkyl or C₁₋₆ alkoxy which is optionally substituted with 1 to 5 halogen atoms; a halogen atom; hydroxy; nitro; and cyano).
(g-1): a group -CONR_{g}R_{g}', -CSNR_{g}R_{g}', -CO-R_{g} or -SO_{y}-R_{g}
wherein:
Rg represents hydrogen atom or a substituent RV (wherein RV represents a C₁₋₆ alkyl, a C₃₋₆ cycloalkyl, a C₆₋₁₀ aryl, a C₇₋₁₀ aralkyl, or a heterocyclic group, and the heterocyclic group is a heterocyclic group containing 1 to 4 heteroatoms selected from oxygen atom, sulfur atom, and nitrogen atom in addition to the carbon atoms which is any one of (i) a five- or six-membered monocyclic aromatic heterocyclic group, (ii) an eight- to twelve-membered, fused aromatic heterocyclic group, and (iii) a three- to eight-membered, saturated or unsaturated, non-aromatic heterocyclic group, and the alkyl, the cycloalkyl, the aryl, the aralkyl, or the heterocyclic group is optionally further mono- to penta-substituted with the substituent RIV as described in the (f)); R_{g}' is a group selected from hydrogen atom, C₁₋₆ alkyl groups, C₃₋₆ cycloalkyl groups, and C₇₋₁₀ aralkyl groups; and y is 0, 1, or 2.

Of the groups shown in (a-1) to (g-1) as described above, the "most preferable groups" are substituents including a C₁₋₆ alkyl, a C₂₋₆ alkenyl, a C₂₋₆ alkynyl, a halogen atom, a halogenated C₁₋₆ alkyl, cyano, amino, hydroxy, carbamoyl, a C₁₋₆ alkoxy, a C₂₋₆ alkenyloxy, a C₂₋₆ alkynyloxy, a C₁₋₆ alkylthio, a C₁₋₆ alkylsulfinyl, a C₁₋₆ alkylsulfonyl, a mono/di C₁₋₆ alkylamino, a C₁₋₆ alkoxycarbonyl, a C₂₋₆ alkanoyl, a C₂₋₆ alkanoylamino, a hydroxy-C₁₋₆ alkyl, a C₁₋₆ alkoxy-C₁₋₆ alkyl, a carboxy-C₁₋₆ alkyl, a C₁₋₆ alkoxycarbonyl-C₁₋₆ alkyl, a carbamoyl-C₁₋₆ alkyl, a N-(C₁₋₆)alkylcarbamoyl-C₁₋₆ alkyl, a N,N-di C₁₋₆ alkylcarbamoyl-C₁₋₆ alkyl, phenyl, phenoxy, phenylthio, phenylsulfinyl, phenylsulfonyl, benzyl, and benzoyl, and the aromatic ring in these substituents may be further substituted with 1 to 3 subustituents selected from a halogen atom, trifluoromethyl, cyano, hydroxy, amino, nitro, carboxyl, carbamoyl, a C₁₋₆ alkyl, a C₁₋₆ alkoxy, a mono/di C₁₋₆ alkylamino, a di-C₁₋₆ alkylcarbamoyl, a C₁₋₆ alkoxycarbonyl, a N-C₁₋₆ alkylcarbamoyl, a N,N-di C₁₋₆ alkylcarbamoyl, and a C₂₋₆ alkenoylamino.

[1-1-b] Preferably, Q is (1) a C₁₋₆ alkyl group, (2) a C₂₋₆ alkenyl group, (7) a C₆₋₁₄ aryl group, or (8) (i) a five- or six-membered, monocyclic aromatic heterocyclic group, (ii) an eight- to twelve-membered, fused aromatic heterocyclic group, or (iii) a three- to eight-membered, saturated or unsaturated, non-aromatic heterocyclic group, which contains 1 to 4 heteroatoms selected from nitrogen atom, oxygen atom and sulfur atom in addition to the carbon atoms, and each group in (1), (2), (7) and (8) is optionally further mono- or di-substituted at an arbitrary position with the substituent of the class selected from [1-1] (a-1) to (g-1) (and most preferably, from those listed as "most preferable groups").

[1-1-c] More preferably, Q is (1') or (2'): a C₁₋₆ alkyl group (most preferably a C₁₋₂ alkyl group) or a C₂₋₆ alkenyl group (most preferably a C₂ alkenyl group) substituted with a substituent selected from substituent (a-1): a C₆₋₁₄ aryl group and substituent (b-1): an aromatic group selected from (i) five- or six-membered monocyclic aromatic heterocyclic groups and (ii) eight- to twelve-membered, fused aromatic heterocyclic groups, which contain 1 to 4 heteroatoms selected from nitrogen atom, oxygen atom and sulfur atom in addition to the carbon atoms; (7'): a C₆₋₁₄ aryl group which is optionally substituted with 1 to 2 halogen atoms; or (8'): a heterocyclic group which is (i) a five- or six-membered, monocyclic, aromatic heterocyclic group, (ii) an eight- to twelve-membered, fused aromatic heterocyclic group, or (iii) a three- to eight-membered, saturated or unsaturated, non-aromatic heterocyclic group, which contains 1 to 4 heteroatoms selected from nitrogen atom, oxygen atom and sulfur atom in addition to the carbon atoms, and wherein the carbon atoms are optionally mono- or di-substituted with a halogen atom.

The aromatic ring in the above substituent (1') or (2') is optionally further substituted with 1 to 3 substituents selected from halogen atoms, trifluoromethyl, cyano, hydroxy, amino, nitro, carboxyl, carbamoyl, a C₁₋₆ alkyl, a C₁₋₆ alkoxy, a mono/di C₁₋₆ alkylamino, a di C₁₋₆ alkylcarbamoyl, a C₁₋₆ alkoxycarbonyl, a N-C₁₋₆ alkylcarbamoyl, a N,N-di C₁₋₆ alkylcarbamoyl, and a C₂₋₆ alkenoylamino.
The aromatic ring in the substituents (7') and (8') is optionally further mono- or di-substituted at arbitrary position with the substituent of the class selected from (a-1) to (g-1) (and most preferably, from those listed as "most preferable groups").

[1-1-d] Still more preferably, Q is phenyl group, benzyl group, phenethyl group, styryl group, 1-naphthyl group, 2-naphthyl group, benzofuran-2-yl group, benzo[b]thiophen-2-yl group, indol-2-yl group, quinolin-3-yl group, 1H-benzimidazol-2-yl group, benzoxazol-2-yl group, benzothiazol-2-yl group, 2H-benzopyran-3-yl group, 4-vinylphenyl group, 4-benzenesulfonyl-thiophen-2-yl group, 5-(2-pyridyl)thiophen-2-yl group, quinolin-6-yl, or (thiophen-2-yl)ethenyl group, and the aromatic ring in such group is optionally further mono- or di-substituted with a halogen atom (most preferably chlorine atom or bromine atom) or a C₁₋₆ alkyl group (most preferably methyl group).

[1-1-e] Particularly preferably, Q is 2-naphthyl group, benzo[b]thiophen-2-yl group, indol-2-yl group, or (thiophen-2-yl)ethenyl group, and the aromatic ring in such group is optionally further mono- or di-substituted with a halogen atom (most preferably chlorine atom or bromine atom) or a C₁₋₆ alkyl group (most preferably methyl group).

[1-1-f] In the compound of formula (I), Q is most preferably indol-2-yl group, and the aromatic ring in such group is optionally further mono- or di-substituted with a halogen atom (most preferably chlorine atom or bromine atom) or a C₁₋₆ alkyl group (most preferably methyl group). [1-2] In formula (I), T is -S(O)ₓ- (wherein x is an integer selected from 0, 1, and 2, and in particular, 2), carbonyl group, an optionally substituted C₁₋₂ alkylene group (and in particular, a C₁₋₂ alkylene group optionally substituted with an optionally halogenated C₁₋₆ alkyl group or an optionally halogenated C₁₋₆ alkoxy group), or single bond. [1-2-a] Preferably, T is -SO₂- or -CH₂-, and [1-2-b] more preferably -SO₂-.

[1-3] In the compound of formula (I), A is hydrogen atom, or
(1) an optionally substituted saturated or unsaturated 3 to 6 membered cyclic hydrocarbon group, or an optionally substituted saturated or unsaturated 3 to 8 membered heterocyclic group,
(2) optionally substituted amino group,
(3) an optionally substituted imidoyl group,
(4) an optionally substituted aliphatic hydrocarbon group,
(5) an optionally substituted C₂₋₆ alkanoyl group.
   [1-3-1] In the (1) an optionally substituted saturated or unsaturated 3 to 6 membered cyclic hydrocarbon group, or an optionally substituted saturated or unsaturated 3 to 6
   membered heterocyclic group, "a saturated or unsaturated 3 to 6 membered cyclic hydrocarbon group" corresponds to the cyclic hydrocarbon groups having 3 to 6 carbon atoms in the "aliphatic hydrocarbon group" and "aryl group" of Q as described above. Exemplary such groups include cyclopropyl, cyclobutyl, cyclopentyl, cyclohexyl, cycloheptyl, cyclopropenyl, cyclobutenyl, cyclopentenyl, cyclopentadienyl, cyclohexadienyl, 1,3-cyclobutadienyl, and phenyl; and
   "a saturated or unsaturated 3 to 8 membered heterocyclic group" corresponds to the 3 to 8 membered monocyclic rings in the "aromatic heterocyclic groups, and saturated and unsaturated non-aromatic heterocyclic groups" of Q as described above. Such rings contain at least one heteroatom (preferably 1 to 4 heteroatoms) selected from N, O, and S in addition to the carbon atoms.

Exemplary "non-aromatic heterocyclic groups" include aziridinyl, azetidinyl, oxilanyl, oxetanyl, thietanyl, pyrolidinyl, tetrahydrofuryl, thiolanyl, pyrazolinyl, pyrazolidinyl, pyperidyl, tetrahydropyranyl, piperazinyl, oxazolinyl, thiazolinyl, morpholinyl, thiomorpholinyl, quinuclidinyl, oxazinyl, isoxazolinyl, and tetrahydropyridinyl.

Exemplary "aromatic heterocyclic groups" include pyrrolyl, furyl, thienyl, oxazolyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, tetrazolyl, pyridyl, pyridazinyl, pyrimidinyl, pyrazinyl, 1,2,3-triazinyl, 1,2,4-triazinyl, 1,2,5-triazinyl, 1,3,5-triazinyl, and thiadiazinyl.

Each of the rings of A may be further mono- to trisubstituted with Rq (wherein R_{q} is any one of substituent (1) to (8) of Q which is optionally substituted with any one of substituents (a) to (g)). Alternatively, A may be mono- or di-substituted with a group selected from R1 (wherein R1 is a substituent arbitrarily selected from group A (hydrogen atom, a halogen atom, trifluoromethyl group, trifluoromethoxy group, carboxyl group, carbamoyl group, sulfamoyl group, amino group, cyano group, nitro group, a lower alkanoyl group, a lower alkoxy group, a lower alkoxycarbonyl group, a lower alkylsulfonyl group, a lower alkylsulfinyl group, a mono- or di-substituted lower alkylamino group, a cyclic amino group, a lower alkanoylamino group, phenyl group, phenoxy group, benzyloxy group, benzoyl group, mercapto group, a lower alkylthio group, a lower alkyl thiocarbonyl group, hydroxy group, or a mono- or di-substituted lower alkyl aminocarbonyl group); oxygen atom which forms N-oxide group with the nitrogen atom on the ring; or a lower alkyl group, a lower alkoxy group, a lower alkenyl group, phenyl group, or a 5 to 6 membered heterocyclic group optionally substituted by any desired number of the substituent of group A).

[1-3-2] Examples of the optionally substituted amino group of (2) include amino group optionally mono- or di-substituted with substitutent RVII (wherein RVII is a C₁₋₁₀ alkyl group, formimide group, acetimidoyl group, a C₂₋₆ alkenyl group, a C₃₋₆ cycloalkenyl group, a C₃₋₉ cycloalkyl group, a C₄₋₆ cycloalkanedienyl group, or a C₆₋₁₄ aryl group). It should be noted that cyclic amino group is included in the "saturated or unsaturated 3 to 8 membered heterocyclic group" of [1-3-1] (1).

[1-3-3] Examples of the optionally substituted imidoyl group of (3) include group: -C(RVII')=N-RVII" wherein RVII' and RVII" are independenly hydrogen atom or any group selected from the substitutent RVII of (2).
It should be noted that cyclic imidoyl group is included in the "saturated or unsaturated 3 to 8 membered heterocyclic group" of [1-3-1] (1).

[1-3-4] Examples of the optionally substituted aliphatic hydrocarbon group of (4) include the group described in [1-1], which may be substituted with the substituent described in (a) to (g) of the "optionally substituted hydrocarbon group" in [1-1].
[1-3-5] Examples of the optionally substituted C₂₋₆ alkanoyl group of (5) include acetyl and propronyl which may be substituted with a substituent selected from amino group optionally mono- or di-substituted with any C₁₋₆ alkoxy or C₁₋₆ alkyl, and hydroxy.

With regard to A in formula (I), A is more preferably hydrogen atom, or
[1-3-1a] a 5 to 6 membered aromatic monoheterocyclic group which may contain 1 to 4 nitrogen atoms or 1 to 3 oxygen atoms or sulfur atoms in addition to the carbon atom; or a group represented by the following formula (A), (B), or (C) : 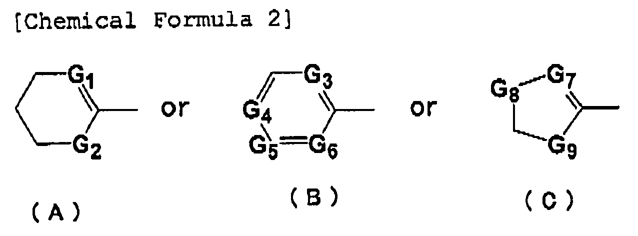 wherein G₁ and G₃ to G₇ are each independently CH or N, and G₂, G₈, and G₉ are each independently CH, O, or N, each ring being optionally mono- or di-substituted with the (a) to (g) as described above. More specifically, examples of the (A) include 5,6-dihydro-4H-1,3-oxazin-2-yl group and 3,4,5,6-tetrahydropyridin-2-yl group and examples of the (B) include 3-pyridazinyl group and 4-pyridyl group, and examples of the (C) include 2-oxazolinyl group and 4,5-dihydroisoxazol-3-yl group,

[1-3-2a] amino group, formimidoyl-amino group, or acetimidoyl amino group optionally mono- or di-substituted with a C₁₋₁₀ alkyl group or a C₆₋₁₄ aryl group,

[1-3-3a] group: -N(Ra")-C(Ra')=N-Ra or group: -C(Ra')=N-Ra wherein Ra" is hydrogen atom or a C₁₋₆ alkyl group; Ra' is hydrogen atom, a C₁₋₆ alkyl group, a C₁₋₆ alkanoyl group, benzoyl group, a C₁₋₆ alkoxy group or an amino group optionally mono- or di-substituted with a group selected from a C₁₋₆ alkyl group, C₁₋₆ alkanoyl group, or benzoyl; Ra is hydrogen atom, a C₁₋₆ alkyl group, a C₁₋₆ alkanoyl group, or benzoyl group; or -C(Ra')=N-Ra moiety of each group may form the group represented by the following formula (D) or (E):

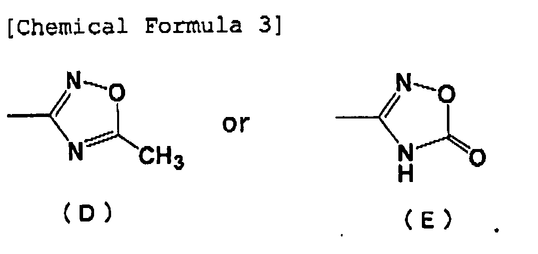

[1-3-4a] Preferable examples of the "aliphatic hydrocarbon group" include an optionally substituted C₁₋₆ alkyl group, an optionally substituted C₂₋₆ alkenyl group, an optionally substituted C₂₋₆ alkynyl group, an optionally substituted C₃₋₄ cycloalkyl group, an optionally substituted C₃₋₄ cycloalkenyl group, and an optionally substituted C₄ cycloalkanedienyl group.
The substituent of these may be selected from a substituent RI (wherein RI represents a group selected from a C₁₋₆ alkoxy, a C₁₋₆ alkoxycarbonyl, carboxyl, carbamoyl optionally mono- or di-substituted with a C₁₋₆ alkyl, a halogen, a C₁₋₆ alkyl, a halogenated C₁₋₆ alkyl, amino group which may be mono- or di-substituted with a C₁₋₆ alkyl, a C₂₋₆ alkenoylamino, nitro, hydroxy, phenyl, phenoxy, benzyl, oxo, cyano, and amidino).

[1-3-4b] More preferable examples of the "aliphatic hydrocarbon group" include "optionally substituted C₁₋₆ alkyl groups" such as methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, and n-hexyl which are optionally substituted with a group selected from a C₁₋₆ alkoxy, amino which is optionally mono- or di-substituted witch a C₁₋₆ alkyl, and hydroxy.

Examples of the "optionally substituted C₂₋₆ alkenyl group" include vinyl, allyl, isopropenyl, 2-methylallyl, butenyl, pentenyl, and hexenyl which are optionally substituted with a group selected from a C₁₋₆ alkoxy, amino which is optionally mono- or di-substituted with a C₁₋₆ alkyl, and hydroxy.

Examples of the "optionally substituted C₂₋₆ alkynyl group" include ethynyl, 1-propynyl, 2-propynyl, butynyl, pentynyl, and hexynyl which are optionally substituted with a group selected from a C₁₋₆ alkoxy, amino which is optionally mono- or di-substituted with a C₁₋₆ alkyl, and hydroxy.

Examples of the "optionally substituted C₃₋₄ cycloalkyl group" include cyclopropyl and cyclobutyl which are optionally substituted with a group selected from a C₁₋₆ alkoxy, amino which is optionally mono- or di-substituted with a C₁₋₆ alkyl, and hydroxy.
Examples of the "optionally substituted C₃₋₄ cycloalkenyl group" include C₃₋₄ cycloalkenyl groups such as 1-cyclopropen-1-yl and 1-cyclobuten-1-yl which are optionally substituted with a group selected from a C₁₋₆ alkoxy, amino which is optionally mono- or di-substituted with a C₁₋₆ alkyl, and hydroxy.
Examples of the "optionally substituted C₄ cycloalkanedienyl group" include C₄ cycloalkanedienyl groups such as 1,3-cyclobutadien-1-y1 which are optionally substituted with a group selected from a C₁₋₆ alkoxy, amino which is optionally mono- or di-substituted with a C₁₋₆ alkyl, and hydroxy.

[1-3-4c] Still more preferable examples of the "aliphatic hydrocarbon group" include C₁₋₄ alkyl groups such as methyl, ethyl, propyl, isopropyl, butyl, isobutyl, sec-butyl, and tert-butyl; C₂₋₆ alkenyl groups such as vinyl, allyl, isopropenyl, and 2-methylallyl; C₂₋₆ alkynyl groups such as ethynyl, 1-propynyl, and 2-propynyl; C₃₋₆ cycloalkyl groups such as cyclopropyl and cyclobutyl; C₃₋₄ cycloalkenyl groups such as 1-cyclopropen-1-yl and 1-cyclobuten-1-yl; and C₄ cycloalkanedienyl groups such as 1,3-cyclobutadien-1-yl which are optionally substituted with a group selected from a C₁₋₆ alkoxy, amino which is optionally mono- or di-substituted with a C₁₋₆ alkoxy, and hydroxy.

[1-3-4d] Examples of the "optionally substituted aliphatic hydrocarbon group" include methyl, ethyl, propyl, isopropyl, butyl, isobutyl, sec-butyl, tert-butyl, methoxymethyl, 2-methoxy-1-ethyl, 3-methoxy-1-propyl, hydroxymethyl, 2-hydroxy-1-ethyl, 3-hydroxy-1-propyl, aminomethyl, 2-amino-1-ethyl, 3-aminopropyl, (N-methylamino)methyl, 2-(N-methyl-amino)-1-ethyl, 3-(N-methylamino)propyl, (N,N-dimethyl-amino)methyl, 2-(N,N-dimethyl-amino)-1-ethyl, and 3-(N,N-dimethyl-amino)propyl; vinyl, allyl, isopropenyl, and 2-methylallyl; ethynyl, 1-propynyl, 2-propynyl, and 2-methoxy-1-vinyl; cyclopropyl and cyclobutyl; and 1-cyclopropen-1-yl, 1-cyclobuten-1-yl, and 1,3-cyclobutadien-1-yl.

[1-3-4e] More preferable examples of the "optionally substituted aliphatic hydrocarbon group" include methyl, ethyl, propyl, isopropyl, butyl, isobutyl, sec-butyl, tert-butyl, methoxymethyl, 2-methoxy-1-ethyl, hydroxymethyl, 2-hydroxy-1-ethyl, aminomethyl, 2-amino-1-ethyl, (N-methylamino)methyl, 2-(N-methyl-amino)-1-ethyl, (N,N-dimethylamino)methyl, and 2-(N,N-dimethyl-amino)-1-ethyl; vinyl and allyl; ethynyl and 2-methoxy-1-vinyl; and cyclopropyl and cyclobutyl.
[1-3-4f] Most preferable examples include C₁₋₂ alkyl groups such as methyl and ethyl.

[1-4] In the compound of formula (I), B is single bond, carbonyl group, -S(O)ₓ- (in particular, -S(O)ₓ- wherein x is 0 to 2, and preferably 2), or an optionally substituted C₁₋₂ alkylene group (in particular, a C₁₋₂ alkylene group optionally substituted with an optionally halogenated C₁₋₆ alkyl group or optionally halogenated C₁₋₆ alkoxy group).
[1-4-a] Preferably, B is single bond, carbonyl group, or -SO₂-.
[1-4-b] More preferably, B is single bond or carbonyl group.

[1-5] In the compound of formula (I), D is hydrogen atom, group -CO-R⁵ (wherein R⁵ is hydrogen atom or a substituent), or an optionally substituted C₁₋₆ alkyl group (preferably a C₁₋₆ alkyl group optionally substituted with R¹⁵ as described below).

[1-5-a] R⁵ is preferably hydrogen atom; hydroxy group; a C₁₋₆ alkyl group; a C₁₋₆ alkoxy group; a C₁₋₆ alkoxycarbonylalkyl group; phenoxy group or benzyloxy group which is optionally substituted with a C₁₋₆ alkyl, a C₁₋₆ alkoxy, or a halogen atom; or optionally substituted amino group, and in particular, group -NR⁶R⁷ (wherein R⁶ and R⁷ are independently hydrogen atom, a C₁₋₆ alkyl, a C₄₋₇ cycloalkyl, or a C₂₋₆ alkenyl; or R⁶ and R⁷ may together form a five- to seven-membered heterocyclic ring with the nitrogen atom to which R⁶ and R⁷ are bonded, the heterocyclic ring optionally further comprising 1 or 2 heteroatoms selected from N, S, and O); and such substituent R⁵ is optionally further substituted with a group selected from hydroxy, amino, carboxyl, a C₁₋₆ alkoxycarbonyl, oxo, a C₁₋₆ alkyl, a hydroxy-C₁₋₆ alkyl, a C₁₋₆ alkoxy-C₁₋₆ alkyl, a carboxy C₁₋₆ alkyl, a C₁₋₆ alkyl-C₁₋₆ alkoxycarbonyl, and a carbamoyl C₁₋₆ alkoxy).

[1-5-b] More preferably, D is hydrogen atom; or
1) a group selected from carboxyl group; a C₁₋₆ alkylcarbonyl group; a C₁₋₆ alkoxycarbonyl group; a C₁₋₆ alkoxycarbonylalkylcarbonyl group; phenoxycarbonyl group optionally substituted with a C₁₋₆ alkyl, a C₁₋₆ alkoxy, or a halogen atom; benzyloxycarbonyl group optionally substituted with a C₁₋₆ alkyl, a C₁₋₆ alkoxy, or a halogen atom;

2) carbamoyl group optionally mono- or di-substituted with a C₁₋₆ alkyl group; a C₁₋₆ alkoxycarbamoyl group; a C₁₋₆ alkoxycarbonylalkylcarbamoyl group; a cyclic aminocarbonyl group (in particular, pyrrolidin-1-ylcarbonyl group, piperidin-1-ylcarbonyl, piperazin-1-ylcarbonyl, 4-morpholinocarbonyl, thiomorpholinocarbonyl, or 1,1-dioxo-4-thiomorpholinocarbonyl) optionally substituted with oxo, hydroxy, amino, or carboxyl; or a group selected from N-phenylcarbamoyl group and a group represented by -CONH(CH₂)ₚS(O)_{q}R¹⁰ or -CONH(CH₂)ₜNR¹¹R¹² wherein R¹⁰, R¹¹, and R¹² independently represent hydrogen atom, a C₁₋₆ alkyl group, phenyl group, or a C₁₋₆ alkylphenyl group, p is an integer of 0 to 4, q is an integer of 0 to 2, and t is an integer of 1 to 4; or

3) a C₁₋₆ alkyl group (preferably methyl or ethyl) optionally substituted with R¹⁵ wherein R¹⁵ is carboxyl group; a C₁₋₆ alkoxycarbonyl group; hydroxy group; a C₁₋₆ alkoxy group; a C₁₋₆ alkanoyloxy group; amino group; amino group which is optionally mono- or di-substituted with substituent RX (wherein the substituent RX is a group selected from a C₁₋₆ alkyl, a C₁₋₆ alkanoyl, a C₁₋₆ alkylsulfonyl, a C₁₋₆ alkoxycarbonyl, a C₁₋₆ alkoxycarbonyl-C₁₋₆ alkyl, and a carboxy-C₁₋₆ alkyl); a five- or six-membered cyclic amino group (and in particular, pyrrolidin-1-yl, piperidin-1-yl, piperazin-1-yl, 4-morpholino, thiomorpholino, or 1,1-dioxo-4-thiomorpholino) or N-hydroxyimino group (aldoxime group) optionally substituted with oxo, hydroxy, amino, or carboxyl; a hydroxy-C₁₋₆ alkoxy group; a C₁₋₆ alkoxy-C₁₋₆ alkoxy group; a carboxy-C₁₋₆ alkoxy group; a C₁₋₆ alkoxycarbonyl-C₁₋₆ alkoxy group; a carbamoyl-C₁₋₆ alkoxy group; a C₀₋₆ alkoxy group (and in particular, a C₀₋₂ alkoxy group in which the C₀ alkoxy group designates the group wherein the substituent is directly bonded to the oxygen atom) mono-substituted with a substituent selected from (i) a five- or six-membered, monocyclic aromatic heterocyclic group and (ii) an eight- to twelve-membered, fused aromatic heterocyclic group containing 1 to 4 heteroatoms selected from oxygen atom, sulfur atom, and nitrogen atom in addition to the carbon atoms.

[1-5-c] Still more preferably, D is hydrogen atom; or
1) a group selected from carboxyl group, a C₁₋₂ alkylcarbonyl group, a C₁₋₂ alkoxycarbonyl group, and a C₁₋₂ alkoxycarbonylalkylcarbonyl group; or a group selected from phenoxycarbonyl group which is optionally substituted with a C₁₋₂ alkyl, a C₁₋₂ alkoxy, or a halogen atom, or benzyloxycarbonyl group which is optionally substituted with a C₁₋₂ alkyl, a C₁₋₂ alkoxy, or a halogen atom;

2) carbamoyl group which is optionally mono- or di-substituted with a C₁₋₂ alkyl; a C₁₋₂ alkoxycarbamoyl group; a C₁₋₂ alkoxycarbonylalkylcarbamoyl group; a cyclic aminocarbonyl group which is optionally substituted with oxo, hydroxy, amino or carboxyl (and in particular, pyrrolidin-1-ylcarbonyl group, piperidin-1-ylcarbonyl, piperazin-1-ylcarbonyl, 4-morpholinocarbonyl group, thiomorpholinocarbonyl group, or 1,1-dioxo-4-thiomorpholinocarbonyl group); or

3) methyl group or ethyl group optionally substituted with R^{15'} (wherein R^{15'} represents carboxyl group; a C₁₋₂ alkoxycarbonyl group; hydroxy group; a C₁₋₂ alkoxy group; a C₁₋₃ alkanoyloxy group; amino group; amino group which is optionally mono- or di-substituted with a substituent RX' wherein the substituent RX' is a group selected from a C₁₋₂ alkyl, a C₁₋₂ alkanoyl, a C₁₋₂ alkylsulfonyl, a C₁₋₂ alkoxycarbonyl, a C₁₋₂ alkoxycarbonyl-C₁₋₂ alkyl, and a carboxy-C₁₋₂ alkyl; pyrrolidin-1-yl group, piperidin-1-yl group, piperazin-1-yl group, 4-morpholino group, thiomorpholino group or 1,1-dioxo-4-thiomorpholino group which is optionally substituted with oxo, hydroxy, amino or carboxyl; hydroxy-methoxy group, 2-hydroxy-ethoxy group, 2-methoxy-ethoxy group, 2-ethoxy-ethoxy group, carboxy-methoxy group, 2-carboxy-ethoxy group, methoxycarbonyl-methoxy group, 2-methoxycarbonyl-ethoxy group, ethoxycarbonyl-methoxy group, 2-ethoxycarbonyl-(ethoxy or)methoxy group, carbamoyl-methoxy group, 2-carbamoyl-ethoxy group; and a C₀₋₂ alkoxy group (in which the C₀ alkoxy group designates the group wherein the substituent is directly bonded to the oxygen atom, for example, pyridyloxy group, pyridylmethoxy group, pyridylethoxy group, pyrimidyloxy group, pyrimidylmethoxy group and pymidylethoxy group) mono-substituted with a five- or six-membered, monocyclic aromatic heterocyclic group containing 1 to 2 nitrogen atoms in addition to the carbon atoms.

[1-5-d] In formula (I), D is most preferably hydrogen atom, a C₁₋₂ alkylcarbonyl group (acetyl group or propionyl group), or a C₁₋₄ alkyl group (in particular, methyl group or ethyl group).

[1-6] In formula (I), Y is methylene group, carbonyl group, or thiocarbonyl group, and
[1-6-a] preferably, methylene group or carbonyl group.

[1-7] In formula (I), Z is methylene group, carbonyl group, or thiocarbonyl group, and
[1-7-a] preferably, carbonyl group.

[1-8] In formula (I), 1, m, n, o, p are each independently an integer selected from 0, 1, and 2 with the proviso that 1 and m are not simultaneously 0, and
[1-8-a] preferably, l is 1, m is 0 or 1, n is 1, o is 1, and p is 1.

[1-9] Exemplary substituents of the N-containing ring having the A-B- bonded thereto, the Y-containing ring, the Z-containing ring having the -T-Q- bonded thereto, and the ring containing two N which forms the spiro union include oxo group (=O), hydroxyimino group (=N∼OH), an alkoxyimino group (=N∼ORi wherein Ri is a C₁₋₆ alkyl group which is optionally substituted with a substituent preferably selected from a halogen, hydroxyl, and carboxy), and the groups mentioned for D in [1-5]. Preferable substituents include oxo group, hydroxy group, carboxyl group, a halogen atom, a C₁₋₆ alkyl group, a C₂₋₆ alkenyl group, and a C₂₋₆ alkynyl group, and among these, a C₁₋₆ alkyl group, a C₂₋₆ alkenyl group, and a C₂₋₆ alkynyl group may be further substituted with a substituent RI (wherein RI represents a group selected from a C₁₋₆ alkoxy, a C₁₋₆ alkoxycarbonyl, carboxyl, carbamoyl which is optionally mono- or di-substituted with a C₁₋₆ alkyl, a halogen, a C₁₋₆ alkyl, a halogenated C₁₋₆ alkyl, amino which is optionally mono- or di-substituted with a C₁₋₆ alkyl, a C₂₋₆ alkenoylamino, nitro, hydroxy, oxo, cyano, and amidino). More preferable substituents include oxo group, a C₁₋₆ alkoxy group, and carboxyl group.

Preferably,
[1-9-a] exemplary substituents for the nitrogen-containing ring having the A-B- bonded thereto include oxo group, hydroxyl group, lower alkyl group, and lower alkoxyalkyl group, while the ring may be unsubstituted,
[1-9-b] exemplary substituents for the Z-containing ring having the -T-Q- bonded thereto include oxo group, hydroxyimino group, substituted alkoxyimino group (=N∼ORi wherein Ri is a C₁₋₆ alkyl group which is optionally substituted with a substituent preferably selected from a halogen, hydroxyl, and carboxy), while the ring may be unsubstituted, and
[1-9-c] exemplary substituents for the ring containing two N which forms the spiro union include oxo group, hydroxyimino group, substituted alkoxyimino group (=N∼ORi wherein Ri is a C₁₋₆ alkyl group optionally substituted with a substituent which is preferably selected from a halogen, hydroxyl, and carboxy), while the ring may be unsubstituted, and the position of the substitution also includes the case wherein the oxo group is substituted with hydroxyimino group or a substituted alkoxyimino group.
Preferable compounds of formula (I) may be defined by adequate combinations of the [1-1] to[1-9]. Exemplary preferable compounds are described in [1-10].

[1-10] In formula (I), 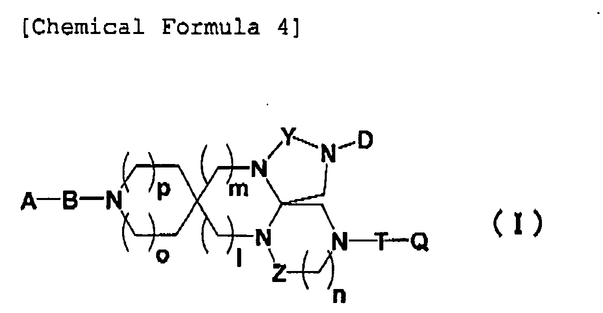 Q is (1) a C₁₋₁₀ alkyl group, (2) a C₂₋₆ alkenyl group, (3) a C₂₋₆ alkynyl group, (4) a C₃₋₉ cycloalkyl group, (5) a C₃₋₆ cycloalkenyl group, (6) a C₄₋₆ cycloalkadienyl group, (7) a C₆₋₁₄ aryl group, or (8) a heterocyclic group containing 1 to 4 heteroatoms selected from oxygen atom, nitrogen atom and sulfur atom in addition to the carbon atoms which is any one of (i) a "five- or six-membered, monocyclic aromatic heterocyclic group", (ii) a "eight- to twelve-membered, fused aromatic heterocyclic group", and (iii) a "three- to eight-membered, saturated or unsaturated, non-aromatic heterocyclic group", and the groups of the (1) to (8) may be either unsubstituted or substituted with 1 to 5 substituents of the class selected from (a) to (g) as described below.

(a) A C₁₋₆ alkyl group or a C₆₋₁₄ aryl group which is optionally further substituted with a substituent RI (wherein RI represents a C₁₋₆ alkoxy group, a halogen, a C₁₋₆ alkyl group, amino group, hydroxy group, cyano group, or amidino group).

(b) A heterocyclic group containing 1 to 4 heteroatoms selected from oxygen atom, nitrogen atom and sulfur atom in addition to the carbon atoms which is any one of (i) a "five- or six-membered, monocyclic aromatic heterocyclic group", (ii) a "eight- to twelve-membered, fused aromatic heterocyclic group", and (iii) a "three- to eight-membered, saturated or unsaturated, non-aromatic heterocyclic group", and which may be further substituted with substituent RII (wherein RII represents a halogen atom such as fluorine, chlorine, bromine, or iodine, a C₁₋₆ alkyl group, a C₁₋₆ alkanoyl group, or benzoyl group).

(c) amino group optionally substituted with a group selected from substituents RIII wherein RIII represents a C₁₋₆ alkyl group, a C₁₋₆ alkanoyl group, benzoyl group, benzyl group, phenyl group, pyridyl group optionally substituted with a group selected from C₁₋₆ alkyl, halogen, and trifluoromethyl, or an optionally halogenated C₁₋₆ alkoxycarbonyl group, a C₁₋₆ alkylimidoyl group, formimidoyl group, or amidino group.

(d) Imidoyl group, amidino group, hydroxy group, or thiol group, which is optionally further substituted with a substituent selected from a C₁₋₆ alkyl group, a C₁₋₆ alkanoyl group, benzoyl group, an optionally halogenated C₁₋₆ alkoxycarbonyl group.
(e) A halogen atom, cyano group, or nitro group.

(f) Carboxyl group, a C₁₋₆ alkoxycarbonyl group, a C₇₋₁₂ aryloxycarbonyl group, or a C₆₋₁₀ aryl-C₁₋₄ alkoxycarbonyl group. The aryl group is optionally further substituted with a substituent RIV (wherein RIV represents hydroxy, amino group optionally mono- or di-substituted with a group selected from those mentioned in the above (c) for the substituent RIII, a halogen atom, nitro group, cyano group, a C₁₋₆ alkyl group optionally substituted with 1 to 5 halogen atoms, or an alkoxy group optionally substituted with 1 to 5 halogen atoms).

(g) -CO-RV wherein RV represents a C₁₋₆ alkyl group, a C₃₋₆ cycloalkyl group, a C₆₋₁₀ aryl group, a C₇₋₁₀ aralkyl group, or a heterocyclic group. This heterocyclic group is a heterocyclic group containing 1 to 4 heteroatoms selected from oxygen atom, nitrogen atom and sulfur atom in addition to the carbon atoms which is any one of (i) a "five- or six-membered, monocyclic aromatic heterocyclic group", (ii) a "eight- to twelve-membered, fused, aromatic heterocyclic group", and (iii) a "three- to eight-membered, saturated or unsaturated, non-aromatic heterocyclic group".
More preferably, Q is phenyl group, benzyl group, phenethyl group, styryl group, 1-naphthyl group, 2-naphthyl group, benzofuran-2-yl group, benzo[b]thiophen-2-yl group, indol-2-yl group, quinolin-3-yl group, 1H-benzimidazol-2-yl group, benzoxazol-2-yl group, benzothiazol-2-yl group, 2H-benzopyran-3-yl group, 4-vinylphenyl group, 4-benzenesulfonyl-thiophen-2-yl group, 5-(2-pyridyl)thiophen-2-yl group, quinolin-6-yl group, or (thiophen-2-yl)ethenyl group wherein the aromatic ring is optionally mono- or di-substituted with a halogen atom (in particular, chlorine atom or bromine atom) or a C₁₋₆ alkyl group (in particular, methyl group), and still more preferably, Q is 2-naphtyl group, benzo[b]thiophen-2-yl group, indol-2-yl group, or (thiophen-2-yl)ethenyl group wherein the aromatic ring is mono- or di-substituted with a halogen atom (in particular, chlorine atom or bromine atom) or a C₁₋₆ alkyl group (in particular, methyl group), and most preferably, Q is indol-2-yl group, and the aromatic ring in such group is optionally mono- or di-substituted with halogen atom (most preferably chlorine atom or bromine atom) or a C₁₋₆ alkyl group (most preferably methyl group);

T is -S(O)ₓ- (wherein x is an integer selected from 0, 1, and 2, and in particular, 2), carbonyl group, an optionally substituted C₁₋₂ alkylene group (and in particular, a C₁₋₂ alkylene group optionally substituted with an optionally halogenated C₁₋₆ alkyl group or an optionally halogenated C₁₋₆ alkoxy group), or single bond; preferably, -SO₂- or -CH₂-; and more preferably -SO₂-; A is hydrogen atom, or
(1) a 5 to 6 membered saturated or unsaturated monoheterocyclic group which may contain 1 to 4 nitrogen atoms or 1 to 3 oxygen atoms or sulfur atoms in addition to the carbon atom; for example, a group represented by the following formula (A), (B), or (C): 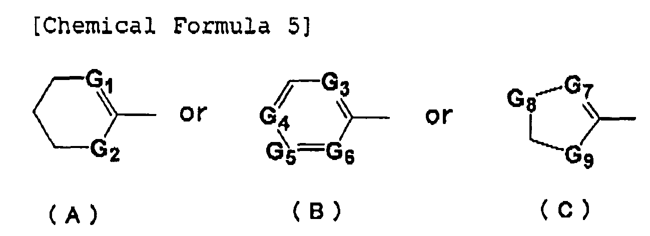 wherein G₁ and G₃ to G₇ are each independently CH or N, and G₂, G₈, and G₉ are each independently CH, O, or N, each ring being optionally mono- or di-substituted with the (a) to (g) as described above. More preferably, specific examples of the (A) include 5,6-dihydro-4H-1,3-oxazin-2-yl group and 3,4,5,6-tetrahydropyridin-2-yl group and specific examples of the (B) include 3-pyridazinyl group and 4-pyridyl group, and specific examples of the (C) include 2-oxazolinyl group and 4,5-dihydroisoxazol-3-yl group,

(2)amino group, formimidoyl-amino group, or acetimidoylamino group optionally mono- or di-substituted with a C₁₋₁₀ alkyl group or a C₆₋₁₄ aryl group,
(3) group: -N(Ra")-C(Ra')=N-Ra or group: -C(Ra')=N-Ra
wherein
Ra" is hydrogen atom or a C₁₋₆ alkyl group,
Ra' is hydrogen atom, a C₁₋₆ alkyl group, a C₁₋₆ alkanoyl group, benzoyl group, a C₁₋₆ alkoxy group, or an amino group optionally mono- or di-substituted with a group selected from a C₁₋₆ alkyl group, a C₁₋₆ alkanoyl group; and benzoyl group,
Ra is hydrogen atom, a C₁₋₆ alkyl group, a C₁₋₆ alkanoyl group, or benzoyl group,

(4) an optionally substituted C₁₋₆ alkyl group, an optionally substituted C₂₋₆ alkenyl group, or an optionally substituted C₂₋₆ alkynyl group (wherein the substituent may be a substituent RI (wherein RI represents a group selected from a C₁₋₆ alkoxy, a C₁₋₆ alkoxycarbonyl, a carboxyl, carbamoyl optionally mono- or di-substituted with a C₁₋₆ alkyl, a halogen, a C₁₋₆ alkyl, a halogenated C₁₋₆ alkyl, amino group which is mono- or di-substituted with a C₁₋₆ alkyl, a C₂₋₆ alkenoylamino, nitro, hydroxy, phenyl, phenoxy, benzyl, oxo, cyano, and amidino)),

Exemplary C₁₋₆ alkyl groups include methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, and n-hexyl; exemplary C₂₋₆ alkenyl groups include vinyl, allyl, isopropenyl, 2-methylallyl, butenyl, pentenyl, and hexenyl; and exemplary C₂₋₆ alkynyl groups include ethynyl, 1-propynyl, 2-propynyl, butynyl, pentynyl, and hexynyl; which are optionally substituted with a group selected from a C₁₋₆ alkoxy, amino group which is optionally mono- or di-substituted with a C₁₋₆ alkyl, or hydroxy.

More preferably, examples of C₁₋₄ alkyl include methyl, ethyl, propyl, isopropyl, butyl, isobutyl, sec-butyl, and tert-butyl; examples of C₂₋₆ alkenyl group include vinyl, allyl, isopropenyl, and 2-methylallyl; and examples of C₂₋₆ alkynyl group include ethynyl, 1-propynyl, and 2-propynyl; and these groups may be substituted with a group selected from a C₁₋₆ alkoxy, amino which is optionally mono- or di-substituted with a C₁₋₆ alkyl or hydroxy. Exemplary such groups include methoxymethyl, 2-methoxy-1-ethyl, 3-methoxy-1-propyl, hydroxymethyl, 2-hydroxy-1-ethyl, 3-hydroxy-1-propyl, aminomethyl, 2-amino-1-ethyl 3-amino-propyl, (N-methyl-amino)methyl, 2-(N-methyl-amino)-1-ethyl, 3-(N-methyl-amino)propyl, (N,N-dimethyl-amino) methyl, 2-(N,N-dimethyl-amino)-1-ethyl, 3-(N,N-dimethylamino)propyl, and 2-methoxy-1-vinyl.
Most preferable examples include C₁₋₂ alkyl groups such as methyl and ethyl.

B is single bond, carbonyl group, -S(O)ₓ- (in particular, -S(O)ₓ- wherein x is 0 to 2, and preferably 2), or an optionally substituted C₁₋₂ alkylene group (in particular, a C₁₋₂ alkylene group optionally substituted with an optionally halogenated C₁₋₆ alkyl group or optionally halogenated C₁₋₆ alkoxy group); preferably, B is single bond, carbonyl group, or -S(O)₂-; and
more preferably, B is single bond or carbonyl group.

Particularly preferable examples of A-B include 5,6-dihydro-4H-1,3-oxazin-2-yl group, 3,4,5,6-tetrahydropyridin-2-yl group, 3-pyridazinyl group, 4-pyridyl group, and 2-oxazolinyl group, 4,5-dihydroisoxazol-3-yl group, acetyl group, and propionyl group.
D is hydrogen atom, group -CO-R⁵ (wherein R⁵ is hydrogen atom or a substituent), or an optionally substituted C₁₋₆ alkyl group (preferably a C₁₋₆ alkyl group optionally substituted with R¹⁵ as described below).

More preferably, D is hydrogen atom;
1) a group selected from carboxyl group; a C₁₋₆ alkylcarbonyl group; a C₁₋₆ alkoxycarbonyl group; a C₁₋₆ alkoxycarbonylalkylcarbonyl group; phenoxycarbonyl group optionally substituted with a C₁₋₆ alkyl, a C₁₋₆ alkoxy, or a halogen atom; benzyloxycarbonyl group optionally substituted with a C₁₋₆ alkyl, a C₁₋₆ alkoxy, or a halogen atom;
2) carbamoyl group optionally mono- or di-substituted with a C₁₋₆ alkyl group; a C₁₋₆ alkoxycarbamoyl group; a C₁₋₆ alkoxycarbonylalkylcarbamoyl group; a cyclic aminocarbonyl group (in particular, pyrrolidin-1-ylcarbonyl group, piperidin-1-ylcarbonyl, piperazin-1-ylcarbonyl, 4-morpholinocarbonyl, thiomorpholinocarbonyl, or 1,1-dioxo-4-thiomorpholinocarbonyl) optionally substituted with oxo, hydroxy, amino, or carboxyl; or a group selected from N-phenylcarbamoyl group and a group represented by -CONH(CH₂)_{P}S(O)_{q}R¹⁰ or -CONH(CH₂)ₜNR₁₁R₁₂ wherein R¹⁰, R¹¹, and R¹² independently represent hydrogen atom, a C₁₋₆ alkyl group, phenyl group, or a C₁₋₆ alkylphenyl group, p is an integer of 0 to 4, q is an integer of 0 to 2, and t is an integer of 1 to 4; or

3) a C₁₋₆ alkyl group (preferably methyl or ethyl) optionally substituted with _{R}¹⁵ wherein R¹⁵ is carboxyl group; a C₁₋₆ alkoxycarbonyl group; hydroxy group; a C₁₋₆ alkoxy group; a C₁₋₆ alkanoyloxy group; amino group; amino group mono- or di-substituted with substituent RX (wherein the substituent RX is a group selected from a C₁₋₆ alkyl, a C₁₋₆ alkanoyl, a C₁₋₆ alkylsulfonyl, a C₁₋₆ alkoxycarbonyl, a C₁₋₆ alkoxycarbonyl-C₁₋₆ alkyl, and a carboxy-C₁₋₆ alkyl); a five- or six-membered cyclic amino group (and in particular, pyrrolidin-1-yl, piperidin-1-yl, piperazin-1-yl, 4-morpholino, thiomorpholino, or 1,1-dioxo-4-thiomorpholino) or N-hydroxyimino group (aldoxime group) optionally substituted with oxo, hydroxy, amino, or carboxyl; a hydroxy-C₁₋₆ alkoxy group; a C₁₋₆ alkoxy-C₁₋₆ alkoxy group; a carboxy-C₁₋₆ alkoxy group; a C₁₋₆ alkoxycarbonyl-C₁₋₆ alkoxy group; a carbamoyl-C₁₋₆ alkoxy group; a C₀₋₆ alkoxy group (and in particular, a C₀₋₂ alkoxy group in which the C₀ alkoxy group designates the group wherein the substituent is directly bonded to the oxygen atom) mono-substituted with a substituent selected from (i) a five- or six-membered, monocyclic aromatic heterocyclic group and (ii) an eight- to twelve-membered, fused aromatic heterocyclic group containing 1 to 4 heteroatoms selected from oxygen atom, sulfur atom, and nitrogen atom in addition to the carbon atoms.

Still more preferably, D is hydrogen atom;
1) a group selected from carboxyl group, a C₁₋₂ alkylcarbonyl group, a C₁₋₂ alkoxycarbonyl group, and a C₁₋₂ alkoxycarbonylalkylcarbonyl group; or a group selected from phenoxycarbonyl group which is optionally substituted with a C₁₋₂ alkyl, a C₁₋₂ alkoxy, or a halogen atom, or benzyloxycarbonyl group which is optionally substituted with a C₁₋₂ alkyl, a C₁₋₂ alkoxy, or a halogen atom;
2) carbamoyl group which is optionally mono- or di-substituted with a C₁₋₂ alkyl; a C₁₋₂ alkoxycarbamoyl group; a C₁₋₂ alkoxycarbonylalkylcarbamoyl group; a cyclic aminocarbonyl group which is optionally substituted with oxo, hydroxyl, amino or carboxyl (and in particular, pyrrolidin-1-ylcarbonyl group, piperidin-1-ylcarbonyl, piperazin-1-ylcarbonyl, 4-morpholinocarbonyl group, thiomorpholinocarbonyl group, or 1,1-dioxo-4-thiomorpholinocarbonyl group); or

3) methyl group or ethyl group optionally substituted with R^{15'} (wherein R^{15'} represents carboxyl group; a C₁₋₂ alkoxycarbonyl group; hydroxyl group; a C₁₋₂ alkoxy group; a C₁₋₃ alkanoyloxy group; amino group; amino group mono- or di-substituted with a substituent RX' wherein the substituent RX' is a group selected from a C₁₋₂ alkyl, a C₁₋₂ alkanoyl, a C₁₋₂ alkylsulfonyl, a C₁₋₂ alkoxycarbonyl, a C₁₋₂ alkoxycarbonyl-C₁₋₂ alkyl, and a carboxy-C₁₋₂ alkyl; pyrrolidin-1-yl group, piperidin-1-yl group, piperazin-1-yl group, 4-morpholino group, thiomorpholino group or 1,1-dioxo-4-thiomorpholino group which is optionally substituted with oxo, hydroxyl, amino or carboxyl; hydroxy-methoxy group, 2-hydroxy-ethoxy group, 2-methoxy-ethoxy group, 2-ethoxy-ethoxy group, carboxy-methoxy group, 2-carboxy-ethoxy group, methoxycarbonyl-methoxy group, 2-methoxycarbonyl-ethoxy group, ethoxycarbonyl-methoxy group, 2-ethoxycarbonyl-ethoxy group, carbamoyl-methoxy group, 2-carbamoyl-ethoxy group; and a C₀₋₂ alkoxy group (in which the C₀ alkoxy group designates the group wherein the substituent is directly bonded to the oxygen atom, for example, pyridyloxy group, pyridylmethoxy group, pyridylethoxy group, pyrimidyloxy group, pyrimidylmethoxy group and pymidylethoxy group) mono-substituted with a five- or six-membered, monocyclic aromatic heterocyclic group containing 1 to 2 nitrogen atoms in addition the carbon atom.
Most preferably, D is hydrogen atom, a C_{1∼2} alkylcarbonyl group (for example, acetyl group or propionyl group), or a C_{1∼4} alkyl group (in particular, methyl group, ethyl group, etc.).

Y is methylene group, carbonyl group, or thiocarbonyl group, and preferably, methylene group or carbonyl group,
Z is methylene group, carbonyl group, or thiocarbonyl group, and preferably carbonyl group, and
l, m, n, o, p are each independently an integer selected from 0, 1, and 2, with the proviso that 1 and m are not simultaneously 0, and preferably, 1 is 1, m is 0 or 1, n is 1, o is 1, and p is 1.

### [1-11]

Exemplary preferable compounds are:
(±)-3',5',8',11'-tetraaza-11'-(6-chloronaphthalen-2-ylsulfonyl)-3'-methyl-1-(2-oxazolinyl) spiro[piperidine-4,6'-tricyclo[6.4.0.0^{1,5}]dodecane]-4',9'-dione (Example 1);
(t)-3',5',8',11'-tetraaza-11'-(6-chloronaphthalen-2-ylsulfonyl)-1-(2-oxazolinyl) spiro[piperidine-4,6'-tricyclo[6.4.0.0^{1,5}]dodecane]-4',9'-dione (Example 2);
(±)-3',5',8',11'-tetraaza-11'-(6-chloronaphthalen-2-ylsulfonyl)-1-(3-pyridazinyl) spiro[piperidine-4,6'-tricyclo[6.4.0.0^{1,5}]dodecane]-4',9'-dione (Example 3);
(±)-3'-acetyl-3',5',8',11'-tetraaza-11'-(6-chloronaphthalen-2-ylsulfonyl)-1-(2-oxazolinyl) spiro[piperidine-4,6'-tricyclo[6.4.0.0^{1,5}]dodecan]-9'-one (Example 4);
(±)-3',5',8',11'-tetraaza-11'-(6-chloronaphthalen-2-ylsulfonyl)-3'-methyl-1-(4-pyridyl) spiro[piperidine-4,6'-tricyclo[6.4.0.0^{1,5}]dodecan]-9'-one (Example 5);

(-)-3',5',8',11'-tetraaza-11'-(6-chloronaphthalen-2-ylsulfonyl)-1-(5,6-dihydro-4H-1,3-oxazin-2-yl)-3'-methylspiro[piperidine-4,6'-tricyclo[6.4.0.0^{1,5}]dodecane]-4',9'-dione (Example 6);
(-)-3',5',8',11'-tetraaza-11'-(6-chloronaphthalen-2-ylsulfonyl)-1-(4,5-dihydroisoxazol-3-yl)-3'-methylspiro[piperidine-4,6'-tricyclo[6.4.0.0^{1,5}]dodecane]-4',9'-dione (Example 7);
(-)-3',5',8',11'-tetraaza-11'-(6-chloronaphthalen-2-ylsulfonyl)-1-(3,4,5,6-tetrahydropyridin-2-yl)-3'-methylspiro[piperidine-4,6'-tricyclo[6.4.0.0^{1,5}]dodecane]-4',9'-dione (Example 8);
(-)-1-acetyl-3',5',8',11'-tetraaza-11'-(6-chloronaphthalen-2-ylsulfonyl)-3'-methyl-spiro[piperidine-4,6'-tricyclo[6.4.0.0^{1,5}]dodecane]-4',9'-dione (Example 9);
(-)-3',5',8',11'-tetraaza-11'-(6-chloronaphthalen-2-ylsulfonyl)-3'-methyl-1-propionylspiro[piperidine-4,6'-tricyclo[6.4.0.0^{1,5}]dodecane]-4',9'-dione (Example 10);

(-)-3',5',8',11'-tetraaza-11'-(6-chlorobenzo[b]thiophen-2-ylsulfonyl)-3'-methyl-1-(2-oxazolinyl) spiro[piperidine-4,6'-tricyclo[6.4.0.0^{1,5}]dodecane]-4',9'-dione (Example 11);
(-)-3',5',8',11'-tetraaza-11'-(6-chlorobenzo[b]thiophen-2-ylsulfonyl)-1-(5,6-dihydro-4H-1,3-oxazin-2-yl)-3'-methylspiro[piperidine-4,6'-tricyclo[6.4.0.0^{1,5}]dodecane]-4',9'-dione (Example 12);
(-)-3',5',8',11'-tetraaza-11'-(6-chlorobenzo[b]thxophez-2-ylsulfonyl)-1-(4,5-dihydroisoxazol-3-yl)-3'-methylspiro[piperidine-4,6'-tricyclo[6.4.0.0^{1,5}]dodecane]-4',9'-dione (Example 13);
(-)-3',5',8',11'-tetraaza-11'-(6-chlorobenzo[b]thiophen-2-ylsulfonyl)-1-(3,4,5,6-tetrahydropyridin-2-yl)-3'-methylspiro[pipexi.dine-4,6'-tricyclo[6.4.0.0^{1,5}]dodecane]-4',9'-dione (Example 14);
(-)-1-acetyl-3',5',8',11'-tetraaza-11'-(6-chlorobenzo[b]thiophen-2-ylsulfonyl)-3'-methyl-spiro[piperidine-4,6'-tricyclo[6.4.0.0^{1,5}]dodecane]-4',9'-dione (Example 15);

(-)-3',5',8',11'-tetraaza-11'-(6-chlorobenzo[b]thiophen-2-ylsulfonyl)-3'-methyl-1-propionylspiro[piperidine-4,6'-tricyclo[6.4.0.0^{1,5}]dodecane]-4',9'-dione (Example 16); (-)-3',5',8',11'-tetraaza-11'-(5-chloro-1H-indol-2-ylsulfonyl)-3'-methyl-1-(2-oxazolinyl) spiro[piperidine-4,6'-tricyclo[6.4.0.0^{1,5}]dodecane]-4',9'-dione (Example 17);
(-)-3',5',8',11'-tetraaza-11'-(5-chloro-1H-indol-2-ylsulfonyl)-1-(5,6-dihydro-4H-1,3-oxazin-2-yl)-3'-methylspiro[piperidine-4,6'-tricyclo[6.4.0.0^{1,5}]dodecane]-4',9'-dione (Example 18);
(-)-3',5',8',11'-tetraaza-11'-(5-chloro-1H-indol-2-ylsulfonyl)-1-(4,5-dihydroisoxazol-3-yl)-3'-methylspiro[piperidine-4,6'-tricyclo[6.4.0.0^{1,5}]dodecane]-4',9'-dione (Example 19);
(-)-3',5',8',11'-tetraaza-11'-(5-chloro-1H-indol-2-ylsulfonyl)-1-(3,4,5,6-tetrahydropyridin-2-yl)-3'-methylspiro[piperidine-4,6'-tricyclo[6.4.0.0^{1,5}]dodecane]-4',9'-done (Example 20);

(-)-1-acetyl-3',5',8',11'-tetraaza-11'-(5-chloro-1H-indol-2-ylsulfonyl)-3'-methylspiro[piperidine-4,6'-tricyclo[6.4.0.0^{1,5}]dodecane]-4',9-dione (Example 21);
(-)-3',5',8',11'-tetraaza-11'-(5-chloro-1H-indol-2-ylsulfonyl)-3'-methyl-1-propionylspiro[piperidine-4,6'-tricyclo[6.4.0.0^{1,5}]dodecane]-4',9'-dione (Example 22);
(-)-3',5',8',11'-tetraaza-11'-[(E)-2-(5-chlorothiophen-2-yl)ethenesulfonyl]-3'-methyl-1-(2-oxazolinyl) spiro[piperidine-4,6'-tricyclo[6.4.0.0^{1,5}]dodecane]-4',9'-dione (Example 23);
(-)-3',5',8',11'-tetraaza-11'-[(E)-2-(5-chlorothiophen-2-yl)ethenesulfonyl]-1-(5,6-dihydro-4H-1,3-oxazin-2-yl)-3'-methylspiro[piperidine-4,6'-tricyclo[6.4.0.0^{1,5}]dodecane]-4',9'-dione (Example 24);
(-)-3',5',8',11'-tetraaza-11'-[(E)-2-(5-chlorothiophen-2-yl)ethenesulfonyl]-1-(4,5-dihydroisoxazol-3-yl)-3'-methylspiro[piperidine-4,6'-txicyclo[6.4.0.0^{1,5}] dodecane]-4',9'-dione (Example 25);

(-)-3',5',8',11'-tetraaza-11'-[(E)-2-(5-chlorothiophen-2-yl)ethenesulfonyl]-1-(3,4,5,6-tetrahydropyridin-2-yl)-3'-methylspiro[piperidine-4,6'-tricyclo[6.4.0.0^{1,5}]dodecane]-4',9'-dione (Example 26);
(-)-1-acetyl-3',5',8',11'-tetraaza-11'-[(E)-2-(5-chlorothiophen-2-yl)ethenesulfonyl]-3'-methylspiro[piperidine-4,6'-tricyclo[6.4.0.0^{1,5}]dodecane]-4',9'-dione (Example 27);
(-)-3',5',8',7.7.'-tetraaza-11'-[(E)-2-(5-chloxothiophen-2-yl)ethenesulfonyl]-3'-methyl-1-propionylspiro[piperidine-4,6'-tricyclo[6.4.0.0^{1,5}]dodecane]-4',9'-dione (Example 28);
(-)-3',5',8',11'-tetraaza-11'-(6-chloronaphthalen-2-ylsulfonyl)-3'-methyl-1-(2-oxazolinyl) spiro[piperidine-9,6'-tricyclo[6.4.0.0^{1,5}]dodecane]-4',9'-dione (Example 29);
(+)-3',5',8',11'-tetraaza-11'-(6-chloronaphthalen-2-ylsulfonyl)-3'-methyl-1-(2-oxazolinyl) spiro[piperidine-4,6'-tricyclo[6.4.0.0^{1,5}]dodecane]-4',9'-dione (Example 30);
(+)-1-acetyl-3',5',8',11'-tetraaza-11'-(6-chloronaphthalen-2-ylsulfonyl)-3'-methyl-spiro[piperidine-4,6'-tricyclo[6.4.0.0^{1,5}]dodecane]-4',9'-dione (Example 31)
their (+) and (-) optical isomers and
   pharmaceutically acceptable salts (such as hydrochlorides and methanesulfonates.

[1-12] More preferable examples of the compound of formula (I) include the compounds represented by formula (Im): 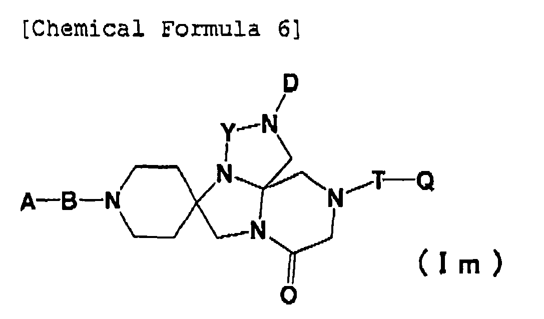 wherein Q, T, A, B, Y, and D are as defined above in [1-1] to[1-9], and any combination of Q, T, A, B, Y, and D as defined above in [1-1] to[1-9] may be employed. Their specific combinations are as illustrated in [1-10] and preferable compounds are described in [1-11].

[2] A second aspect of the present invention provides a pharmaceutical composition characterized by its inclusion of the compound represented by formula (I) or (Im), the compound described in the above [1-1] to [1-12], or a pharmaceutically acceptable salt thereof as an effective component.
[2-a] more specifically, the pharmaceutical composition is
1) an anticoagulant; or a prophylactic and/or therapeutic agent for diseases induced by thrombosis or embolism;
2) a prophylactic and/or therapeutic agent for diseases wherein an anticoagulant is effective; or a prophylactic and/or therapeutic agent for diseases wherein inhibition of FXa is effective;
3) a prophylactic agent against embolism associated with atrial fibrillation/artificial valve or valvular heart disease (and preferably, a prophylactic agent against the onset of cerebral embolism associated with these diseases); or a prophylactic and/or therapeutic agent for (and in particular, a prophylactic agent against the recurrence of) transient ischemic attack; or
4) a prophylactic and/or therapeutic agent for DIC; a prophylactic and/or therapeutic agent for influenza virus infection; or a prophylactic and/or therapeutic agent for deep vein thrombosis.
[2-b] The second aspect of the present invention also provides a prophylactic and/or therapeutic process in which an effective amount of the pharmaceutical composition is administered to patients suffering from the diseases as mentioned above.
[2-c] The second aspect of the present invention also provides use of the compound represented by formula (I) or (Im), the compound described in the above [1-1] to [1-12], or a pharmaceutically acceptable salt thereof for producing a drug for preventing and/or treating the diseases as mentioned above.
The pharmaceutical composition of the present invention contains at least one compound represented by formula (I) or (Im) (definition of the formula is as described above) or its salt as its effective component, and it may also contain a pharmaceutically acceptable carrier. Preferable examples of the compound of formula (I) or (Im) are as described in the above [1-1] to [1-12].

[3] A third aspect of the present invention provides an FXa inhibitor containing the compound represented by formula (I) or (Im), the compound described in the above [1-1] to [1-12], or a pharmaceutically acceptable salt thereof; and
[3-a] More specifically, the third aspect of the present invention relates to a specific FXa inhibitor containing the compound represented by formula (I) or (Im), the compound described in the above [1-1] to [1-12], or a pharmaceutically acceptable salt thereof as its effective component; and also, to an orally administerable FXa inhibitor, and an orally administerable specific FXa inhibitor.
[3-b] The third aspect of the present invention also provides a reagent using the compound represented by formula (I) or (Im), the compound described in the above [1-1] to [1-12], or a pharmaceutically acceptable salt thereof. Exemplary such reagents include a reagent which diagnoses abnormal blood coagulability of a mammal by using the FXa inhibitory action, and a reagent used in physiological experiments which uses quantitative FXa inhibitory action.
[3-c] The third aspect of the present invention also provides a prophylactic and/or therapeutic method in which an effective amount of the FXa inhibitor is administered to a patient.
[3-d] The third aspect of the present invention also provides use of the compound represented by formula (I) or (Im), the compound described in the above [1-1] to [1-12], or a pharmaceutically acceptable salt thereof for producing the FXa inhibitor.

[4] The fourth aspect of the present invention is a compound represented by formula (X), or a salt or a solvate thereof 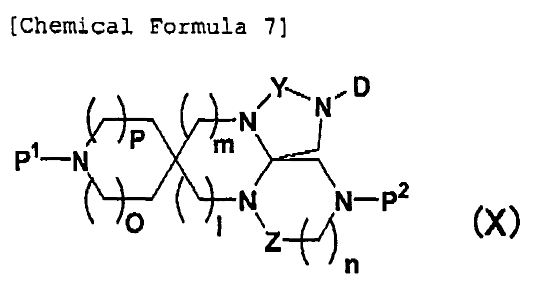 wherein P¹ and P² each independently represent hydrogen atom or protective group of imino group (-NH-), and D, Y, Z, l, m, n, o, and p are as defined above for formula (I).

[5] The aspect of the present invention is a compound represented by formula (XI), or a salt or a solvate thereof 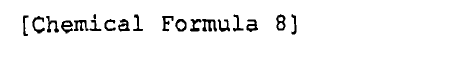 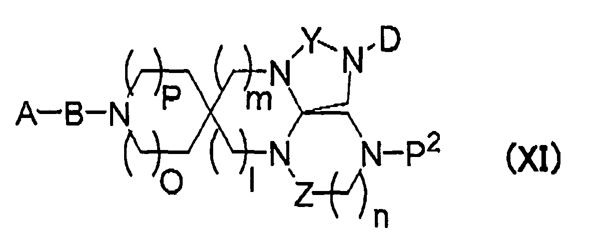 wherein P² represents hydrogen atom or protective group of imino group (-NH-), and A, B, D, Y, Z, l, m, n, o, and p are as defined above for formula (I).

[6] The sixth aspect of the present invention is a compound represented by formula (XII), or a salt or a solvate thereof 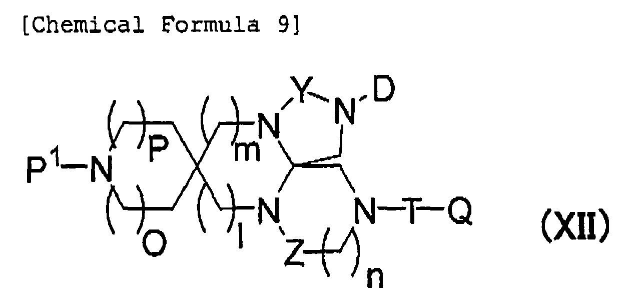 wherein P¹ represents hydrogen atom or protective group of imino group (-NH-), and D, T, Q, Y, Z, l, m, n, o, and p are as defined above for formula (I).

In all of the aspects as described above, the expression "compound" should be deemed to also include "the pharmaceutically acceptable salts thereof". The compound of the present invention may include an asymmetric carbon, and the compound of the present invention may be a mixture or an isolation product of geometric isomer, tautomer, optical isomer or other stereoisomer. Isolation or purification of such stereoisomer may be accomplished by those skilled in the art using any of the techniques commonly used in the art, for example, by optical resolution using preferential crystallization or column chromatography, or by asymmetric synthesis.

The compound (I) of the present invention may be in the form of an acid addition salt, and depending on the type of the substituent, the compound (I) may also be in the form of a salt with a base. Such salt is not particularly limited as long as the salt is a pharmaceutically acceptable salt, and exemplary salts include acid addition salts with a mineral acid such as hydrochloric acid, hydrobromic acid, hydroiodic acid, sulfuric acid, nitric acid, or phosphoric acid; an organic carboxylic acid such as acetic acid, propionic acid, oxalic acid, malonic acid, succinic acid, fumaric acid, maleic acid, lactic acid, formic acid, malic acid, tartaric acid, citric acid, or mandelic acid; an organic sulfonic acid such as methanesulfonic acid, ethanesulfonic acid, p-toluenesulfonic acid, or 2-hydroxyethanesulfonic acid; or acidic amino acid such as aspartic acid or glutamic acid; and salts with a base of an alkaline metal or alkaline earth metal such as sodium, potassium, magnesium, calcium, or aluminum and an organic base such as methylamine, ethylamine, ethanolamine, pyridine, lysine, arginine, or ornithine; and ammonium salt.

Furthermore, the salts of the compound of the present invention also include mono-salts, di-salts, and tri-salts. Still further, the compound of the present invention may simultaneously form an acid addition salt and a salt with a base depending on the type of the substituent on the side chain.
Still further, the present invention also includes hydrates of the compound (I) as well as pharmaceutically acceptable solvates and crystalline polymorphic forms of the compound (I). It should also be taken for granted that the present invention is by no means limited to the compounds mentioned in the Examples as described below, and all of the tetracyclic compounds having the spiro union as represented by formula (I) and their pharmaceutically acceptable salts are within the scope of the present invention.

The compound of the present invention also includes all "prodrugs" which are compounds converted in vivo to the compound of the present invention. The method for producing the prodrug of the present compound is described, for example, in "Current Drug Design Chemistry" Volume 2/2, pp. 271-319, 1999 (Technomics, Inc.) and the groups used in such method are described, for example, in "Development of Pharmaceuticals" vol. 7, pp. 163-198, 1990 (Hirokawa Shoten).
Exemplary such prodrugs include N-alkylated compound for the NH group, and acetal compound and oxime compound for the carbonyl group. The prodrugs are not limited to such compounds, and also included are the case in which the compound of the present invention functions as a prodrug.
It would be readily understood that such situation also applies to the compounds of formula (Im).

Next, the therapeutic and/or prophylactic agent and the pharmaceutical composition of the present invention are described. The pharmaceutical composition of the present invention contains at least one compound represented by formula (I) or (Im) (definition of the formula is as described above) as its effective component, and it may also contain a pharmaceutically acceptable carrier. Preferable examples of the compound of formula (I) are as described above.

### <FXa inhibitory action of the compound of the present invention>

The compound of the present invention has strong FXa inhibitory activity. In other words, the composition of the present invention is a strong FXa inhibitor, and more specifically, a FXa inhibitor which can be orally administered and which does not at all inhibit trypsin, chymotrypsin, or thrombin which is a serine protease in the same blood coagulation system. This has enabled to overcome the problems such as bleeding tendency associated with the use of a thrombin inhibitor. Still further, the compound of the present invention exhibits good absorption from digestive tracts after oral administration with no reduction in its activity by the absorption, and it also exhibits favorable absorption, distribution, metabolism, and excretion characteristics. Its value as an orally administerable agent is quite high.

The composition containing the compound of the present invention is a prophylactic and/or therapeutic agent for diseases wherein an FXa inhibitor is useful. The composition containing the compound of the present invention is also an anticoagulant which is a prophylactic and/or therapeutic agent for diseases wherein the anticoagulant is useful. To be more specific, such agent is effective in prevention and/or treatment of diseases caused by thrombus or embolus. Specific examples of such diseases include: diseases from ischemic cerebrovascular disorders such as cerebral thrombosis, brain infarction, cerebral embolism, transient cerebral ischemic attack (TIA) and cerebrovascular spasm after subarachnoid hemorrhage; Alzheimer's disease, cerebrovascular dementia, asymptomatic cerebrovascular disorder, diseases associated with ischemic heart diseases such as acute and chronic myocardial infarction, sequelae of myocardial infarction, unstable angina pectoris, angina pectoris and coronary artery thrombolysis; thrombogenesis after artificial blood vessel or artificial valve replacement, reocclusion and restenosis after coronary artery bypass grafting, reocclusion and restenosis after PTCA or PTCA or stent placement, pulmonary infarction, pulmonary thrombosis/pulmonary embolism, diseases associated with pulmonary vascular disorder (for example, drug-induced pneumonia), acute respiratory distress syndrome (ARDS), acute nephritis, acute progressive nephritis, chronic nephritis (for example, diabetic nephropathy, chronic glomerulonephritis, and IgA nephropathy), acute arterial occlusion, thromboangiitis obliterans (Buerger's disease), arteriosclerosis obliterans, peripheral arterial occlusive disease, peripheral venous occlussive disease, deep vein thrombosis, thrombophlebitis, disseminated intravascular coagulation (DIC), organ failures induced with the progress of the shock or DIC, thrombotic microangiopathy (TMA), systemic inflammatory response syndrome (SIRS), thrombotic thrombocytopenic purpura, hemolytic uremic syndrome (HUS), diseases associated with various vascular disorders such as thrombogenesis in extracorporeal circulation, thrombocytopenia in major operation, arterial sclerosis, cancer metastasis, rejection in transplantation, and organ protection or functional improvement in transplantation. Also included are prophylaxis of vascular endothelial cell injury associated with diabetes, hypercoagulability associated with transplantation or activated protein C (APC) resistance, excessive blood coagulation associated with vascular disease, injury after operation, obesity, pregnancy, use of oral contraceptive, sustained depression, heparin-induced thrombocytopenia, collagen disease (for example, antiphospholipid antibody syndrome, polyarteritis, and systemic lupus erythematosus), Bechet's disease, ischemic reperfusion injury, cancer or the like, and toxemia of pregnancy.
The agent of the present invention is particularly adapted for use in prevention of embolism associated with atrial fibrillation/artificial valve or valvular heart disease, and preferably for prevention of onset of cerebral embolism, prevention of transient cerebral ischemic attack and especially for prevention of recurrence of the transient cerebral ischemic attack, and prevention/treatment of deep vein thrombosis or DIC.

When the agent of the present invention is used as a drug for these diseases, preventive administration is recommended and such use is particularly important since the agent of the present invention is neither a direct thrombolytic agent nor a direct platelet aggregation inhibitor. In other words, the agent of the present invention is adapted for preventive use in patients suffering from thrombophilia or patients having the risk factor of thrombosis/embolism for the purpose of preventing thrombus/embolus. In the case of the patients with atrial fibrillation/artificial valve or valvular heart disease, a thrombus is easily generated at the site of the lesion or the transplantation, and such thrombus often triggers cerebral infarction, which is more than often a fatal attack. The agent of the present invention has a good potential to be a potent drug for preventing onset of the thrombosis/embolism, and in particular, cerebral embolism induced in such patients.

Such therapy is continued for a long time. The agent of the present invention can be orally administered with fewer side effects such as bleeding, and therefore, the agent of the present invention can be reliably used for a long time with no need of frequent monitoring.
In other words, the agent of the present invention is a prophylactic and/or therapeutic agent for embolism associated with atrial fibrillation/artificial valve or valvular heart disease. The agent of the present invention is preferably a prophylactic agent against the onset of cerebral embolism associated with such disease. The agent of the present invention is also a prophylactic and/or therapeutic agent for transient cerebral ischemic attack, and in particular, a prophylactic agent for preventing the recurrence of the onset of transient cerebral ischemic attack; and a prophylactic and/or therapeutic agent for deep vein thrombosis or DIC.
In addition, some compounds of the present invention are easily metabolized in the course of the absorption and secretion of the pharmaceutical substance by the substituent in D, and some of the thus produced metabolites are within the scope of the compound of the present invention as represented by formula (I), and exhibit a potent FXa inhibitory activity. This is a finding quite interesting in pharmacological/pharmacokinetical point of view.

The composition containing the compound of the present invention as an active ingredient is also effective as a veterinary drug and has high value of use. The composition is also useful as a reagent adapted for use in measuring various blood coagulation functions and as a laboratory reagent.
Owing to the FXa inhibitory action of the compound of the present invention, such composition is also useful as a prophylactic/therapeutic agent against infection with influenza virus based on the inhibitory activity against the propagation of the influenza virus, and also, as a prophylactic/therapeutic agent against periodontal disease.

[Production method of the compound of the present invention]
Next, the production method for the derivative of formula (I) of the present invention is described.
The derivative of formula (I) and the salt and solvate thereof according to the present invention can be produced by a combination of chemical processes commonly known to the art, and in particular, by the processes similar to those described in WO 01/02397 and WO 02/053568. Typical production methods are as described below.
Unless otherwise noted, A, B, D, T, Q, Y, Z, l, m, n, r, o, and p in the formulae shown in the description of the production method are as defined above for formula (I). The alkylene group in the side chain or ring of the compound may be substituted with the substituents defined for formula (I).

Unless otherwise noted, P¹ and P² in the production method each independently designate hydrogen atom or protecting group of the imino group (-NH-), and exemplary appropriate protecting groups include typical acyl protecting groups, namely, an alkanoyl group such as acetyl group; an alkoxycarbonyl group such as methoxycarbonyl group, ethoxycarbonyl group, or t-butoxycarbonyl group; an arylmethoxycarbonyl group such as benzyloxycarbonyl group, paramethoxybenzyloxycarbonyl group, or para(ortho)nitrobenzyloxycarbonyl group; an arylmethyl group such as benzyl group or triphenylmethyl group; or an aroyl group such as benzoyl group. The method used for deprotecting such protecting group differs depending on the chemical nature of the protecting group employed, and in the case of an acyl protecting group such as an alkanoyl group, an alkoxycarbonyl group, or aroyl group, the deprotection can be accomplished by the hydrolysis using an appropriate base such as an alkaline metal hydroxide such as lithium hydroxide, sodium hydroxide, or potassium hydroxide.

The substituted methoxycarbonyl protecting group such as t-butoxycarbonyl group or paramethoxybenzyloxycarbonyl group can be removed by an appropriate acid such as acetic acid, hydrochloric acid, hydrobromic acid, sulfuric acid, phosphoric acid, trifluoroacetic acid, trifluoromethanesulfonic acid, or a combination thereof. The arylmethoxycarbonyl group such as benzyloxycarbonyl group, paramethoxybenzyloxycarbonyl group, or para(ortho)nitrobenzyloxycarbonyl group and the arylmethyl group such as benzyl group can be removed by the hydrolysis using a palladium-carbon catalyst. The benzyl group can be removed by Birch reduction using metal sodium in liquid ammonia or by using chloroformate ester, for conversion into nitrogen-hydrogen bond. The triphenylmethyl group can be removed by using an appropriate acid such as acetic acid, hydrochloric acid, hydrobromic acid, sulfuric acid, phosphoric acid, trifluoroacetic acid, trifluoromethanesulfonic acid, or a combination thereof, or alternatively, by Birch reduction using metal sodium in liquid ammonia, or hydrolysis using a palladium-carbon catalyst.

The protecting groups of the imino group (-NH-) in P¹ and P² can be independently or simultaneously deprotected by adequately selecting the type of the protecting group and deprotection conditions, and if desired, the protecting group can be re-introduced.
Unless otherwise noted, "W" in the production method designates a leaving group such as a halogen atom (for example, fluorine, chlorine, bromine, or iodine), methanesulfonyloxy group, p-toluenesulfonyloxy group, or nitro group, or a replaceable substituent such as hydroxy group or an alkoxy group.

It should be noted that, when the derivative of formula (I) of the present invention synthesized has a reactive group such as hydroxy group, amino group, carboxyl group, or thiol group as its substituent, such group may be adequately protected with a protective group in each reaction step and the protective group may be removed at an adequate stage. The process of such introduction and removal of the protective group may be adequately determined depending on the group to be protected and the type of the protective group, and such introduction and removal are conducted, for example, by the process described in the review section of "Protective Groups in Organic Synthesis", Second edition, 1991, John Wiley & Sons, Inc. The required starting materials are either commercially available, or capable of being readily synthesized by the method commonly used in the organic chemistry from commercially available products. Unless otherwise noted, the reaction conditions employed in the production method are as described below: Reaction temperature is in the range of -78°C to the solvent-reflux temperature, and the reaction time is the time sufficient for required progress of the reaction. Solvent which is not involved in the reaction may be any of the aromatic hydrocarbon solvents such as toluene and benzene; polar solvents such as water, methanol, DMF, and DMSO; basic solvents such as triethylamine and pyridine; halogen solvents such as chloroform, methylene chloride, and 1,2-dichloroethane; ethereal solvents such as diethylether, tetrahydrofuran, and dioxane; and mixed solvents thereof; and the solvent used may be adequately selected depending
on the reaction conditions. Base may be any of inorganic bases such as potassium carbonate, cesium carbonate, sodium hydroxide, potassium hydroxide, and sodium hydride; and organic bases such as triethylamine, pyridine, N,N-dialkylaniline, and lithium diisopropylamide; and acid may be any of mineral acids such as hydrochloric acid, and sulfuric acid; and organic acids such as methanesulfonic acid and p-toluenesulfonic acid. The base and the acid are not necessarily limited to those mentioned above.

Next, the production method is described. The present invention, however, is by no means limited to the processes as described below.
(1) Next, the method for producing the derivative of formula (I) is described.
It is to be noted that "the compound represented by formula (I)" includes not only the compound represented by formula (I) but also the salts and the solvates thereof.

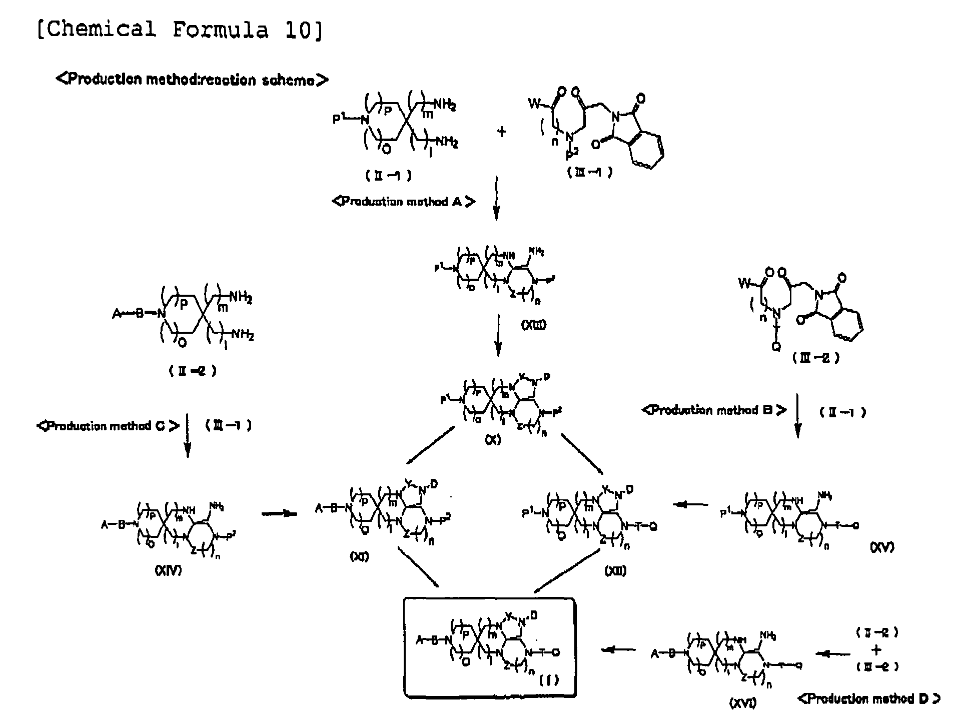

The compounds represented by formula (XIII) or a salt
or a solvate thereof which is a universal intermediate of the present compound may be prepared by combining known chemical production methods commonly used in the art, and in particular, by the method disclosed in the WO01/02397 or WO02/053568.

### <Production method A>

### (Step a)

In this step, the compounds represented by formula (II-1) and formula (III-1):

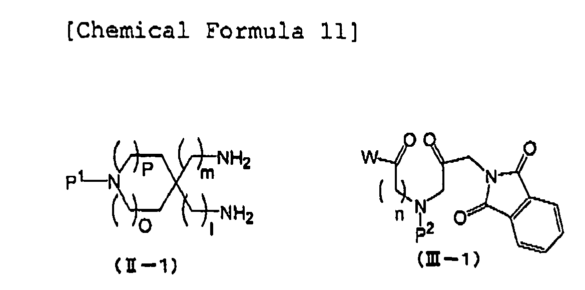

(wherein P¹, P², l, m, n, o, and p, and substitution of each alkylene chain are as defined above) or their salts which are commercially available or readily derived from commercially available compounds may be reacted in accordance with a known process described in a document (for example, Journal of Medicinal Chemistry, vol. 19, page 436, 1976; Journal of American Chemical Society, vol. 107, page 7776, 1985; or Journal of Organic Chemistry, vol. 63, page 1732, 1998) preferably by using toluene for the solvent in the presence or absence of an acid catalyst, and preferably, in the presence of p-toluenesulfonic acid. The reaction may be promoted at a temperature in the range of 0°C to the solvent reflux temperature, and preferably at the solvent reflux temperature for a time sufficient for the progress of the required reaction, and preferably for 2 to 6 hours; and after optional reduction or thiocalbonylation reaction, the phthalimide is deprotected by the method commonly used in the art as described in WO02/053568 to produce the compound represented by formula (XIII) or its salt

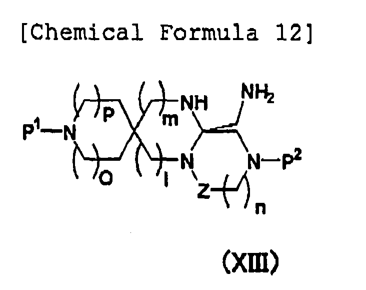

(wherein P¹, P², Z, l, m, n, o, and p, and substitution of each alkylene chain are as defined above).

### (Step b)

In this step, the compound (XIII) or its salt is optionally subjected to protection of the primary amino group (NH₂ group) with an appropriate protecting group, preferably nosyl group, then is subjected to alkylation with D-W (wherein D is an optionally substituted C₁₋₆ alkyl group), and deprotection by a method adequately selected depending on the type of the protecting group used for the protection to thereby convert the primary amino group into substituted secondary amino group. The resulting compound is used in the subsequent reaction. In an embodiment, this conversion to the secondary amino group is accomplished by subjecting the compound of formula (XIII) to o-nitrobenzenesulfonylation (hereinafter referred to as "protection with Ns") using o-nitrobenzenesulfonyl chloride in the presence of triethylamine by the method commonly used in the art, and alkylation by a method commonly used in the art using a halogenated alkyl. The protection with Ns is preferably conducted at a reaction temperature of 0°C to room temperature for a reaction time of preferably 1 to 3 hours. The alkylation is preferably conducted at a reaction temperature of 0°C to room temperature for a reaction time of preferably 1 to 3 hours. Next, Ns is removed for deprotection from the compound of formula (XIII) by a method commonly used in the art using thiophenol under basic condition to thereby produce the compound of formula (XIII) wherein the primary amino group has been alkylated. The deprotection by Ns removal is preferably conducted at a reaction temperature of 0°C to room temperature for a reaction time of preferably 1 to 3 hours.

The compound (XIII) or its salt produced in the step "a" with no further processing or the compound (XIII) or its salt wherein the primary amino group has been converted to the secondary amine by the method described above is then reacted with phosgene or related compound (e.g., triphosgene), urea, 1,1-carbonyl diimidazole (CDI), a carbonate compound (e.g., diethyl carbonate, diphenyl carbonate, dimethyl carbonate, 2-oxo-1,3-dioxolane, or S,S-dimethyl dithiocarbonate), carbon dioxide, carbon monoxide, hexachloroacetone, ethyl heptafluorobutylate, a halogenated formate (e.g., methyl chloroformate, ethyl chloroformate, phenyl chloroformate, or 4-nitrophenyl chloroformate), or the like under neutral or basic conditions for intramolecular condensation cyclization to thereby produce cyclic urea compound of formula (X) (wherein Y is carbonyl). In some cases, however, this reaction is accomplished by adding a metal catalyst such as zinc or tungsten. The reaction is conducted at a temperature of 0°C to the refluxing temperature of the solvent, and optionally with pressurization in gas-tight conditions. The reaction time is preferably in the range of 30 minutes to several hours, and the reaction is preferably conducted in basic conditions, and in particular, in the presence of triethylamine, by treating with triphosgene to thereby produce the compound represented by formula (X) (Y= carbonyl). The reaction is preferably conducted in an ice bath for a reaction time of 30 minutes.

Alternatively, the compound (XIII) or its salt produced in the step "a" with no further processing or the compound (XIII) or its salt wherein the primary amino group has been converted to the secondary amine by the method described above may be treated with paraformaldehyde or formaldehyde by using a solvent which is preferably methanol in basic condition preferably in the presence of sodium methoxide to produce the compound represented by formula (X) (Y= methylene) or its salt.

### (Step c)

Protecting group P¹ of the compound represented by formula (X) or its salt is deprotected by the method appropriate for the type of the protecting group, and the deprotected product is then reacted with a compound represented by formula (VIII-1) or (VIII'-1): 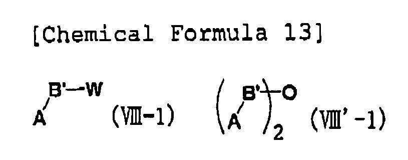 (wherein A and W are as defined above, and B' is carbonyl group) or its salt which is commercially available or readily derived from a commercially available compound for formation of an amide to thereby produce the compound represented by formula (XI) or its salt. When W is a halogen atom, hydroxyl group, or an alkoxy group in the compound represented by formula (VIII-1), amidation in normal peptide is carried out. For example, when W is hydroxyl group, a phenol such as 2,4,5-trichlorophenol, pentachlorophenol, 2-nitrophenol, or 4-nitrophenol, or a N-hydroxy compound such as N-hydroxysuccinimide, N-hydroxy-5-norbornene-endo-2,3-dicarboxyimide, or N-hydroxypiperidine is condensed in the presence of a condensing agent such as N,N-dicyclohexylcarbodiimide for conversion into active ester form, and allowed for reaction. Alternatively, the reaction may be conducted after producing a mixed acid anhydride by reacting with a halogenated acyl compound such as isobutyl chloroformate. The reaction may be also promoted by using a peptide condensation reagent such as N,N-dicyclohexylcarbodiimide, diphenylphosphoric acid azide or diethyl cyanophosphate alone.

Alternatively, sulfonamidation may be conducted with the compound represented by formula (VIII-2): 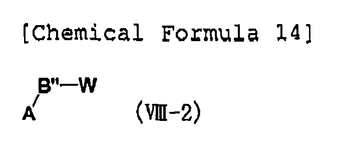 (wherein A and W are as defined above, and B" is sulfonyl group) or its salt which is commercially available or readily derived from a commercially available compound to thereby produce the compound represented by formula (XI) or its salt.

Also a bond may be formed with the compound represented by formula (VIII-3): 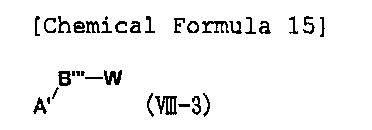 (wherein A' is an alkyl derivative having isocyanate at its end, W is as defined above, and B" is single bond) or its salt which is commercially available or readily derived from a commercially available compound to thereby produce the compound represented by formula (XI) or its salt.

Also a coupling may be conducted with the compound represented by formula (VIII-4): 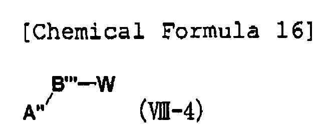 (wherein A" is any saturated or unsaturated cyclic compound, W is as defined above, and B"' is single bond) or its salt which is commercially available or readily derived from a commercially available compound to thereby produce the compound represented by formula (XI) or its salt.

### <Step d>

In this step, protecting group P² of the compound represented by formula (XI) or its salt is removed by the method adequately selected depending on the type of the protecting group, and after the deprotection, condensation is conducted by using the compound represented by formula (VII): 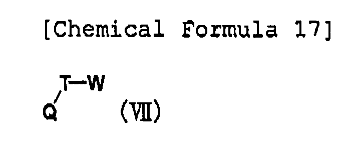 (wherein W, Q, and T are as defined above) or its salt which is commercially available or readily derived from a commercially available compound to thereby produce the compound represented by formula (I) or its salt. For example, the reaction is conducted in methylene chloride and in the presence of triethylamine when T is sulfonyl group and W is chlorine atom.

The compound represented by formula (I) or its salt can also be produced by deriving the compound represented by formula (XII) from the compound represented by formula (X) or its salt by the step d, and then conducting the protection and deprotection of step c.

### <Production method B>

The compound represented by formula (XII) in the reaction scheme as described above may also be produced by the method as described below.

### (Step a)

The compounds represented by formulae (II-1) and (III-2): 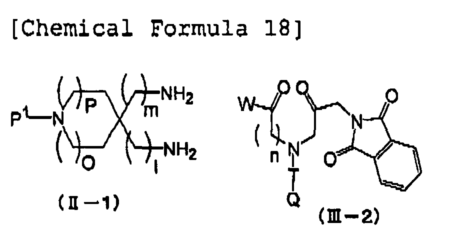 (wherein P¹, T, Q, 1, m, n, o, and p, and substitution of the alkylene chain are as defined above) or its salt which is commercially available or readily derived from a commercially available compound may be used to produce the compound represented by formula (XV): 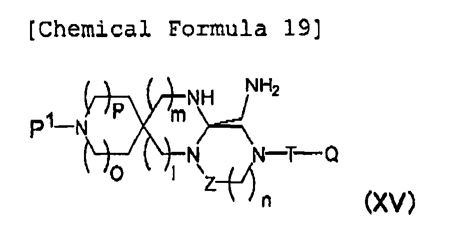 (wherein P¹, T, Q, Z, l, m, n, r, o, and p, and substitution of each alkylene chain are as defined above) or its salt by the same method as the <Production method A>, step "a".

### (Step b)

The compound represented by formula (XII) may be produced by the method described in the <Production method A>, step b by using the compound represented by formula (XV).

### <Production method C>

The compound represented by formula (XI) in the reaction scheme as described above may also be produced by the method as described below.

### (Step a)

The compounds represented by formulae (II-2) and formula (III-1): 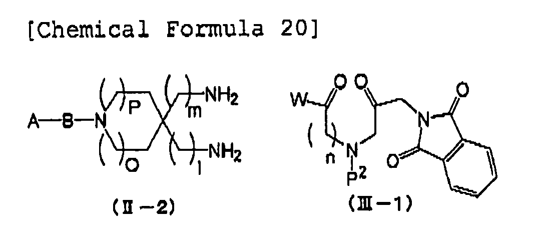 (wherein P², A, B, l, m, n, o, and p, and substitution of each alkylene chain are as defined above) or its salt which is commercially available or readily derived from a commercially available compound may be used to produce the compound represented by formula (XIV): 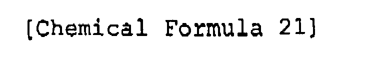 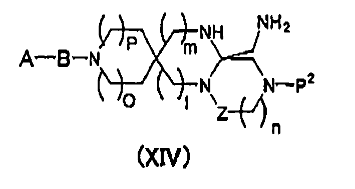 (wherein P², A, B, Z, l, m, n, r, o, and p, and substitution of each alkylene chain are as defined above) or its salt by the same method as the <Production method A>, step "a".

### (Step b)

The compound represented by formula (XI) can be produced by the method described in the <Production method A>, step b by using the compound represented by formula (XIV).

### <Production method D>

The compound represented by formula (I) in the reaction scheme as described above may also be produced by the method as described below.

### (Step a)

The compounds represented by formulae (II-2) and (III-2): 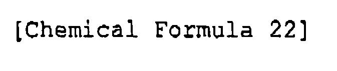 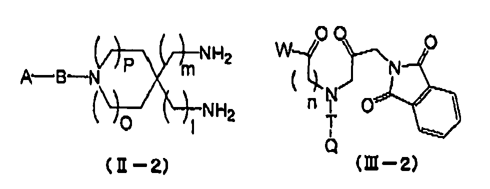 (wherein A, B, T, Q, l, m, n, o, and p, and substitution of each alkylene chain are as defined above) or its salt which is commercially available or readily derived from a commercially available compound may be used to produce the compound represented by formula (XVI): 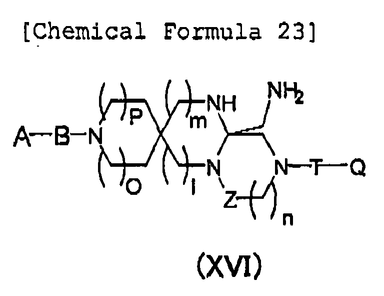 (wherein A, B, T, Q, Z, l, m, n, r, o, and p, and substitution of each alkylene chain are as defined above) or its salt by the same method as the Production method A>, step "a".

### (Step b)

The compound represented by formula (I) can be produced by the method described in the <Production method A>, step b by using the compound represented by formula (XVI).

Various functional groups may be protected and deprotected at adequate stages in each reaction step during the production of the compound of the present invention. Such protection and deprotection may be accomplished by introducing and removing an adequate protecting group by the method adequately selected depending on the type of the functional group protected and the protecting group used for the protection. The protection and the deprotection may be carried out, for example, by the method described in the review section of "Protective Groups in Organic Synthesis", second edition, 1991, John Wiley & Sons, Inc.
Furthermore, geometric isomer, tautomer, optical isomer, and other stereoisomers may be present for the compound of the present invention. These isomers and mixtures thereof are within the scope of the present invention. Isolation or purification of such stereoisomer may be accomplished by any of the separation/purification techniques commonly used in the art, for example, recrystalization and various chromatographic processes. It is also possible to selectively produce such isomer, for example, by asymmetric synthesis.
Next, the present invention is further described by referring to Experimental Examples and Examples which by no means limit the scope of the present invention.

### [Experimental Examples]

Excellent FXa inhibitory activity of the compounds of the present invention is confirmed by the test as described below.

### 1) Measurement of the enzyme inhibitory action

### a) Measurement of the human FXa inhibitory action

In vitro FXa inhibitory activity may be measured according to the method of Kettner et al. (Journal of Biological Chemistry, vol. 265, pages 18289 to 18297, 1990). To be more specific, human FXa (product of Enzyme Research Laboratories, Inc., 0.019 U/ml) is mixed with the specimens (of the compound of the present invention) prepared by diluting the compound of the invention with dimethylsulfoxide (DMSO) to different concentrations and synthetic substrate S-2222 (Chromogenix AB, 0.4 mM), and the mixtures are incubated at 37°C in Tris-hydrochloric acid buffer (pH 7.5). The FXa inhibitory activity of the specimen is calculated by measuring the absorbance at 405 nm.
It should be noted that the FXa inhibitory activity of the specimen is generally indicated as IC₅₀.
When the compound of the present invention is evaluated for its FXa inhibitory activity by the procedure as describe above, the strength is in the range of 0.1 nM to 0.5 µM and preferably 0.1 nM to 50 nM in terms of IC₅₀. Table 1 shows typical measurements.

[Table 1]

**Table 1**

| Example No. of the compound | IC₅₀ (µM) |
|---|---|
| Example 2 | 0.0048 |
| Example 3 | 0.0041 |
| Example 5 | 0.0040 |
| Example 11 | 0.0047 |
| Example 14 | 0.0036 |
| Example 17 | 0.0004 |
| Example 18 | 0.0043 |
| Example 20 | 0.0030 |
| Example 22 | 0.0040 |
| Example 29 | 0.0024 |

### 2) Measurement of Anticoagulant Activity (in Vitro) Measurement of Intrinsic Coagulation Time

Activated partial thromboplastin time (APTT) is measured in the presence of the test compounds of the present invention diluted at various concentrations. To be more specific, each of the test compounds diluted with DMSO at various concentrations is mixed with human plasma and APTT reagent. The mixture is incubated at 37°C for 2 minutes; calcium chloride (25 mM) is added to the mixture; and the coagulation time is thereafter measured. It should be noted that the anticoagulant activity of the test compound is described in terms of the concentration required to double the coagulation time for the case where no test compound is added. In this test, the compounds of the present invention were found to be effective in extending the APTT. The effects of the compounds of the present invention are shown in Table 2.

[Table 2]

**Table 2**

| Example No. of the compound | Concentration when APTT was doubled (µM) |
|---|---|
| Example 8 | 0.27 |
| Example 12 | 0.34 |
| Example 14 | 0.24 |
| Example 18 | 0.26 |
| Example 20 | 0.24 |

### 3) Characteristics of Anticoagulant Activity (ex vivo)

### a) Ex Vivo Measurement of Coagulation Time in Rats (i.v.)

Male Wistar rats (200 g to 300 g; Japan SLC Inc.) that have been fasting for more than 12 hours are administered through a femoral vein with a single dose (3 to 30 mg/kg) of a drug (compound of the present invention) dissolved in physiological saline (or 10% DMSO solution), and blood is collected at certain time intervals (3.8% sodium citrate (1/10 volume)), and plasma is then separated by centrifugation at 3000 rpm for 10 minutes. Prothrombin time (PT) is measured by the procedure as described below by using the separated plasma.
50 µl of the plasma is incubated at 37°C for 3 minutes and 100 µl of thromboplastin solution is added to start coagulation. The coagulation time is measured. In the actual test, the intravenously administered compounds of the present invention are found to be effective in extending the PT on account of enzyme inhibition.

### b) Ex Vivo Measurement of Coagulation Time in Rats (p.o.)

The test compound is compulsorily administered by oral administration using a feeding needle instead of the administration from the femoral vein at a single dose in the test a), and a certain volume of blood is collected at certain time intervals so as to contain 3.8% sodium citrate (1/10 volume). The blood is evaluated by the procedure as described in a) for extrinsic coagulation time and intrinsic coagulation time.
In this test b), the compounds of the present invention are found to be effective in extending the coagulation time upon oral administration of 10 to 100 mg/kg.
It should be noted that no abnormality in the aspect of safety is observed in the ex vivo test of the rats.

### 4) Evaluation of hERG inhibition by Rb efflux

HEK cells expressing hERG (human ether-a-go-go) is inoculated in a 96 well plate, and the cells are incubated for about 24 hours. After removing the culture medium by using a washing buffer, K⁺ channel open buffer containing the test substance (the compound of the present invention) is added. After incubating at 37°C for 3 hours, the K⁺ channel open buffer is replaced with Rb⁺ Load buffer containing the test substance, and the incubation at 37°C is continued for another 3 hours for incorporation of the Rb⁺ in the cell. After washing with the washing buffer containing the test substance, K⁺ channel open buffer containing the test substance is added, and the incubation at 37°C is continued for 5 minutes for release of the Rb⁺ in the cell to the exterior of the cell. After transferring the supernatant to a different plate, the cells are lysed, and the cell lysate is transfered to a different plate. The supernatant and the cell lysate are evaluated for their Rb⁺ content in order to calculate hERG inhibition rate. Inhibition rate (%) of the test substance (25 mg/mL) is shown in Table 3. In this test, the compound of the present invention showed an inhibition rate of not more than 50% at 50 µM.

[Table 3]

**Table 3**

| Example No. of the compound | hERG inhibition rate (%) |
|---|---|
| Example 20 | 5 |
| Example 26 | 6 |

As described above, the compound of the present invention has strong inhibition of the FXa activity, and exhibits antithrombotic action when it is orally administered to rats at 0.1 to 10 mg/kg, or intravenously administered at 0.01 to 1 mg/kg.

The compound of the present invention has an FXa inhibitory activity (IC₅₀) of 0.1nM to 50µM, and it is highly adapted for oral administration with adequate sustainability of the action. In the meanwhile, the compound of the present invention does not exhibit extension of bleeding time when it is orally administered to rats at a dose of 10 mg/kg or intravenously administered at a dose of 1 mg/kg. Accordingly, the compound of the present invention exhibits anticoagulation activity with no risk of bleeding tendency, and this is the difference from the known anticoagulants such as heparin and warfarin. In addition, since the IC₅₀ of hERG inhibition is higher than 50µM, a discrepancy of at least 100 folds is expected for the effective dose required for QT prolongation in the preferable case, and therefore, the compound of the present invention is highly safe.

The compounds of the present invention may be administered for the disease as described above which is to be prevented or treated by the present invention either alone or by combined application with other pharmacologically active component. Exemplary such pharmacologically active components include known fibrinolytic agents (for example, tissue plasminogen activators (tPA) and their derivatives (including modified agents or so-called "second generation" agents), urokinase, and streptokinase); known anticoagulants (for example, warfarin, heparin, and thrombomodulin); known inhibitors of platelet aggregation (for example, aspirin, P₂Y₁₂ antagonist, thromboxane antagonist, inhibitor of thromboxane synthesis, and GPIIb/IIIa inhibitor); known therapeutic agents for hyperlipidemia (for example, clofibrate and related drugs, HMG-CoA inhibitor, and EPA-E); and known hypotensive agents (for example, nifedipine and diltiazem).
The term "combined application" as used herein covers not only the administration of a combination drug containing both the compound of the present invention and another pharmacologically active component but also the case where the two are in separate dosage forms and administered either at a time or at different times. The mode of administration is in no way limited as long as the compound of the present invention and another pharmacologically active component exist simultaneously in the patient's blood.

The pharmaceutical composition containing one or more of the compounds of the present invention and their pharmaceutically acceptable salts as its effective component may be prepared with a commonly used pharmaceutical vehicle, excipient, or other additives in the form of capsules, pills, tablets, granules, fine granules, or powder; oral solution such as suspension, emulsion, limonade, elixir, or syrup; injection; transnasal formulation; suppository; ointment; and epithem, and are orally or parenterally administered to human and other animals.
The clinical dose of the compound of the present invention to humans may be adequately determined in consideration of the symptom, body weight, age, sex, and the like of the patient to which the compound is to be administered. The adult daily dose in oral administration is generally in the range of 0.1 mg to 1000 mg, and preferably 1 mg to 300 mg, and the dose in parenteral administration is 0.01 to 300 mg, and preferably 0.1 mg to 100 mg. Such dose may be administered as a single dose or divided into several doses. The dose may vary depending on various conditions, and the dose below the above described range may be sufficient in some cases.

In order to accomplish oral administration according to the present invention, capsules, pills, tablets, powder, granules, and the like may be employed for the solid composition. Such solid composition is produced by combining at least one active substance with at least one inactive carrier. To be more specific, the composition may contain an excipient (for example, lactose, saccharose, mannitol, glucose, hydroxypropylcellulose, microcrystalline cellulose, or metasilicic acid), a binder (for example, crystalline cellulose, saccharide, dextrin, hydroxypropylcellulose, hydroxypropylmethylcellulose, polyvinyl pyrrolidone, or Macrogol), a lubricant (for example, magnesium stearate, calcium stearate, or talc), a disintegrant (for example, corn starch, carboxymethylcellulose, or carboxymethylcellulose calcium), a stabilizer (for example, lactose and other sugar alcohols or sugar), a solubilizer or a solubilizing aid (for example, cholesterol, triethanolamine, glutamic acid, or aspartic acid), a colorant, a flavoring agent, an antiseptic, an isotonic agent, a dispersant, an antioxidant (for example, ascorbic acid, or butylhydroxyanisole), a buffer, or a preservative (for example, paraben or benzyl alcohol).
It should be noted that the tablet, the pill and the granules may be coated with sucrose, gelatin, hydroxypropyl methylcellulosse phthalate or other gastric or enteric film coating.

Exemplary injections used for parenteral administration include aseptic aqueous or nonaqueous solution, suspension, and emulsion. Exemplary carriers for the aqueous solution and suspension include distilled water for injection and physiological saline, and exemplary carriers for the nonaqueous solution and suspension include propylene glycol, polyethylene glycol, vegetable oils such as olive oil, and alcohols such as ethyl alcohol, and polysorbate 80^{(™)}.

Such composition may further comprise an isotonic agent, antiseptic, humectant, emulsifier, dispersant, stabilizer, solubilizer, solubilizing aid, or other additives as described above, and these additives may be sterilized, for example, by filtration with a membrane filter, inclusion of an antimicrobial agent, or UV irradiation.

The composition may be also produced in the form of sterilized solid composition which can be dissolved, emulsified, or suspended before its use as an injection. When the compound of the present invention has low solubility, the compound may be solubilized as desired. Such solubilization may be accomplished by any of known processes applicable for the production of drugs, for example, addition of a surfactant (a polyoxyethylene hydrogenated castor oil, a higher fatty acid ester of polyoxyethylene sorbitan, a sucrose fatty acid ester, and the like); and formation of a solid dispersion of the drug and a solubilizer, for example, a polymer (a water-soluble polymer such as polyethylene glycol (PEG), hydroxypropyl methylcellulose (HPMC), or polyvinyl pyrrolidone (PVP); or an enteric polymer such as hydroxypropyl methylcellulose phthalate (HPMCP), or methyl methacrylate-methacrylic acid copolymer (Eudragit L,S^{(™)} manufactured by Rohm and Haas Company)). If desired, an inclusion compound may be formed by using α-, β-, or γ-cyclodextrin, hydroxypropyl cyclodextrin, or the like. The procedure employed for the solubilization may also be modified as desired depending on the drug to be prepared by referring to Nagai, T., et al., "Monograph in Pharmacology No.1, Biochemical Availability", Soft Science Inc., 78-82(1988) or Utsumi, I., et al., "Current Pharmaceutical Technology and Its Application", Iyaku Journal, 157-159(1983). Among these, the preferred is formation of a solid dispersion comprising the drug and the solubilizer which exhibits an improved solubility (JP 56-49314 A, FR 2460667 A).

### [Formulation Examples]

Next, examples of the pharmaceutical composition of the present invention are described.

**(a) Tablet (1 mg)**

| | |
|---|---|
| Compound of Example 1 | 1.0 g |
| Lactose | 90.0 g |
| Sodium carboxymethyl cellulose | 7.0 g |
| Corn starch paste (5% W/V paste) | 1.0 g |
| Magnesium stearate | 1.0 g |

The ingredients as described above were weighed and compressed in the usual manner to prepare tablets each weighing 100 mg.

**(b) Tablet (10 mg)**

| | |
|---|---|
| Compound of Example 4 | 10 g |
| Lactose | 150 g |
| Crosscarmellose sodium | 6.0 g |
| Corn starch | 28.5 g |
| Polyvinyl pyrrolidone | 2.5 g |
| Magnesium stearate | 3 g |

The ingredients as described above were weighed and compressed in the usual manner to prepare tablets each weighing 200 mg, and the tablets were coated with cellulose acetate phthalate to produce enteric-coated tablets.

**(c) Tablet (100 mg)**

| | |
|---|---|
| Compound of Example 11 | 100 g |
| Lactose | 180 g |
| Crosscarmellose sodium | 13 g |
| Corn starch (5% W/V paste) | 4 g |
| Magnesium stearate | 3 g |

The ingredients as described above were weighed and compressed in the usual manner to obtain tablets each weighing 300 mg.

**(d) Capsule (50 mg)**

| | |
|---|---|
| Compound of Example 31 | 100 g |
| Lactose | 395.5 g |
| Magnesium stearate | 4.5 g |

The ingredients as described above were weighed and uniformly mixed. The uniform powder was filled into hard capsules (Pharmacopeia No.1) in an amount of 250 mg/capsule.

**(e) Injection (0.1 mg/ml)**

| | |
|---|---|
| Compound of Example 17 | 0.1% W/V |
| Sodium phosphate buffer | 2.3% W/V |
| Citric acid | 0.4% |
| Macrogol 400 | 3.5% |
| Distilled water for injection | adequate amount to make up 100%. |

The ingredients as described above were mixed, and
the resulting solution was put in 1 ml portions into injection ampules, which were sealed to prepare injections.

**(f) Injection (1.0 mg/ml)**

| | |
|---|---|
| Compound of Example 24 | 1.0% W/V |
| Sodium phosphate buffer | 3.6% W/V |
| 1M Aqueous solution of sodium hydroxide | 15% W/V |
| Distilled water for injection | adequate amount to make up 100%. |

The ingredients as described above were mixed, and the resulting solution was put in 1 ml portions into injection ampules, which were sealed to prepare injections.

### EXAMPLES

Next, the present invention is described in further detail by referring to Examples which by no means limit the scope of the present invention.
Nuclear magnetic resonance (NMR) spectrum was measured by using JEOL JNM-EX270 FT-NMR (manufactured by JEOL Ltd.) or JEOL JNM-LA300 FT-NMR (the data taken with this model are preceded by an asterisk; manufactured by JEOL Ltd.).
High performance liquid chromatography (HPLC) was conducted by using Shimadzu LC-10A (manufactured by Shimadzu Corporation).
LC-MS was conducted by using Waters Fraction Lynx MS system (manufactured by Waters) with the column of SunFire column (4.6 mm x 5 cm, 5 µm) manufactured by Waters and mobile phase of acetonitrile and 0.5% aqueous acetic acid solution under the gradient condition of acetonitrile : 0.5% aqueous acetic acid solution = 1 : 9 (0 minutes) to 1 : 1 (6 minutes) to 9 : 1 (9 minutes) to 9 : 1 (10 minutes).

### [Example 1]

### Synthesis of (±)-3'5'8',11'-tetraaza-11'-(6-chloronaphthalen-2-ylsulfonyl)-3'-methyl-1-(2-oxazolinyl) spiro[piperidine-4,6'-tricyclo[6.4.0.0^{1,5}]dodecane]-4',9'-dione

### <Step 1>

### Synthesis of tert-butyl (±)-tetrahydro-5-oxo-1'-(phenylmethyl)-8a-(phthalimid-1-ylmethyl)-spiro[imidazo[1,2-a]pyrazine-2(3H),4'-piperidine]-7(1H)-carboxylate

N-[3-(phthalimid-1-yl)-2-oxopropyl]-N-[(tert-butoxy) carbonyl]-glycine ethyl ester (0.21 g) prepared by the method commonly used in the art as described in WO02/053568 and 4-amino-1-(phenylmethyl)-4-piperidine methanamine (0.14 g) were dissolved in toluene (15 ml), and acetic acid (0.04 ml) was added to this mixture. The mixture was stirred at room temperature for 30 minutes, at 50 to 60°C for 3 hours, and at 70 to 80°C for 3 hours with heating. After adding saturated aqueous solution of sodium bicarbonate, the mixture was extracted with methylene chloride, and the methylene chloride layer was washed with saturated aqueous solution of sodium chloride, dried with anhydrous sodium sulfate. The solvent was evaporated under reduced pressure, and the residue was purified by silica gel column chromatography (eluent: methylene chloride / methanol = 98/2 to 95/5) to produce the title compound (0.18 g) as a pale yellow amorphous compound.

### <Step 2>

### Synthesis of tert-butyl (±)-8a-(aminomethyl)-tetrahydro-5-oxo-1'-(phenylmethyl)-spiro[imidazo[1,2-a]pyrazine-2(3H),4'-piperidine]-7(1H)-carboxylate

The compound obtained in <step 1> (0.15 g) was dissolved in ethanol (3.0 ml), and after adding hydrazine monohydrate (65 µl), the mixture was heated under reflux for 1 hour. The insoluble content was removed by filtration, and washed with methylene chloride. The filtrate and the washings were concentrated under reduced pressure to produce the title compound (0.12 g) as a colorless amorphous compound.

### <Step 3>

### Synthesis of tert-butyl (±)-tetrahydro-8a-[N-(2-nitrobenzenesulfonyl)aminomethyl]-5-oxo-1'-(phenylmethyl) spiro[imidazo[1,2-a]pyrazine-2(3H),4'-piperidine]-7(1H)-carboxylate

The compound obtained in <step 2> (5.3 g) was dissolved in methylene chloride (50 ml), and triethylamine (3.4 ml) and o-nitrobenzenesulfonyl chloride (3.0 g) were added in an ice bath. The mixture was stirred at room temperature for 1 hour. After adding another portion of o-nitrobenzenesulfonyl chloride (1.4 g), the mixture was stirred at room temperature for another 1 hour, and after adding still another portion of o-nitrobenzenesulfonyl chloride (1.4 g), the mixture was stirred at room temperature for 1 hour. Saturated aqueous solution of sodium bicarbonate and methylene chloride were added to the reaction mixture, and the mixture was extracted with methylene chloride. The methylene chloride layer was washed with saturated aqueous solution of sodium chloride, and dried with anhydrous sodium sulfate. The solvent was evaporated under reduced pressure, and the residue was purified by silica gel column chromatography (eluent: methylene chloride / methanol = 50/1 to 40/1) to produce the title compound (3.2 g) as a brown amorphous compound.

### <Step 4>

### Synthesis of tert-butyl (±)-tetrahydro-8a-[N-methyl-N-(2-nitrobenzenesulfonyl)aminomethyl]-5-oxo-1'-(phenylmethyl) spiro[imidazo[1,2-a]pyrazine-2(3H),4'-piperidine]-7(1H)-carboxylate

The compound obtained in <step 3> (3.2 g) was dissolved in DMF (50 ml), and after adding potassium carbonate (1.4 g) and methyl iodide (0.42 ml) in an ice bath, the mixture was stirred at room temperature for 3.5 hours. After adding saturated aqueous solution of sodium chloride, the reaction mixture was extracted with ethyl acetate, and the ethyl acetate layer was washed with saturated aqueous solution of sodium chloride and dried with anhydrous sodium sulfate. the solvent was evaporated under reduced pressure to produce the title compound (3.0 g) as a colorless amorphous compound.

### <Step 5>

### Synthesis of tert-butyl (±)-tetrahydro-8a-(N-methylaminomethyl)-5-oxo-1'-(phenylmethyl) spiro[imidazo[1,2-a]pyrazine-2(3H),4'-piperidine]-7(1H)-carboxylate

The compound obtained in <step 4> (3.0 g) was dissolved in acetonitrile (50 ml), and after adding cesium carbonate (3.1 g) and thiophenol (0.49 ml) in an ice bath, the mixture was stirred at room temperature for 2.5 hours. After adding 1N aqueous sodium hydroxide, the mixture was extracted with methylene chloride, and the methylene chloride layer was washed with saturated aqueous solution of sodium chloride and dried with anhydrous sodium sulfate. The solvent was evaporated under reduced pressure, and the residue was purified by silica gel column chromatography (eluent: methylene chloride / methanol = 50/1 → methylene chloride / 4N ammonia-containing methanol = 10/1) to produce the title compound (0.79 g) as a brown amorphous compound.

### <Step 6>

### Synthesis of (±)-3',5',8',11'-tetraaza-11'-(tert-butyloxycarbonyl)-3'-methyl-1-(phenylmethyl) spiro[piperidine-4,6'-tricyclo[6.4.0.0^{1,5}]dodecane]-4',9'-dione

The compound obtained in <step 5> (0.79 g) was dissolved in THF(20 ml), and after adding triethylamine (0.5 ml) and triphosgene (0.53 g) in an ice bath, the mixture was stirred for 10 minutes. After adding saturated aqueous solution of sodium bicarbonate to the reaction mixture, the mixture was extracted with methylene chloride, and the methylene chloride layer was washed with saturated aqueous solution of sodium chloride and dried with anhydrous sodium sulfate. The solvent was evaporated under reduced pressure, and the residue was purified by silica gel column chromatography (eluent: methylene chloride / methanol = 98/2 → 90/10) to produce the title compound (0.55 g) as a yellow amorphous compound.

### <Step 7>

### Synthesis of (±)-3',5',8',11'-tetraaza-3'-methyl-1-(phenylmethyl) spiro[piperidine-4,6'-tricyclo[6.4.0.0^{1,5}]dodecane]-4',9'-dione

The compound obtained in <step 6> (0.50 g) was dissolved in ethyl acetate (5.0 ml), and after adding 4N hydrogen chloride-ethyl acetate (2.5 ml) in an ice bath, the mixture was stirred at room temperature. Water was added to the reaction mixture for separation, and the ethyl acetate layer was extracted with 1N hydrochloric acid. The aqueous layer was combined and adjusted with 1N sodium hydroxide to a pH of 11 to 12. After extracting with methylene chloride, the methylene chloride layer was washed with saturated aqueous solution of sodium chloride, and dried with anhydrous sodium sulfate. The solvent was evaporated under reduced pressure to produce the title compound (0.36 g) as a pale yellow amorphous compound.

### <Step 8>

### Synthesis of (±)-3',5',8',11'-tetraaza-11'-(6-chloronaphthalen-2-ylsulfonyl)-3'-methyl-1-(phenylmethyl) spiro[piperidine-4,6'-tricyclo[6.4.0.0^{1,5}]dodecane]-4',9'-dione

The compound obtained in <step 7> (9.0 mg) was dissolved in methylene chloride (1.0 ml), and after adding triethylamine (6.8 µl) and 6-chloronaphthalene-2-sulfonyl chloride (6.4 mg) in an ice bath and allowing the mixture to return to room temperature, the mixture was stirred overnight. After adding methylene chloride and saturated aqueous solution of sodium chloride, the mixture was extracted with methylene chloride. The methylene chloride layer was washed with saturated aqueous solution of sodium chloride, and dried with anhydrous sodium sulfate. The solvent was evaporated under reduced pressure, and the residue was purified by silica gel column chromatography (eluent: methylene chloride / methanol = 19/1) to produce the title compound (12 mg) as a colorless oil.

### <Step 9>

### Synthesis of (±)-3',5',8',11'-tetraaza-11'-(6-chloronaphthalen-2-ylsulfonyl)-3'-methylspiro[piperidine-4,6'-tricyclo[6.4.0.0^{1,5}]dodecane]-4',9'-dione

The compound obtained in <step 8> (40 mg) was dissolved in 1,2-dichloroethane (2 ml), and after adding 1,8-bis(N,N-dimethylamino) naphthalene (1.4 mg), and then, 1-chloroethyl chloroformate (8.1 µl), the mixture was stirred at room temperature for 30 minutes, and concentrated under reduced pressure. Methanol (2 ml) was added to the residue, and the mixture was heated with refluxing for 1 hour. After allowing to cool, the reaction mixture was concentrated under reduced pressure, and diethylether was added to the residue for solidification. After pulverization, the supernatant was removed by decantation. 1N aqueous sodium hydroxide was added to the residue to adjust the pH to 10. The aqueous layer was extracted with methylene chloride, and the methylene chloride layer was washed with saturated aqueous solution of sodium chloride, and dried with anhydrous sodium sulfate. The solvent was evaporated under reduced pressure, and the residue was purified by silica gel column chromatography [Chromatorex NH^{™}](eluent: n-hexane / methylene chloride = 1/1 → methylene chloride → methylene chloride / methanol = 10/1) to produce the title compound (27 mg) as a white solid.

### <Step 10>

### Synthesis of (±)-3',5',8',11'-tetraaza-11'-(6-chloronaphthalen-2-ylsulfonyl)-3'-methyl-1-(2-oxazolinyl) spiro[piperidine-4,6'-tricyclo[6.4.0.0^{0,5}]dodecane]-4',9'-dione

The compound obtained in <step 9> (17 mg) was dissolved in THF (1 ml), and after adding 2-chloroethyl isocyanate (5.6 µl) in an ice bath, the mixture was stirred at room temperature for 1 hour. After adding saturated aqueous solution of sodium bicarbonate, the aqueous layer was extracted with methylene chloride, and the methylene chloride layer was washed with saturated aqueous solution of sodium chloride and dried with anhydrous sodium sulfate. The solvent was evaporated under reduced pressure, and after adding water (2 ml) to the residue, the mixture was heated with reflux for 1 hour. After allowing to cool, methylene chloride and saturated aqueous solution of sodium bicarbonate were added, and the aqueous layer was extracted with methylene chloride. The methylene chloride layer was washed with saturated aqueous solution of sodium chloride, and dried with anhydrous sodium sulfate. The solvent was evaporated under reduced pressure, and the residue was purified by preparative thin-layer chromatography [Chromatorex NH^{™}] (developing solvent: methylene chloride / methanol = 19/1) to produce the title compound (13 mg) as a white solid.

### [Example 2]

### Synthesis of (±)-3'5',8',11'-tetraaza-11'-(6-chloronaphthalen-2-ylsulfonyl)-1-(2-oxazolinyl) spiro[piperidine-4,6'-tricyclo[6.4.0.0^{0,5}]dodecane]-4',9'-dione

### <Step 1>

### Synthesis of (±)-7-(6-chloronaphthalen-2-ylsulfonyl)-tetrahydro-1'-(phenylmethyl)-8a-(phthalimid-1-ylmethyl)-spiro[imidazo[1,2-a]pyrazine-2(3H),4'-piperidin]-5(1H)-one

The procedure of Example 1, <step 1> was repeated by using N-(6-chloronaphthalen-2-ylsulfonyl)-N-[3-(phthalimid-1-yl)-2-oxopropyl]-glycine ethyl ester (5.00 g) prepared by the method commonly used in the art as described in WO02/053568 and 4-amino-1-(phenylmethyl)-4-piperidine methanamine (2.70 g) to produce the title compound (5.30 g) as a yellow amorphous compound.

### <Step 2>

### Synthesis of (±)-8a-(aminomethyl)-7-(6-chloronaphthalen-2-ylsulfonyl)-tetrahydro-1'-(phenylmethyl) spiro[imidazo[1,2-a]pyrazine-2(3H),4'-iperidine]-5(1H)-one

The procedure of Example 1, <step 2> was repeated by using the compound obtained in <step 1> (0.30 g) to produce the title compound (0.22 g) as a pale yellow amorphous compound.

### <Step 3>

### Synthesis of (±)-3',5',8',11'-tetraaza-11'-(6-chloronaphthalen-2-ylsulfonyl)-1-(phenylmethyl) spiro[piperidine-4,6'-tricyclo[6.4.0.0^{1,5}]dodecane]-4',9'-dione

The procedure of Example 1, <step 6> was repeated by using the compound obtained in <step 2> (0.3 g) to produce the title compound (0.26 g) as a white solid.

### <Step 4>

### Synthesis of (±)-3',5',8',11'-tetraaza-11'-(6-chloronaphthalen-2-ylsulfonyl) spiro[piperidine-4,6'-tricyclo[6.4.0.0^{1,5}]dodecane]-4',9'-dione

The procedure of Example 1, <step 9> was repeated by using the compound obtained in <step 3> (30 mg) to produce the title compound (19 mg) as a white solid.

### <Step 5>

### Synthesis of (±)-3',5',8',11'-tetraaza-11'-(6-chloronaphthalen-2-ylsulfonyl)-1-(2-oxazolinyl) spiro[piperidine-4,6'-tricyclo[6.4.0.0^{1,5}]dodecane]-4',9'-dione

The procedure of Example 1, <step 10> was repeated by using the compound obtained in <step 4> (15 mg) to produce the title compound (16 mg) as a white solid.

### [Example 3]

### Synthesis of (±)-3',5',8',11'-tetraaza-11'-(6-chloronaphthalen-2-ylsulfonyl)-1-(3-pyridazinyl) spiro[piperidine-4,6'-tricyclo[6.4.0.0^{1,5}]dodecane]-4',9'-dione

The compound obtained in Example 2, <step 4> (20 mg) was suspended in dioxane (1.5 ml) and ethanol (1.5 ml). After adding triethylamine (17 µl) and 3-chloropyridazine (19 mg), the mixture was stirred in a sealed tube at 130 to 140°C for 2 hours, and at 140 to 155°C for 5.5 hours. Another portion of 3-chloropyridazine (9 mg) was added, and the mixture was stirred at 140 to 155°C for 7.5 hours. The solvent was evaporated under reduced pressure, and the residue was purified by preparative thin-layer chromatography [Chromatorex NH^{™})](developing solvent: methylene chloride / methanol = 19/1) to produce the title compound (8 mg) as a white solid.

### [Example 4]

### Synthesis of (±)-3'-acetyl-3',5',8',11'-tetraaza-11'-(6-chloronaphthalen-2-ylsulfonyl)-1-(2-oxazolinyl) spiro[piperidine-4,6'-tricyclo[6.4.0.0^{1,5}]dodecan]-9'-one

### <Step 1>

### Synthesis of (±)-3',5',8',11'-tetraaza-11'-(6-chloronaphthalen-2-ylsulfonyl)-1-(phenylmethyl) spiro[piperidine-4,6'-tricyclo[6.4.0.0^{1,5}]dodecan]-9'-one

The compound obtained in Example 2, <step 2> (50 mg) was dissolved in methanol (1.0 ml), and after adding paraformaldehyde (2.7 mg) and sodium methoxide (4.9 mg), the mixture was stirred at room temperature for 3 days. The reaction mixture was concentrated, and after adding water, it was extracted with methylene chloride. The methylene chloride layer was washed with saturated aqueous solution of sodium chloride, and dried with anhydrous sodium sulfate. The solvent was evaporated under reduced pressure to obtain the title compound (54 mg) as a colorless amorphous compound.

### <Step 2>

### Synthesis of (±)-3'-acetyl-3',5',8',11'-tetraaza-11'-(6-chloronaphthalen-2-ylsulfonyl)-1-(phenylmethyl) spiro[piperidine-4,6'-tricyclo[6.4.0.0^{1,5}]dodecan]-9'-one

The compound obtained in <step 1> (40 mg) was dissolved in pyridine (1 ml), and after adding acetic anhydride (13 µl) at room temperature, the mixture was stirred for 1 hour. The reaction mixture was concentrated under reduced pressure, and the residue was purified by preparative thin-layer chromatography (silica gel) (developing solvent: methylene chloride / methanol = 20/1) to produce the title compound (36 mg) as a colorless amorphous compound.

### <Step 3>

### Synthesis of (±)-3'-acetyl-3',5',8',11'-tetraaza-11'-(6-chloronaphthalen-2-ylsulfonyl) spiro[piperidine-4,6'-tricyclo[6.4.0.0^{1,5}]dodecan]-9'-one

The procedure of Example 1, <step 9> was repeated by using the compound obtained in <step 2> (36 mg) to produce the title compound (12 mg) as a colorless oil.

### <Step 4>

### Synthesis of (±)-3'-acetyl-3',5',8',11'-tetraaza-11'-(6-chloronaphthalen-2-ylsulfonyl)-1-(2-oxazolinyl) spiro[piperidine-4,6'-tricyclo[6.4.0.0^{1,5}]dodecan]-9'-one

The procedure of Example 1, <step 10> was repeated by using the compound obtained in <step 3> (10 mg) to produce the title compound (9 mg) as a white solid.

### [Example 5]

### Synthesis of (±)-3',5',8',-tetraaza-11'-(6-chloronaphthalen-2-ylsulfonyl)-3'-methyl-1-(4-pyridyl) spiro[piperidine-4,6'-tricyclo[6.4.0.0^{1,5}]dodecan]-9'-one

### <Step 1>

### Synthesis of (±)-7-(6-chloronaphthalen-2-ylsulfonyl)-tetrahydro-8a-(phthalimid-1-ylmethyl)-1'-(4-pyridyl) spiro[imidazo[1,2-a]pyrazine-2(3H),4'-piperidin]-5(1H)-one

The procedure of Example 1, <step 1> was repeated by using N-(6-chloronaphthalen-2-ylsulfonyl)-N-[3-(phthalimid-1-yl)-2-oxopropyl]-glycine ethyl ester (9.27 g) prepared by the method commonly used in the art as described in WO02/053568 and 4-amino-1-(4-pyridyl)-4-piperidine methanamine trihydrochloride (8.02 g) to produce the title compound (3.06 g) as a yellow amorphous compound.

### <Step 2>

### Synthesis of (±)-8a-(aminomethyl)-7-(6-chloronaphthalen-2-ylsulfonyl)-tetrahydro-1'-(4-pyxidyl) spiro[imidazo[1,2-a]pyrazine-2(3H),4'-piperidin]-5(1H)-one

The procedure of Example 1, <step 2> was repeated by using the compound obtained in <step 1> (2.59 g) to produce the title compound (2.09 g) as a yellow amorphous compound.

### <Step 3>

### Synthesis of (±)-3',5',8',11'-tetraaza-11'-(6-chloronaphthalen-2-ylsulfonyl)-3'-methyl-1-(4-pyxidyl) spiro[piperidine-4,6'-tricyclo[6.4.0.0^{1,5}]dodecan]-9'-one

36% formaldehyde aqueous solution (0.25 ml) and formic acid (0.25 ml) were added to the compound obtained in <step 2> (30 mg), and the mixture was stirred at 50 to 60°C for 1.5 hours. To this reaction mixture, 1N aqueous sodium hydroxide was added in an ice bath, and the mixture was extracted with methylene chloride. The methylene chloride layer was washed with saturated aqueous solution of sodium chloride, and dried with anhydrous sodium sulfate. The solvent was evaporated under reduced pressure, and the residue was purified by silica gel column chromatography [Chromatorex NH^{™})] (eluent: methylene chloride / methanol = 99/1), and then, by preparative thin-layer chromatography (silica gel) (developing solvent: methylene chloride / methanol = 4/1). The title compound (14 mg) was obtained as a white solid.

### [Example 6]

### Synthesis of (-)-3',5',8',11'-tetraaza-11'-(6-chloronaphthalen-2-ylsulfonyl)-1-(5,6-dihydro-4H-1,3-oxazin-2-yl)-3'-methylspiro[piperidine-4,6'-tricyclo[6.4.0.0^{1,5}]dodecane]-4',9'-dione

### <Step 1>

### Synthesis of (+)-3',5',8',11'-tetraaza-3'-methyl-1-(phenylmethyl) spiro[piperidine-4,6'-tricyclo[6.4.0.0^{1,5}]dodecane]-4',9'-dione and (-)-3',5',8',11'-tetraaza-3'-methyl-1-(phenylmethyl) spiro[piperidine-4,6'-tricyclo[6.4.0.0^{1,5}]dodecane]-4',9'-dione

The compound obtained in Example 1, <step 7> (6.25 g) was fractionated by an optically active column [DAICEL CHIRALCEL OJ-H, eluent: MeOH: diethylamine = 100/0.1 (v/v)] to produce (+)-3',5',8',11'-tetraaza-3'-methyl-1-(phenylmethyl) spiro[piperidine-4,6'-tricyclo[6.4.0.0^{1,5}]dodecane]-4',9'-dione [first peak, 2.7 g, [α]_{D}²⁸+17.9^{*} (c1.00, CHCl₃), >98%ee] and (-)-3',5',8',11'-tetraaza-3'-methyl-1-(phenylmethyl) spiro[piperidine-4,6'-tricyclo[6.4.0.0^{1,5}]dodecane]-4',9'-dione [second peak, 2.7 g, [α]_{D}²⁸-17.6° (c1.00. CHCl₃), >98%ee].

### <Step 2>

### Synthesis of (-)-3',5',8',11'-tetraaza-11'-(6-chloronaphthalen-2-ylsulfonyl)-3'-methyl-1-(phenylmethyl) spiro[piperidine-4,6'-tricyclo[6.4.0.0^{1,5}]dodecane]-4',9'-dione

The procedure of Example 1, <step 8> was repeated by using (-)- form (second peak) obtained in <step 1> (0.40 g) to produce the title compound (0.56 g) as a colorless amorphous compound. [α]_{D}²⁸-140.0° (c1.00, CHCl₃)

### <Step 3>

### Synthesis of (-)-3',5',8',11'-tetraaza-11'-(6-chloronaphthalen-2-ylsulfonyl)-3'-methylspiro[piperidine-4,6'-tricyclo[6.4.0.0^{1,5}]dodecane]-4',9'-dione

The procedure of Example 1, <step 9> was repeated by using the compound obtained in <step 2> (0.55 g) to produce the title compound (0.41 g) as a colorless amorphous compound.
[α]D²⁷-177.2° (c1.00, CHCl₃)

### <Step 4>

### Synthesis of (-)-3',5',8',11'-tetraaza-11'-(6-chloronaphthalen-2-ylsulfonyl)-1-(5,6-dihydro-4H-1,3-oxazin-2-yl)-3'-methylspiro[piperidine-4,6'-tricyclo[6.4.0.0^{1,5}]dodecane]-4',9'-dione

The procedure of Example 1, <step 10> was repeated by using the compound obtained in <step 3> (40 mg) and 3-chloropropyl isocyanate (19 mg) to produce the title compound (41 mg) as a colorless amorphous compound.

### [Example 7]

### Synthesis of (-)-3',5',8',11'-tetraaza-11'-(6-chloronaphthalen-2-ylsulfonyl)-1-(4,5-dihydroisoxazol-3-yl)-3'-methylspiro[piperidine-4,6'-tricyclo[6.4.0.0^{1,5}]dodecane]'-4',9'-dione

N-ethyldiisopropylamine (69 µl) and solution of 4,5-dihydro-3-nitroisoxazole (46 mg) in ethanol (0.6 ml) were added to the solution of the compound obtained in Example 6, <step 3> (100 mg) in ethanol (0.6 ml), and the mixture was heated under reflux for 5 hours. After allowing to cool, saturated aqueous solution of sodium bicarbonate and methylene chloride were added to the reaction mixture, and the mixture was extracted by methylene chloride. The methylene chloride layer was washed with saturated aqueous solution of sodium chloride, and dried with anhydrous sodium sulfate. The solvent was evaporated under reduced pressure, and the residue was purified by silica gel column chromatography [Chromatorex NH^{™}](eluent: methylene chloride-methylene chloride / methanol = 99/1) to produce the title compound (43 mg) as a colorless amorphous compound.

### [Example 8]

### Synthesis of (-)-3',5',8',11'-tetraaza-11'-(6-chloronaphthalen-2-ylsulfonyl)-1-(3,4, 5,6-tetrahydropyridin-2-yl)-3'-methylspiro[piperidine-4,6'-tricyclo[6.4.0.0^{1,5}]dodecane]-4',9'-dione

Triethylamine (42 µl) and 3,4,5,6-tetrahydro-2-methylthiopyridine hydroiodide (51 mg) were added to the solution of the compound obtained in Example 6, <step 3> (50 mg) in ethanol (2 ml), and the mixture was refluxed with heating for 5 hours. After allowing to cool, saturated aqueous solution of sodium bicarbonate and methylene chloride were added to the reaction mixture, and the mixture was extracted with methylene chloride. The methylene chloride layer was washed with saturated aqueous solution of sodium chloride, and dried with anhydrous sodium sulfate. The solvent was evaporated under reduced pressure, and the residue was purified by silica gel column chromatography [Chromatorex NH^{™}] (eluent: methylene chloride → methylene chloride / methanol = 98/2), and then, by preparative thin-layer chromatography [Chromatorex NH™] (developing solvent: methylene chloride / methanol = 50/1) to produce the title compound (34 mg) as a pale yellow amorphous compound.

### [Example 9]

### Synthesis of (-)-1-acetyl-3',5',8',11'-tetraaza-11'-(6-chloronaphthalen-2-ylsulfonyl)-3'-methyl-spiro[piperidine-4,6'-tricyclo[6.4.0.0^{1,5}]dodecane]-4',9'-dione

The procedure of Example 4, <step 2> was repeated by using the compound obtained in Example 6, <step 3> (40 mg) and acetic anhydride (9 µl) to produce the title compound (30 mg) as a colorless amorphous compound.

### [Example 10]

### Synthesis of (-)-3',5',8',11'-tetraaza-11'-(6-chloronaphthalen-2-ylsulfonyl)-3'-methyl-1-propionylspiro[piperidine-4,6'-tricyclo[6.4.0.0^{1,5}]dodecane]-4',9'-dione

The compound obtained in Example 6, <step 3> (30 mg) was dissolved in methylene chloride (1 ml), and to this solution, triethylamine (17 µl) and propionyl chloride (6 µl) were added in an ice bath. The mixture was stirred for 5 minutes at the same temperature, and then, propionyl chloride (1 µl) was added, and the mixture was stirred for 5 minutes at the same temperature. Water was added to the reaction mixture, and the mixture was extracted with methylene chloride. The methylene chloride layer was washed with saturated aqueous solution of sodium chloride, and dried with anhydrous sodium sulfate. The solvent was evaporated under reduced pressure, and the residue was purified by silica gel column chromatography [Chromatorex NH^{™}] (eluent: methylene chloride → methylene chloride / methanol = 99/1), and then, by silica gel column chromatography (eluent: methylene chloride / methanol = 99/1 to 95/5) to produce the title compound (24 mg) as a colorless amorphous compound.

### [Example 11]

### Synthesis of (-)-3',5',8',11'-tetraaza-11'-(6-chlorobenzo[b]thiophen-2-ylsulfonyl)-3'-methyl-1-(2-oxazolinyl) spiro[piperidine-4,6'-tricyclo[6.4.0.0^{1,5}]dodecane]-4',9'-dione

### <Step 1>

### Synthesis of (-)-3',5',8',11'-tetraaza-11'-(6-chlorobenzo[b]thiophen-2-ylsulfonyl)-3'-methyl-1-(phenylmethyl) spiro[piperidine-4,6'-tricyclo[6.4.0.0^{1,5}]dodecane]-4',9'-dione

The procedure of Example 1, <step 8> was repeated by using (-)- form (second peak) obtained in Example 6, <step 1> (0.50 g) and 6-chlorobenzo[b]thiophene-2-sulfonyl chloride (0.38 g) to produce the title compound (0.71 g) as a pale yellow solid.

### <Step 2>

### Synthesis of (-)'-3',5',8',11'-tetraaza-11'-(6-chlorobenzo[b]thiophen-2-ylsulfonyl)-3'-methylspiro[piperidine-4,6'-tricyclo[6.4.0.0^{1,5}]dodecane]-4',9'-dione

The procedure of Example 1, <step 9> was repeated by using the compound obtained in <step 1> (0.70 g) to produce the title compound (0.48 g) as a pale brown solid.

### <Step 3>

### Synthesis of (-)-3',5',8',11'-tetraaza-11'-(6-chlorobenzo[b]thiophen-2-ylsulfonyl)-3'-methyl-1-(2-oxazolinyl) spiro[piperidine-4,6'-tricyclo[6.4.0.0^{1,5}]dodecane]-4',9'-dione

The procedure of Example 1, <step 10> was repeated by using the compound obtained in <step 2> (50 mg) to produce the title compound (46 mg) as a white solid.

### [Example 12]

### Synthesis of (-)-3',5',8',11'-tetraaza-11'-(6-chlorobenzo[b]thiophen-2-ylsulfonyl)-1-(5,6-dihydro-4H-1,3-oxazin-2-yl)-3'-methylspiro[piperidine-4,6'-tricyclo[6.4.0.0^{1,5}]dodecane]-4',9'-dione

The procedure of Example 6, <step 4> was repeated by using the compound obtained in Example 11, <step 2> (50 mg) to produce the title compound (48 mg) as a white solid.

### [Example 13]

### Synthesis of (-)-3',5',8',11'-tetraaza-11'-(6-chlorobenzo[b]thiophen-2-ylsulfonyl)-1-(4,5-dihydroisoxazol-3-yl)-3'-methylspiro[piperidine-4,6'-tricyclo[6.4.0.0^{1,5}]dodecane]-4',9'-dione

The procedure of Example 7 was repeated by using the compound obtained in Example 11, <step 2> (80 mg) to produce the title compound (47 mg) as a white solid.

### [Example 14]

### Synthesis of (-)-3',5',8',11'-tetraaza-11'-(6-chlorobenzo[b]thiophen-2-ylsulfonyl)-1-(3,4,5,6-tetrahydropyridin-2-yl)-3'-methylspiro[piperidine-4,6'-tricyclo[6.4.0.0^{1,5}]dodecane]-4',9'-dione

The procedure of Example 8 was repeated by using the compound obtained in Example 11, <step 2> (50 mg) to produce the title compound (46 mg) as a white solid.

### [Example 15]

### Synthesis of (-)-1-acetyl-3',5',8',11'-tetraaza-11'-(6-chlorobenzo[b]thiophen-2-ylsulfonyl)-3'-methylspiro[piperidine-4,6'-tricyclo[6.4.0.0^{1,5}]dodecane]-4',9'-dione

The procedure of Example 9 was repeated by using the compound obtained in Example 11, <step 2> (50 mg) to produce the title compound (52 mg) as a white solid.

### [Example 16]

### Synthesis of (-)-3',5',8',11'-tetraaza-11'-(6-chlorobenzo[b]thiophen-2-ylsulfonyl)-3'-methyl-1-propionylspiro[piperidine-4,6'-tricyclo[6.4.0.0^{1,5}]dodecane]-4',9'-dione

The procedure of Example 10 was repeated by using the compound obtained in Example 11, <step 2> (50 mg) to produce the title compound (49 mg) as a white solid.

### [Example 17]

### Synthesis of (-)-3',5',8',11'-tetraaza-11'-(5-chloro-1H-indol-2-ylsulfonyl)-3'-methyl-1-(2-oxazolinyl) spiro[piperidine-4,6'-tricyclo[6.4.0.0^{1,5}]dodecane]-4',9'-dione

### <Step 1>

### Synthesis of (-)-3',5',8',11'-tetraaza-11'-(1-benzenesulfonyl-5-chloro-1H-indol-2-ylsulfonyl)-3'-methyl-1-(phenylmethyl) spiro[piperidine-4,6'-tricyclo[6.4.0.0^{1,5}]dodecane]-4',9'-dione

The procedure of Example 1, <step 8> was repeated by using the compound obtained in Example 6, <step 1> (-)-form (second peak)(0.80 g) and 1-benzenesulfonyl-5-chloro-1H-indole-2-sulfonyl chloride (0.93 g) to produce the title compound (0.47 g) as a white amorphous compound.

### <Step 2>

### Synthesis of (-)-3',5',8',11'-tetraaza-11'-(1-benzenesulfonyl-5-chloro-1H-indol-2-ylsulfonyl)-3'-methylspiro[piperidine-4,6'-tricyclo[6.4.0.0^{1,5}]dodecane]-4',9'-dione

The procedure of Example 1, <step 9> was repeated by using the compound obtained in <step 1> (0.47 g) to produce the title compound (0.39 g) as a white amorphous compound.

### <Step 3>

### Synthesis of (-)-3',5',8',11'-tetraaza-11'-(5-chloro-1H-indol-2-ylsulfonyl)-3'-methyl-1-(2-oxazolinyl) spiro[piperidine-4,6'-tricyclo [6.4.0.0^{1,5}]dodecane]-4',9'-dione

The procedure of Example 1, <step 10> was repeated by using the compound obtained in <step 2> (50 mg), and the residue was dissolved in methanol. 1N solution of potassium hydroxide in methanol was added, and the mixture was stirred at room temperature for 1 hour. After adding water, the reaction mixture was extracted with methylene chloride, and the methylene chloride layer was washed with saturated aqueous solution of sodium chloride and dried with anhydrous sodium sulfate. The solvent was evaporated under reduced pressure, and the residue was purified by silica gel column chromatography [Chromatorex NH™] (eluent: methylene chloride / methanol = 100/1 to 50/1) to produce the title compound (22 mg) as a white amorphous compound.

### [Example 18]

### Synthesis of (-)-3',5',8',11'-tetraaza-11'-(5-chloro-1H-indol-2-ylsulfonyl)-1-(5,6-dihydro-4H-1,3-oxazin-2-yl)-3'-methylspiro[piperidine-4,5'-tricyclo[6.4.0.0^{1,5}]dodecane]-4',9'-dione

The procedure of Example 6, <step 4> was repeated by using the compound obtained in Example 17, <step 2> (50 mg), and the residue was dissolved in methanol. 1N solution of potassium hydroxide in methanol was added, and the mixture was stirred at room temperature for 1 hour. After adding water, the reaction mixture was extracted with methylene chloride, and the methylene chloride layer was washed with saturated aqueous solution of sodium chloride and dried with anhydrous sodium sulfate. The solvent was evaporated under reduced pressure, and the residue was purified by silica gel column chromatography [Chromatorex NH™] (eluent: methylene chloride / methanol = 100/0 to 100/2) to produce the title compound (25 mg) as a white solid.

### [Example 19]

### Synthesis of (-)-3',5',8',11'-tetraaza-11'-(5-chloro-1H-indol-2-ylsulfonyl)-1-(4,5-dihydroisoxazol-3-yl)-3'-methylspiro[piperidine-4,6'-tricyclo[6.4.0.0^{1,5}]dodecane]-4',9'-dione

The procedure of Example 7 was repeated by using the compound obtained in Example 17, <step 2> (0.10 mg), and the residue was dissolved in methanol. 1N solution of potassium hydroxide in methanol was added, and the mixture was stirred at room temperature for 1 hour. After adding water, the reaction mixture was extracted with methylene chloride, and the methylene chloride layer was washed with saturated aqueous solution of sodium chloride and dried with anhydrous sodium sulfate. The solvent was evaporated under reduced pressure, and the residue was purified by silica gel column chromatography [Chromatorex NH^{™}] (eluent: methylene chloride / methanol = 100/0 to 100/1) to produce the title compound (10 mg) as a pale brown solid.

### [Example 20]

### Synthesis of (-)-3',5',8',11'-tetraaza-11'-(5-chloro-1H-indol-2-ylsulfonyl)-1-(3,4,5,6-tetrahydropyridin-2-yl)-3'-methylspiro[piperidine-4,6'-tricyclo[6.4.0.0^{1,5}]dodecane]-4',9'-dione

The procedure of Example 8 was repeated by using the compound obtained in Example 17, <step 2> (50 mg), and the residue was dissolved in methanol. 1N solution of potassium hydroxide in methanol was added, and the mixture was stirred at room temperature for 1 hour. After adding water, the reaction mixture was extracted with methylene chloride, and the methylene chloride layer was washed with saturated aqueous solution of sodium chloride and dried with anhydrous sodium sulfate. The solvent was evaporated under reduced pressure, and the residue was purified by silica gel column chromatography [Chromatorex NH™] (eluent: methylene chloride / methanol = 100/0 to 100/2) to produce the title compound (29 mg) as a colorless amorphous compound.

### [Example 21]

### Synthesis of (-)-1-acetyl-3',5',8',11'-tetraaza-11'-(5' chloro-1H-indol-2-ylsulfonyl)-3'-methylspiro[piperidine-4,6'-tricyclo[6.4.0.0^{1,5}]dodecane]-4',9'-dione

The procedure of Example 9 was repeated by using the compound obtained in Example 17, <step 2> (50 mg), and the residue was dissolved in methanol. 1N solution of potassium hydroxide in methanol was added, and the mixture was stirred at room temperature for 1 hour. After adding water, the reaction mixture was extracted with methylene chloride, and the methylene chloride layer was washed with saturated aqueous solution of sodium chloride and dried with anhydrous sodium sulfate. The solvent was evaporated under reduced pressure, and the residue was purified by silica gel column chromatography [Chromatorex NH™] (eluent: methylene chloride / methanol = 100/0 to 100/1) to produce the title compound (23 mg) as a colorless amorphous compound.

### [Example 22]

### Synthesis of (-)-3',5',8',11'-tetraaza-11'-(5-chloro-1H-indol-2-ylsulfonyl)-3'-methyl-1-propionylspiro[piperidine-4,6'-tricyclo[6.4.0.0^{1,5}]dodecane]-4',9'-dione

The procedure of Example 10 was repeated by using the compound obtained in Example 17, <step 2> (50 mg), and the residue was dissolved in methanol. 1N solution of potassium hydroxide in methanol was added, and the mixture was stirred at room temperature for 1.5 hours. After adding water, the reaction mixture was extracted with methylene chloride, and the methylene chloride layer was washed with saturated aqueous solution of sodium chloride and dried with anhydrous sodium sulfate. The solvent was evaporated under reduced pressure, and the residue was purified by silica gel column chromatography [Chromatorex NH^{™}] (eluent: methylene chloride / methanol = 100/0 to 100/1) to produce the title compound (28 mg) as a colorless amorphous compound.

### [Example 23]

### Synthesis of (-)-3',5',8',11'-tetraaza-11'-[(E)-2-(5-chlorothiophen-2-yl)ethenesulfonyl]-3'-methyl-1-(2-oxazolinyl) spiro[piperidine-4,6'-tricyclo[6.4.0.0^{1,5}]dodecane]-4',9'-dione

### <Step 1>

### Synthesis of (-)-3',5',8',11'-tetraaza-11'-[(E)-2-(5-chlorothiophen-2-yl)ethenesulfonyl]-3'-methyl-1-(phenylmethyl) spiro[piperidine-4,6'-tricyclo[6.4.0.0^{1,5}]dodecane]-4',9'-dione

The procedure of Example 1, <step 8> was repeated by using (-)- form (second peak) obtained in Example 6, <step 1> (0.50 g) and (E)-2-(5-chlorothiophen-2-yl)ethenesulfonyl chloride (0.35 g) synthesized by a method commonly used in the art to produce the title compound (0.85 g) as a yellowish brown amorphous compound.

### <Step 2>

### Synthesis of (-)-3',5',8',11'-tetraaza-11'-[(E)-2-(5-chlorothiophen-2-yl)ethenesulfonyl]-3'-methylspiro[piperidine-4,6'-tricyclo[6.4.0.0^{1,5}]dodecane]-4',9'-dione

The procedure of Example 1, <step 9> was repeated by using the compound obtained in <step 1> (0.85 g) to produce the title compound (0.60 g) as a pale yellow amorphous compound.

### <Step 3>

### Synthesis of (-)-3',5',8',11'-tetraaza-11'-[(E)-2-(5-chlorothiophen-2-yl)ethenesulfonyl]-3'-methyl-1-(2-oxazolinyl) spiro[piperidine-4',6'-tricyclo[6.4.0.0^{1,5}]dodecane]-4',9'-dione

The procedure of Example 1, <step 10> was repeated by using the compound obtained in <step 2> (49 mg) to produce the title compound (46 mg) as a colorless amorphous compound.

### [Example 24]

### Synthesis of (-)-3',5',8',11'-tetraaza-11'-[(E)-2-(5-chlorothiophen-2-yl)ethenesulfonyl]-1-(5,6-dihydro-4H-1,3-oxazin-2-yl)-3'-methylspiro[piperidine-4,6'-tricyclo[6.4.0.0^{1,5}]dodecane]-4',9'-dione

The procedure of Example 6, <step 4> was repeated by using the compound obtained in Example 23, <step 2> (49 mg) to produce the title compound (41 mg) as a colorless amorphous compound.

### [Example 25]

### Synthesis of (-)-3',5',8',11'-tetraaza-11'-[(E)-2-(5-chlorothiophen-2-yl)ethenesulfonyl]-1-(4,5-dihydroisoxazol-3-yl)-3'-methylspiro[piperidine-4,6'-tricyclo[6.4.0.0^{1,5}]dodecane]-4',9'-dione

The procedure of Example 7 was repeated by using the compound obtained in Example 23, <step 2> (97 mg) to produce the title compound (53 mg) as a pale yellow amorphous compound.

### [Example 26]

### Synthesis of (-)-3',5',8',11'-tetraaza-11'-[(E)-2-(5-chlorothiophen-2-yl)ethenesulfonyl]-1-(3,4,5,6-tetrahydropyridin-2-yl)-3'-methylspiro[piperidine-4,6'-tricyclo[6.4.0.0^{1,5}]dodecane]-4',9'-dione

The procedure of Example 8 was repeated by using the compound obtained in Example 23, <step 2> (49 mg) to produce the title compound (45 mg) as a pale yellow amorphous compound.

### [Example 27]

### Synthesis of (-)-1-acetyl-3',5',8',11'-tetraaza-11'-[(E)-2-(5-chlorothiophen-2-yl)ethenesulfonyl]-3'-methylspiro[piperidine-4,6'-tricyclo[6.4.0.0^{1,5}]dodecane]-4',9'-dione

The procedure of Example 9 was repeated by using the compound obtained in Example 23, <step 2> (49 mg) to produce the title compound (41 mg) as a colorless amorphous compound.

### [Example 28]

### Synthesis of (-)-3',5',8',11'-tetraaza-11'-[(E)-2-(5-chlorothiophen-2-yl)ethenesulfonyl]-3'-methyl-1-propionylspiro[piperidine-4,6'-tricyclo[6.4.0.0^{1,5}]dodecane]-4',9'-dione

The procedure of Example 10 was repeated by using the compound obtained in Example 23, <step 2> (49 mg) to produce the title compound (44 mg) as a colorless amorphous compound.

### [Example 29]

### Synthesis of (-)-3',5',8',11'-tetraaza-11'-(6-chloronaphthalen-2-ylsulfonyl)-3'-methyl-1-(2-oxazolinyl) spiro[piperidine-4',6'-tricyclo[6.4.0.0^{1,5}]dodecane]-4',9'-dione

The procedure of Example 1, <step 10> was repeated by using the compound obtained in Example 6, <step 3> (40 mg) to produce the title compound (32 mg) as a colorless amorphous compound.

### [Example 30]

### Synthesis of (+)-3',5',8',11'-tetraaza-11'-(6-chloronaphthalen-2-ylsulfonyl)-3'-methyl-1-(2-oxazolinyl) spiro[piperidine-4,6'-tricyclo[6.4.0.0^{1,5}]dodecane]-4',9'-dione

### <Step 1>

### Synthesis of (+)-3',5',8',11'-tetraaza-11'-(6-chloronaphthalen-2-ylsulfonyl)-3'-methyl-1-(phenylmethyl) spiro[piperidine-4,6'-tricyclo[6.4.0.0^{1,5}]dodecane]-4',9'-dione

The procedure of Example 1, <step 8> was repeated by using (+)- form (first peak) obtained in Example 6, <step 1> (0.40 g) to produce the title compound (0.58 g) as a colorless amorphous compound.
[α]_{D}²⁸+139.4° (c1.00, CHCl₃)

### <Step 2>

### Synthesis of (+)-3',5',8',11'-tetraaza-11'-(6-chloronaphthalen-2-ylsulfonyl)-3'-methylspiro[piperidine-4,6'-tricyclo[6.4.0.0^{1,5}]dodecane]-4',9'-dione

The procedure of Example 1, <step 9> was repeated by using the compound obtained in <step 1> (0.57 g) to produce the title compound (0.49 g), as a colorless amorphous compound.
[α]_{D}²⁸+173.9° (c1-00, CHCl₃)

### <Step 3>

### Synthesis of (+)-3',5',8',11'-tetraaza-11'-(6-chloronaphthalen-2-ylsulfonyl)-3'-methyl-1-(2-oxazolinyl) spiro[piperidine-4,6'-tricyclo[6.4.0.0^{1,5}]dodecane]-4',9'-dione

The procedure of Example 1, <step 10> was repeated by using the compound obtained in <step 2> (50 mg) to produce the title compound (48 mg) as a colorless amorphous compound.

### [Example 31]

### Synthesis of (+)-1-acetyl-3',5',8'11'-tetraaza-11'-(6-chloronaphthalen-2-ylsulfonyl)-3'-methyl-spiro[piperidine-4,6'-tricyclo[6.4.0.0^{1,5}]dodecane]-4',9'-dione

The procedure of Example 9 was repeated by using the compound obtained in Example 30, <step 2> (50 mg) and acetic anhydride (10 µl) to produce the title compound (45 mg) as a colorless amorphous compound.
The structures of the present compounds produced in the Examples are shown in Table 4. Physical data of the Examples are shown in Table 5 (NMR) and Table 6 (LC-MS).

[Table 4]

**Table 4-1**

| | | | |
|---|---|---|---|
| 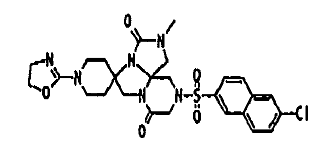 | | 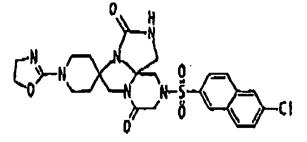 | |
| Example 1 | Racemate | Example 2 | Racemate |
| 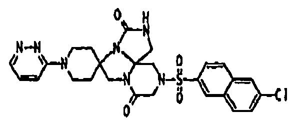 | | 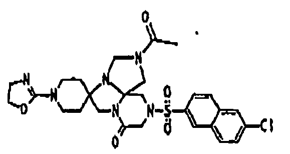 | |
| Example 3 | Racemate | Example 4 | Racemate |
| 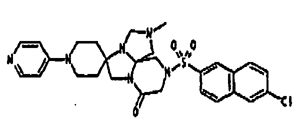 | | 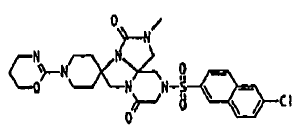 | |
| Example 5 | Racemate | Example 6 | (-)-Form |
| 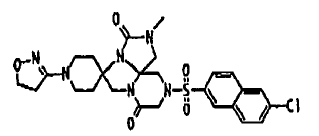 | | 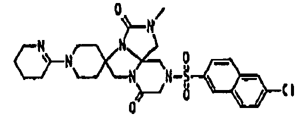 | |
| Example 7 | (-)-Form | Example 8 | (-)-Form |
| 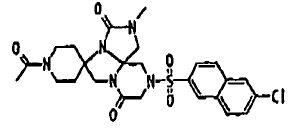 | | 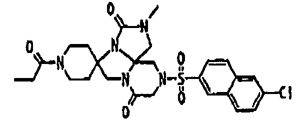 | |
| Example 9 | (-)-Form | Example 1 0 | (-)-Form |
| 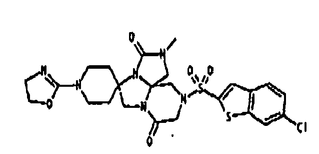 | | 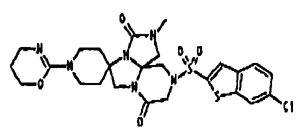 | |
| Example 11 | (-)-Form | Example 2 | (-)-Form |

[Table 5]

**Table 4-2**

| | | | |
|---|---|---|---|
| 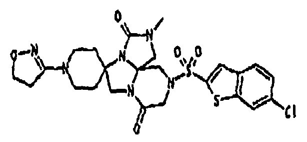 | | 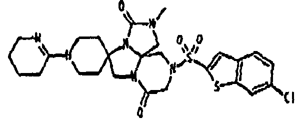 | |
| Example 13 | (-) -Form | Example 14 | (-)-Form |
| 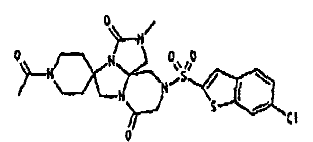 | | 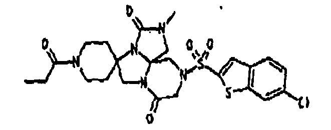 | |
| Example 15 | (-)-Form | Example 16 | (-)-Form |
| 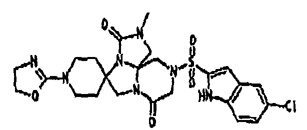 | | 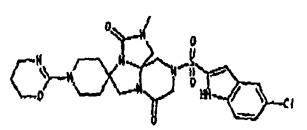 | |
| Example 17 | (-)-Form | Example 18 | (-)-Form |
| 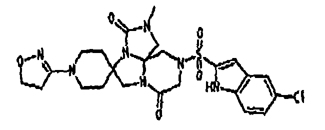 | | 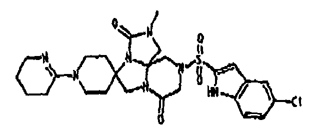 | |
| Example 19 | (-)-Form | Example 20 | (-)-Form |
| 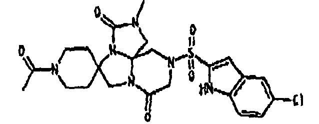 | | 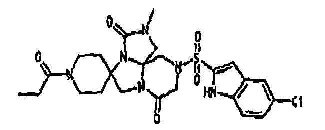 | |
| Example 21 | (-)-Form | Example 22 | (-)-Form |
| 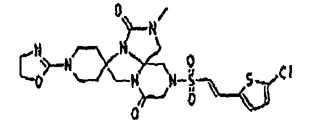 | | 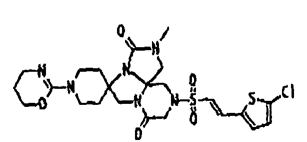 | |
| Example 23 | (-)-Form | Example 24 | (-)-Form |

[Table 6]

**Table 4-3**

| | | | |
|---|---|---|---|
| 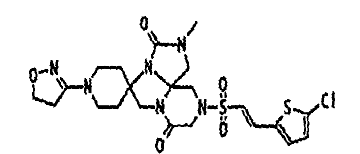 | | 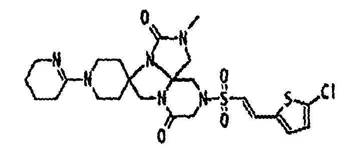 | |
| Example 25 | (-) -Form | Example 26 | (-)-Form |
| 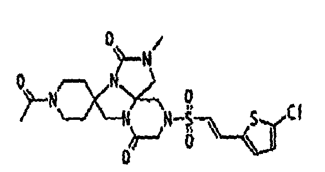 | | 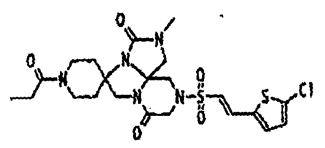 | |
| Example 27 | (-)-Form | Example 28 | (-) -Form |
| 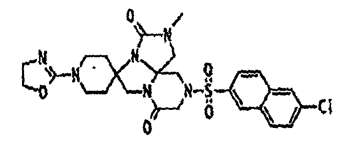 | | 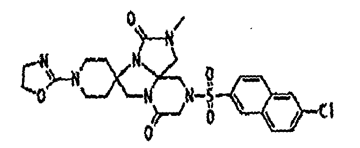 | |
| Example 29 | (-) -Form | Example 30 | (+) -Form |
| 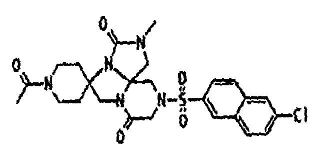 | | | |
| Example 31 | (+)-Form | | |

[Table 7]

**Table 5-1**

| Example No. | NMR(ppm) 300MHz, *:270MHz) |
|---|---|
| Example1 <Step3> | DMSO-d6, 100°C:8.01-7.77 (4H, m), 7.65 (1H, brs), 7.35-7.14 (5H, m), 4.07 (1H, d, J=18Hz), 4.05 (1H, d, J=13Hz), 3.94 (1H, d, J=12Hz), 3.65 (1H, d, J=18Hz), 3.46 (2H, s), 3.13-2.72 (3H, m), 3.05 (1H, d, J=12Hz), 2.60-2.37 (2H, m), 2.32-2.15 (2H. m), 1.70-1.55 (2H, m), 1.48-1.30 (2H, m), 1.39 (9H, s) |
| Example1 <Step4> | DMSO-d6, 100°C:8.00-7.79 (4H, m), 7.35-7.15 (5H, m), 4.16-4.00 (3H, m), 3.69 (1H, d, J=18Hz), 3.50-3.37 (1H, m), 3.47 (2H, s), 3.32 (1H. d, J=15Hz), 3.14-269 (2H, m), 2.94 (3H, s), 2.60-2.40 (2H, m), 2.34-2.16 (2H, m), 1.80-1.60 (2H, m), 1.52-1.34 (2H, m), 1.41 (9H, s) |
| Example1 <Step5> | DMSO-d6, 100°C*:7.34-7.16 (5H, m), 4.15 (1H, d, J=13Hz), 4.12 (1H, d, J=18Hz), 3.97 (1H, d, J=12Hz), 3.63 (1H, d, J=18Hz), 3.47 (2H, s), 3.06-2.44 (4H, m), 2.86 (1H, d, J=12Hz), 2.69 (1H, d, J=13Hz), 2.34-2.17 (2H, m). 2.33 (3H, s), 1.73-1.62 (2H, m), 1.48-1.36 (2H, m), 1.43 (9H, s) |
| Example1 <Step6> | DMSO-d6, 100°C:7.35-7.12 (5H, m), 4.33 (1H, d, J=12Hz). 4.22-4.06 (2H. m), 3.73 (1H, d, J=18Hz), 3.58-3.38 (2H, m), 3.34 (1H, d, J=9Hz), 3.27 (1H, d, J=9Hz), 3.06-2.80 (1H, m). 2.87 (1H, d, J=12Hz), 2.75-2.36 (2H, m), 2.66 (3H, s), 2.27-1.93 (2H, m), 1.64-1.30 (4H, m), 1.41 (9H, s) |
| Example1 <Step7> | CDCl3:7.39-7.14 (5H, m), 4.65 (1H, d, J=12Hz), 3.69 (1H, d, J=18Hz), 3.67 (1H, d. J=10Hz), 3.57 (1H, d, J=18Hz), 3.50 (2H, s), 3.35 (2H, dd, J=2,10Hz), 3.24 (1H, d, J=12Hz), 3.03-2.91 (1H, m), 2.91-2.60 (3H, m). 2.82 (3H, s), 2.76 (1H, d, J=12Hz), 2.26-2.00 (2H, m), 1.87-1.36 (3H, m) |
| Example1 <Step8> | CDCl3*:8.36-8.30 (1H, m), 8.00-7.88 (3H, m), 7.76 (1H, dd, J=2, 9Hz). 7.62 (1H, dd, J=2, 9Hz), 7.33-7.16 (5H, m). 4.55 (1H, d, J=12Hz), 4.40 (1H, d, J=17Hz), 4.13 (1H, d, J=12Hz), 3.84 (1H, d, J=10Hz), 3.47-3.32 (1H, m). 3.44 (2H; s), 3.31 (1H, d, J=17Hz), 3.00-2.70 (2H, m), 2.88 (3H, s). 2.77 (1H, d, J=12Hz), 2,63-2,50 (1H, m), 2.38-2.28 (1H, m), 2.10-1.93 (2H, m), 1.70-1.48 (2H, m), 1.21-1.07 (1H, m) |
| Example1 <Step10> | CDCl3:8.40-8.30 (1H, m), 8.02-7.90 (3H, m), 7.77 (1H, dd, J=2, 9Hz), 7.63 (1 H. dd, J=2.9Hz), 4.51 (1H, d, J=12Hz), 4.42 (1H, d, J=17Hz), 4.26 (2H, t, J=9Hz), 4.15 (1H, d, J=12Hz), 4.09-3.96 (1H, m), 3.88 (1H, d, J=10Hz), 3.75 (2H, t, J=9Hz), 3.60-3.47 (1H, m), 3.45-3.37 (1H, m), 3.35 (1H, d, J=17Hz). 3.13-2.97 (2H, m), 2.89 (3H, s), 2.85 (1H, d, J=12Hz), 2.66-2.53 (1H, m), 2.37 (1H, d, J=12Hz), 1.70-1.45 (2H, m), 1.30-1.12 (1H, m) |
| Example2 <Step5> | CDCl3:8.40-8.30 (1H, m), 8.05-7.86 (3H, m), 7.78 (1H, dd, J=2, 9Hz), 7.63 (1H, dd, J=2, 9Hz), 5.46 (1H, brs), 4.57 (1H, d, J=12Hz), 4.41 (1H, d, J=17Hz), 4.33-4.17 (1H, m), 4.26 (2H, t, J=9Hz), 4.12-3.93 (1H, m), 3.99 (1H, d, J=10Hz), 3.74 (2H, t, J=9Hz), 3.64-3.50 (1H, m), 3.46-3.35 (1H, m), 3.36 (1H, d, J=17Hz), 3.10-2.93 (2H, m), 2.90 (1H, d, J=12Hz), 2.73-2.56 (1H, m), 2.42-2.30 (1H, m), 1.72-1.47 (2H, m), 1.30-1.13 (1H, m) |

[Table 8]

**Table 5-2**

| Example No. | NMR(ppm) (300MHz, *:270MHz) |
|---|---|
| Example 3 | CDCl3:8.56 (1H, d, J=5Hz), 8.40-8.31 (1H, m), 8.02-7.90 (3H, m), 7.79 (1H, dd, J=2, 9Hz), 7.64 (1H, dd, J=2, 9Hz), 7.18 (1H, dd, J=5, 9Hz), 6.90-6.80 (1H, m), 4.91 (1H, brs), 4.62 (1H, d, J=12Hz), 4.44 (1H, d, J=17Hz), 4.36-4.20 (1H, m), 4.27 (1H, d, J=12Hz), 4,06-3,91 (1H, m), 4.02 (1H, d, J=10Hz), 3.52-3.18 (4H, m), 2.97 (1H, d, J=12Hz), 2.79-2.62 (1H, m), 2.45-2.34 (1H, m), 1.85-1.46 (2H, m), 1.41-1.15 (1H, m) |
| Example 4 <Step1> | CDCl3*:8.37-8.28 (1H, m), 7.98-7.86 (3H, m), 7.75 (1H, dd, J=2, 9Hz), 7.60 (1H, dd, J=2,9Hz), 7.36-7.13 (5H, m), 4.40-4,21 (2H, m), 4.00 (1H, d, J=9Hz), 3.95 (1H, d, J=9Hz), 3.88 (1H, d, J=12Hz), 3.66 (1H, d, J=13Hz), 3.42 (2H, s). 3.28 (1H, d, J=17Hz), 2,85-2,73 (2H, m), 2.72-2.41 (2H, m), 2.36-2.26 (1 H, m), 2.21-2.00 (2H, m), 200-1.84 (1H, m), 1.80-1.67 (1H, m), 1.43-1.23 (1H, m), 1.21-1,07 (1H, m) |
| Example 4 <Step2> | CDCl3:8.39-8.29 (1H, m), 8.02-7.89 (3H, m), 7.76 (1H, dd, J=2, 9Hz), 7.62 (1H, dd, J=2.9Hz), 7.40-7.17 (5H, m), 4.86 (1H, d, J=11Hz), 4.45-4.27 (1H, m), 4.31 (1H, d. J=11 Hz), 4.12-3.96 (3H, m), 3.45 (2H, s), 3.31 (1H, d, J=17Hz), 3.25 (1H, d, J=12Hz), 3.11 (1H, d, J=12Hz), 2.95-2.60 (2H, m), 2.35 (1H, d, J=11Hz), 2.20-1.83 (3H, m). 2.10 (3H, s), 1.65-1.39 (2H, m), 1.36-1.21 (1H, m) |
| Example 4 <Step3> | CDCl3:8.40-8.30 (1H, m), 8.02-7.90 (3H, m), 7.77 (1H. dd, J=2, 9Hz), 7.66-7.58 (1H, m), 4.89 (1H, d. J=11Hz), 4.41 (1H, d, J=17Hz), 4.32 (1H, d, J=11Hz), 4.13 (1H, d, J=12Hz), 4.08 (1H, d, J=11Hz), 4.02 (1 H, d, J=12Hz), 3.48-3.23 (1 H, m), 3.32 (1H, d, J=17Hz), 3.17-3.05 (1H, m), 3.11 (1H, d, J=12Hz), 3.00-2.87 (1H, m), 2.65-2.50 (2H, m), 2.41-2.33 (1H, m), 2.12 (3H, s), 1.85-1.72 (1H, m), 1.64-1.20 (3H, m) |
| Example 4 <Step4> | CDCl3:8.40-8.28 (1H, m), 8.02-7.88 (3H, m), 7.76 (1H, dd, J=2, 9Hz), 7.66-7.57 (1H, m), 4.83 (1H, d, J=11Hz), 4.41 (1H, d, J=17Hz), 4.33 (1H, d, J=12Hz), 4.27 (2H, t, J=9Hz), 4.17-4.05 (2H, m), 4,02 (1H, d, J=12Hz), 3.94-3.62 (2H, m), 3.75 (2H, t, J=9Hz), 3.33 (1H, d. J=17Hz), 3.33-3.22 (1H, m). 3.13 (1H, d, J=12Hz). 3.01-2.80 (2H, m), 2.37 (1H, d, J=12Hz), 2.12 (3H, s), 1.94-1.77 (1H, m), 1.57-1.37 (2H, m), 1.37-1.20 (1H, m) |
| Example 5 <Step3> | CDCl3:8.40-8.30 (1H, m), 8.23 (2H, d, J=6Hz), 8.02-7.89 (3H, m). 7.82-7.73 (1H, m), 7.61 (1H, dd, J=2, 9Hz), 6.58 (2H, d, J=6Hz), 4.36 (1H, d, J=17Hz), 4.27 (1H, d, J=11Hz), 4.13 (1H, d, J=11Hz), 3.73 (1H, d, J=7Hz), 3.66-3.52 (1H, m), 3.51-3.23 (1H, m), 3.33 (1H, d, J=7Hz), 3.31 (1H, d, J=17Hz), 3.19-2.95 (2H, m), 3.02 (1H, d, J=11Hz), 2.92 (1H, d, J=10Hz), 2.86 (1H, d, J=10Hz), 2.39 (1H, d, J=11Hz), 2.30 (3H, s), 2.05-1.70 (2H, m), 1.50-1.15 (2H, m) |
| Example 10 | DMSO-d6, 100°C*:8.61-8.50 (1H, m), 8.25-8.11 (1H, m). 8.24 (1H, d, J=9Hz). 8.15 (1H, d, J=9Hz), 7.90 (1H, dd, J=2, 9Hz), 7.69 (1H, dd, J=2, 9Hz), 4.31 (1H, d, J=12Hz). 4,17 (1H, d, J=16Hz), 4.10-3.90 (1H, m), 4.02 (1H, d, J=12Hz), 3.61 (1H, d, J=16Hz), 3.60-3.40 (3H, m), 3.27-2.90 (3H, m), 2.83-2.68 (1H, m), 2.72 (3H, s), 2.40-2.16 (1H, m), 2.25 (2H, q, J=8Hz), 1.69-1.50 (1H, m), 1.45-1.27 (2H, m), 0.98 (3H, t, J=8Hz) |

[Table 9]

**Table 5-3**

| Example No. | NMR(ppm) (300MHz, *.270MHz) |
|---|---|
| Example17 <Step1> | CDCl3:8.24 (1H, d. J=9Hz), 8.08-7.95 (1H, m), 8.02 (1H, d, J=8Hz), 7.66-7.41 (5H, m), 7.49 (1H, d, J=8Hz). 7.38-7.17 (5H, m), 4.61 (1H, d, J=12Hz), 4.39 (1 H. d, J=17Hz), 4.28 (1H, d, J=13Hz), 3.77-3.66 (1H, m), 3.76 (1H, d, J=17Hz, 3.52 (1H, d. J=14Hz), 3.47 (1H, d, J=14Hz), 3.38 (1H, dd, J=1, 10Hz), 3.12-2.62 (3H, m), 3.06 (1 H. dd. J=1, 13Hz), 2.83 (1H, d, J=12Hz), 2.79 (3H, s), 2.25-1.96 (2H, m), 1.90-1.72 (1H, m), 1.69-1.50 (1H, m), 1.48-1.34 (1H, m) |
| Example19 | CDCl3:9.11 (1H, brs), 7.74-7.68 (1H, m), 7.42 (1H, d, J=9Hz), 7.42-7.34 (1 H, m), 7.05 (1H, s), 4.53 (1H, d, J=12Hz), 4.37 (1H, d, J=17Hz), 4.30 (2H, t, J=9Hz), 4.10 (1H, d, J=12Hz), 3.86-3.73 (1H, m), 3.82 (1H, d, J=10Hz), 3.52-3.30 (2H, m), 3.47 (1H, d, J=17Hz). 3.13-2.78 (3H, m), 2.92 (2H, t, J=9Hz), 2.88 (3H, s), 2.71-2.55 (1H, m), 2.50-2.40 (1H, m), 1.74-1.45 (2H, m), 1.36-1.20 (1H, m) |
| Example21 | DMSO-d6, 100°C;12.12 (1 H, brs), 7,74 (1 H, d, J=2Hz), 7.50 (1 H, d, J=9Hz), 7.30 (1H, dd, J=2, 9Hz), 7.11 (1H, s), 4.31 (1H, d, J=12Hz), 4.17-3.85 (1H, m), 4.13 (1H, d, J=17Hz), 3.96 (1H, d. J=12Hz), 3.68 (1H, d, J=17Hz), 3.57-3.35 (1H, m), 3.47 (1H, d, J=10Hz), 3.43 (1H, d, J=10Hz), 3.26-2.77 (3H, m), 2.81 (1H, dd, J=1.12Hz), 2.69 (3H, s), 2.37-2.20 (1H, m). 1.94 (3H, s), 1.74-1.50 (1 H, m), 1.45-1.27 (2H, m) |
| Example22 | DMSO-d6, 100°C:12.23 (1H, brs), 7.74 (1H, d, J=2Hz), 7.50 (1H, d, J=9Hz). 7.30 (1H, dd, J=2, 9Hz), 7.11 (1H, s), 4.31 (1H, d, J=12Hz), 4.20-3.90 (1H, m). 4.13 (1H, d, J=17Hz), 3.96 (1H, d, J=12Hz), 3.68 (1H, d, J=17Hz), 3.58-3.36 (1H, m), 3.48 (1H, d, J=10Hz), 3.43 (1H, d, J=10Hz), 3.25-2.75 (3H, m), 2.81 (1H, d. J=12Hz), 2.69 (3H, s), 2.38-2.13 (1H, m), 2.25 (2H, q, J=8Hz),1.67-1.52 (1H, m), 1.47-1.29 (2H, m), 0.98 (3H, t, J=8Hz) |

[Table 10]

**Table 6**

| Example No. | LC-MS(ESI⁺) | | | Example No. | LC-MS(ESI⁺) | | | Example No. | LC-MS(ESI⁺) | |
|---|---|---|---|---|---|---|---|---|---|---|
| | *m*/*z* [M + H]⁺ | Retention time (min) | | | *m*/*z* [M + H]⁺ | Retention time (min) | | | *m*/*z* [M + H]⁺ | Retention time (min) |
| Example1 <Step1> | 560 | 3.72 | | Example6 <Step2> | 594 | 2.91 | | Example23 <Step1> | 576 | 2.79 |
| Example1 <Step2> | 430 | 0.21 | | Example6 <Step3> | 504 | 2.57 | | Example23 <Step2> | 486 | 2.35 |
| Example1 <Step3> | 615 | 2.73 | | Example6 <Step4> | 587 | 2.73 | | Example23 <Step3> | 555 | 2.49 |
| Example1 <Step4> | 629 | 2.83 | | Example7 | 573 | 4.02 | | Example24 | 569 | 2.55 |
| Example1 <Step5> | 444 | 0.21 | | Example8 | 585 | 2.81 | | Example25 | 555 | 3.72 |
| Example1 <Step8> | 470 | 2.23 | | Example9 | 546 | 3.88 | | Example26 | 567 | 2.61 |
| Example1 <Step7> | 370 | 0.23 | | Example10 | 560 | 4.06 | | Example27 | 528 | 3.60 |
| Example1 <Step8> | 594 | 2.93 | | Example11 <Step1> | 600 | 2.99 | | Example28 | 542 | 3.76 |
| Example1 <Step9> | 504 | 2.49 | | Example11 <Step2> | 510 | 2.61 | | Example29 | 573 | 2.69 |
| Example1 <Step10> | 573 | 2.69 | | Example11 <Step3> | 579 | 2.73 | | Example30 <Step1> | 594 | 2.91 |
| Example2 <Step1> | 684 | 3.07 | | Example12 | 593 | 2.75 | | Example30 <Step2> | 504 | 2.59 |
| Example2 <Step2> | 554 | 2.11 | | Example13 | 579 | 4.08 | | Example30 <Step3> | 573 | 2.69 |
| Example2 <Step3> | 580 | 2.73 | | Example14 | 591 | 2.85 | | Example31 | 546 | 3.88 |
| Example2 <Step4> | 490 | 2.47 | | Example15 | 552 | 3.92 | | | | |
| Example2 <Step5> | 559 | 2.59 | | Example16 | 566 | 4.10 | | | | |
| Example3 | 568 | 3.38 | | Example17 <Step1> | 723 | 3.19 | | | | |
| Example4 <Step2> | 608 | 2.83 | | Example17 <Step2> | 633 | 2.89 | | | | |
| Exemple4 <Step3> | 518 | 2.53 | | Example17 <Step3> | 562 | 2.59 | | | | |
| Example5 <Step1> | 671 | 2.97 | | Example18 | 576 | 2.63 | | | | |
| Example5 <Step2> | 541 | 2.02 | | Example19 | 562 | 3.78 | | | | |
| Example5 <Step3> | 567 | 0.61 | | Example20 | 574 | 2.71 | | | | |
| Example6 <Step1> First peak | 370 | 0.21 | | Example21 | 535 | 3.64 | | | | |
| Example6 <Step1> Second peak | 370 | 0.21 | | Example22 | 549 | 3.82 | | | | |

## Claims

1. A compound represented by the following formula (I) or its pharmaceutically acceptable salt or a solvate thereof: 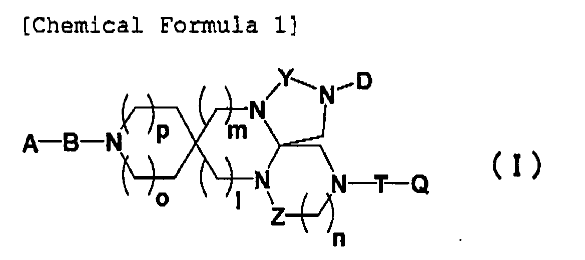 wherein
Q is an optionally substituted hydrocarbon group or heterocyclic group;
T is -S(O)ₓ-, carbonyl group, an optionally substituted C₁₋₂ alkylene group, or single bond;
A is hydrogen atom, or a group selected from (1) a saturated or unsaturated 3 to 6 membered cyclic hydrocarbon group, or a saturated or unsaturated 3 to 6 membered heterocyclic group, (2)amino group, (3) imidoyl group, (4) an aliphatic hydrocarbon group, (5) a C₂₋₆ alkanoyl group, wherein the group selected from (1) to (5) is optionally substituted;
B is single bond, carbonyl group, -S(O)_{y}-, or an optionally substituted C₁₋₂ alkylene group;
D is hydrogen atom, -CO-R⁵ (wherein R⁵ is hydrogen atom or a substituent), or an optionally substituted C₁₋₆ alkyl group;
Y is methylene group, carbonyl group, or thiocarbonyl group;
Z is methylene group, carbonyl group, or thiocarbonyl group; and
l, m, n, o, p, x and y are each independently an integer selected from 0, 1, and 2, with the proviso that 1 and m are not simultaneously 0; and
the ring containing N to which A-B- is bonded, the ring containing Y, the ring containing Z to which -T-Q is bonded, and the ring forming spiro union and containing two nitrogen atoms are optionally further substituted.

2. A pharmaceutical composition comprising at least one of the compound represented by formula (I) as described in claim 1 or its pharmaceutically acceptable salt as its effective component.

3. An anticoagulant comprising at least one of the compound represented by formula (I) as described in claim 1 or its pharmaceutically acceptable salt as its effective component.

4. A preventive and/or therapeutic agent for a disease caused by thrombus or embolus comprising at least one of the compound represented by formula (I) as described in claim 1 or its pharmaceutically acceptable salt as its effective component.

5. A FXa inhibitor comprising at least one of the compound represented by formula (I) as described in claim 1 or its pharmaceutically acceptable salt as its effective component.
